# EUROPÄISCHE PATENTSCHRIFT

(11) **EP 3 365 347 B1**
(45) Veröffentlichungstag und Bekanntmachung des Hinweises auf die Patenterteilung: **05.05.2021**
(21) Anmeldenummer: 16784909.0
(22) Anmeldetag: 21.10.2016
(51) Int. Cl.: C07F 5/02, A61K 31/4706, A61P 37/00

(54) **BORHALTIGE VERBINDUNGEN ALS INHIBITOREN DER LIPOXYGENASE UND DES LIPOXYGENASEWEGES, DEREN HERSTELLUNG UND VERWENDUNG**
BORON COMPOUNDS AS INHIBITORS OF LIPOXYGENASE AND THE LIPOXYGENASE PATHWAY, AND PREPARATION AND USE THEREOF
COMPOSÉS CONTENANT DU BORE COMME INHIBITEURS DE LA LIPOXYGÉNASE ET DE LA VOIE DE LA LIPOXYGÉNASE, LEUR PRÉPARATION ET LEUR UTILISATION

(30) Priorität: 22.10.2015 DE 102015220700
(43) Veröffentlichungstag der Anmeldung: 29.08.2018
(73) Patentinhaber: Universität Leipzig, 04109 Leipzig (DE)
(72) Erfinder: HEY-HAWKINS, Evamarie, 04821 Polent (DE); KUHNERT, Robert, 09557 Flöha (DE)
(74) Vertreter: Kailuweit & Uhlemann Patentanwälte Partnerschaft mbB
(86) Internationale Anmeldenummer: PCT/EP2016/075419
(87) Internationale Veröffentlichungsnummer: WO 2017/068145

(56) Entgegenhaltungen:
- WO-A2-2008/145733
- US-A- 4 794 188
- FRANCESC TEIXIDOR ET AL: "The First Optically Pure n ido -Monothiocarborane Cluster", ORGANOMETALLICS, Bd. 18, Nr. 25, 1. Dezember 1999 (1999-12-01), Seiten 5409-5411, XP055317928, US ISSN: 0276-7333, DOI: 10.1021/om990653s

## Beschreibung

Die Erfindung betrifft neue, borhaltige chemische Verbindungen, ein Verfahren zu deren Herstellung und deren Verwendung in der Medizin, aber auch für Katalysatoren oder andere Materialien.

Lipoxygenasen sind eine Klasse von Enzymen, welche am Metabolismus der Arachidonsäure beteiligt sind. Diese wird dabei zu biologisch aktiven Leukotrienen umgewandelt, welche an Entzündungsreaktionen beteiligt sind. Damit spielen Lipoxygenasen zum Beispiel bei Asthma sowie einigen Krebserkrankungen eine Rolle, da sie von einigen Tumoren überexprimiert werden. Die nachfolgende Entzündungsreaktion sorgt für Blutgefäßneubildung (Angiogenese), sodass der Tumor eine eigenständige Blutversorgung aufbauen und Metastasen generieren kann.
Die Inhibierung der Lipoxygenase ist deshalb von zentraler Bedeutung, um den Abbau der Arachidonsäure zu unterbinden und die nachfolgenden Entzündungsreaktionen zu minimieren.
Besondere Bedeutung kommt der Inhibierung der 5-Lipoxygenase, auch 5-LO oder 5-LOX genannt, zu.
Derzeit sind nur wenige Medikamente auf dem Markt, welche die Lipoxygenase inhibieren. Viele bisherige Kandidaten klinischer Studien scheiterten an zu geringer Bioverfügbarkeit und starkem metabolischem Abbau sowie damit verbundener geringer *in vivo* Aktivität.

Wie in einer Publikation von Mano et al. [T. Mano, K. Miyamoto, Bioorg. Med. Chem., 2003, 11, 3879-3887] beschrieben, wurden bereits Versuche mit Imidazolderivaten durchgeführt, um das Lösungsverhalten von 5-LOX-Inhibitoren zu verbessern. Allerdings sind die Synthesen für diese Derivate sehr aufwändig, was eine sehr kostenintensive Herstellung zur Folge hat. Gleichzeitig leiden die Verbindungen innerhalb des Organismus teilweise unter starkem Wirkverlust, da sie metabolisch äußerst instabil sind.

Es ist bekannt, dass Dicarba-*closo*-dodecaborane (auch Carbaborane oder Carborane, C₂B₁₀H₁₂) ungiftige [M. S. Koo, S. B. Kahl, J. Med. Chem., 2007, 50,820-827] und chemisch sowie metabolisch äußerst stabile Verbindungen darstellen [Z. J. Lesnikowski, Collect. Czech. Chem. Commun., 2007, 72, 1646-1658]. Außerdem konnte bereits gezeigt werden, dass der Einsatz von Carboranen in Arzneimitteln zu erhöhter metabolischer Stabilität führen kann [M. L. Beer, J. Lemon, J. F. Valliant, J. Med. Chem., 2010, 53, 8012-8020; J. F. Valliant, P. Schaffer, K. Stephenson, CA 2348853 A1].

US4794188 offenbart verschiedene unsymmetrische Chinolin-Ether mit antientzündlichen und antiallergischen Eigenschaften
CA 2348853 A1 offenbart eine zu Tamoxifen analoge Verbindung, bei der einer der drei Phenylringe durch einen Carborancluster ersetzt wird, wobei der Cluster eine Monofunktionalisierung aufweist. Das Clusteranalogon zeigt eine pharmakologische Aktivität zur Therapie von Brustkrebs und kann u.a. in der boron neuron capture therapy (BNCT) verwendet werden.

Teixidor et al. [F. Teixidor, M. A. Flores, C. Viñas, Organometallics, 1999, 18, 5409-5411] beschreiben optisch aktive Thiocarborancluster und den Weg der Enantiomeretrennung.

WO 2008/145733 beschreibt u.a. die Substitution von aromatischen Ringstrukturen innerhalb bestimmter Verbindungen durch borhaltige Cluster, bevorzugt Carborane, um damit verbesserte pharmazeutische Eigenschaften zu erzielen. Offenbart wird ein Verfahren zur Herstellung von *ortho*-substituierten Carboranen, die die Position von *ortho*-substituierten Phenylringen innerhalb von bekannten Verbindungen, wie beispielsweise Salicylsäure, einnehmen. Nachteilig weisen diese *ortho*-Carborane jedoch eine hohe metabolische Instabilität und eingeschränkte Wirkung auf. Die dargelegten Verbindungen sollen der verbesserten Inhibition der Cyclooxygenase dienen, allerdings sind weder Inhibitionstests am Enzym noch Cytotoxizitätstests durchgeführt und gezeigt worden. Auch wird die Wirksamkeit der Substanzen nicht belegt. *Meta-* und para-Carboranylverbindungen werden als literaturbekannte Carbonsäuren und Carbonsäurechloride genannt, jedoch haben diese Verbindungen keinerlei strukturelle Ähnlichkeit mit Inhibitoren oder Vorstufen von Inhibitoren der Lipoxygenase oder des Lipoxygenasewegs. Die weitere Umsetzung zu Verbindungen mit LOX-inhibitorischen Eigenschaften wurde in WO 2008/145733 weder angestrebt noch untersucht.

WO 2006/073938 beschreibt u.a. die Verwendung monofunktionalisierter, d.h. nur an einer Stelle substituierter Bor-Cluster zur Substitution von Indolizinringen in Verbindungen, die als antibakterielle, entzündungshemmende und antivirale Verbindungen, insbesondere gegen HIV wirksam sein sollen.

Nachteilig weisen monofunktionalisierte Cluster nur ungenügende Cytotoxizitäten gegenüber diversen Krebszelllinien auf.
Veröffentlichungen von *W. Neumann et al.* zeigen die Einführung tertiärer Alkoholsubstituenten am Carborangerüst, die in *ortho*-Position zu bereits vorhandenen Substituenten stehen. Zum Einsatz kommt dabei die doppelte Reduktion/Alkylierung einer am Carboragerüst eingeführten Carbonsäureeinheit mittels Alkyllithium. [W. Neumann, Dalton Trans., 2014, 43, 4935-4937; W. Neumann, Dalton Trans., 2015, 44, 6638 - 6644]. Nachteilig lassen sich auf diesem Wege lediglich *ortho*-substituierte Carborangerüste generieren. Auch lassen sich keine primären oder sekundären Alkohole auf diese Art und Weise erzeugen, was die Anwendbarkeit der Synthese erheblich einschränkt. Die weitere Umsetzung zu Verbindungen mit LOX-inhibitorischen Eigenschaften wurde in den Veröffentlichungen nicht untersucht.
Aufgabe der Erfindung ist es, neue chemische Verbindungen anzugeben, welche verbesserte pharmakokinetische Eigenschaften besitzen und potentiell zur Inhibition der Lipoxygenasen dienen. Sie sollen insbesondere Anwendung in der Medizin als Pharmakophore bzw. Arzneimittel, aber auch für Katalysatoren oder andere Materialien finden.
Gelöst wird die Aufgabe durch Verbindungen der allgemeinen Struktur

**[A - R₃ - X - R₄]**

Wobei
A = **[R₁** - **R₂]** oder **[R₁]**
R₁ = Aryl mit 6 - 20 C-Atomen oder Heteroaryl mit 2-20 C-Atomen
R₂ = C1-C5-Alkyl oder Carbonyl
R₃ = O, S
X = *closo*- oder *nido-* Borcluster 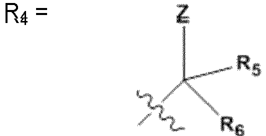
wobei Z = OH
wobei R₅ ausgewählt ist aus H, Alkyl, Aryl, Heteroaryl, Alkylether, Alkylthioether, Alkylamin, und
   R₆ ausgewählt ist aus Alkyl, Aryl, Heteroaryl, Alkylether, Alkylthioether, Alkylamin,
   oder R₅ und R₆ eine Tetrahydropyranyl-Einheit bilden,
und wobei R₃ und R₄ in *meta-* oder *para-* Position zueinander stehen

Abgeleitet werden diese Verbindungen aus Grundstrukturen oder Leitstrukturen, die bereits inhibierende Effekte auf die Lipoxygenase aufweisen, beispielsweise Naphthyl- und Chinolinderivate wie Rev-5901, MK-0591, L-674 oder ICI-211965 oder Phenyltetrahydropyranderivate wie ZD2138 oder CJ-13610. All diesen Verbindungen ist gemein, dass sie ein zentrales aromatisches Ringsystem, z.B. einen Phenylring aufweisen, der durch einen, vorzugsweise borhaltigen, Cluster ersetzt werden kann.

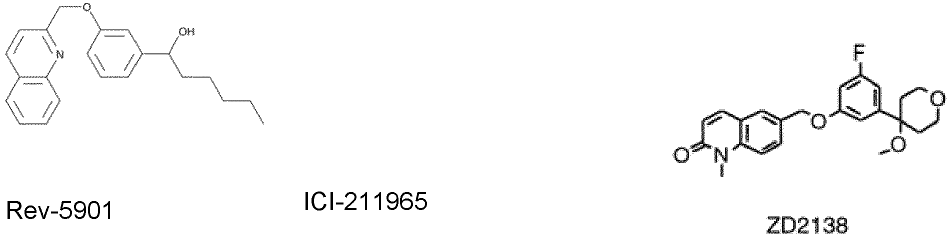

Der Erfindung liegt die Idee zugrunde, dass dieser zentrale, sterisch anspruchsvolle, aromatische Rest durch einen Cluster ersetzt werden soll. Alle erfindungsgemäßen Verbindungen weisen einen prinzipiell ähnlichen Aufbau auf, der allgemein der Struktur

**[A - R₃ - X - R₄]**

entspricht. Dies sei am Beispiel des erfindungsgemäßen Clusteranalogons **CA3** des Lipoxygenase-Inhibitors Rev-5901 verdeutlicht:

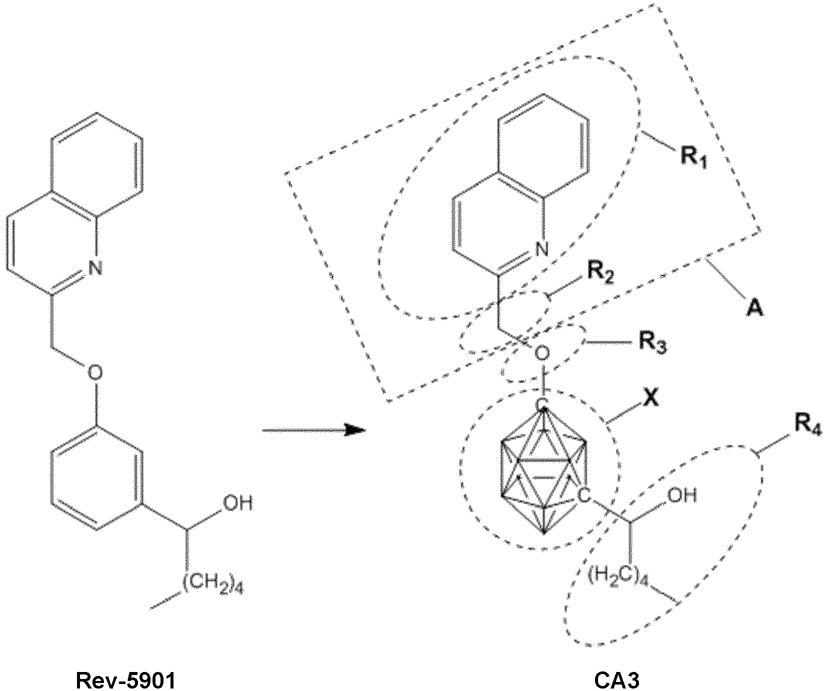

Der zentrale Phenylring in Rev-5901 ist hier durch einen Carborancluster ersetzt, womit man zum Clusteranalogon **CA3** gelangt. Die einzelnen Strukturbestandteile der allgemeinen Struktur **[A - R₃ - X - R₄]** sind in der Abbildung dargestellt.

Die vorteilhaften strukturellen Unterschiede der neuen Verbindungen führen zu verbesserter metabolischer Stabilität und verbesserter Bioverfügbarkeit gegenüber den bekannten, beispielsweise phenylsubstituierten Verbindungen, bei vergleichbarer oder verbesserter Aktivität gegenüber den Enzymen.

Erfindungsgemäß ist der Bor-Cluster *meta-* oder *para*-substituiert, da die *ortho*-Verbindungen dafür bekannt sind, chemisch sowie metabolisch wesentlich instabiler zu sein und auch synthetisch nur äußerst aufwändig generiert werden können.
Auch birgt die zweifache Substitution Vorteile, da monofunktionalisierte Clusterin vielen Fällen eine wesentlich geringere selektive Cytotoxizität gegenüber Tumorzellen aufweisen.

Der Ersatz von Phenylringen durch borhaltige Cluster führt zudem zu einer deutlichen Erhöhung der selektiven Cytotoxizität gegenüber diversen Krebszelllinien.

Zusätzlich eignen sich die borhaltigen Verbindungen prinzipiell für die Bor-Neutronen-Einfang-Therapie (BNCT), wohingegen die Phenylderivate nicht dafür geeignet sind.

Die erfindungsgemäßen Verbindungen besitzen die oben definierte allgemeine Struktur

**[A - R₃ - X - R₄]**

Der Rest A ist verbunden mit dem Rest R₃ und wird gebildet aus R₁ oder [R₁ - R₂]. Wenn A = [R₁ - R₂], dann ist R₂ ein divalenter Rest, der R₁ und R₃ miteinander verbindet.

R₁ stellt eine für die pharmakologische Wirkung wichtige funktionelle Gruppe im Molekül dar und umfasst aromatische als auch heteroaromatische Gruppen, die jeweils substituiert oder unsubstituiert sind. Der aromatische Rest weist 6 bis 20 C-Atome, bevorzugt 6 bis 15 C-Atome. Bevorzugt enthält der heteroaromatische Rest ein oder mehrere Stickstoff-Atome als Heteroatome. Erfindungsgemäß enthält der heteroaromatische Rest 2 bis 20 C-Atome, bevorzugt 2 bis 10 C-Atome.

In einer bevorzugten Ausführung handelt es sich bei R₁ um einen Chinolin- oder Naphthylrest, der jeweils substituiert oder unsubstituiert ist.

Für Naphthyl- und Chinolinverbindungen, die an zentraler Stelle im Molekül eine Phenyl-Substitution aufweisen, sind bereits LOX-inhibitorische Eigenschaften bekannt. Beispiele dafür sind die bekannten pharmazeutischen Verbindungen Rev-5901, MK-0591, L-674 oder ICI-21965.

Erfindungsgemäß wird der Rest R₂ ausgewählt aus C1-C5-Alkyl und Carbonyl.

Erfindungsgemäß gilt für die oben genannte Formeln sowie für alle nachfolgend aufgeführten Formeln:
Unter Alkyl versteht man verzweigte und unverzweigte, substituierte und unsubstituierte Reste. Bevorzugt sind C1 bis C20 Alkylreste, besonders bevorzugt C1 bis C10 Alkylreste, ganz besonders bevorzugt C1 bis C5 Alkylreste. Bevorzugte Substituenten an den Alkylresten sind ausgewählt aus Hydroxy-, Thio- oder Aminogruppen.
Unter Aryl versteht man substituierte und unsubstituierte aromatische Reste mit bevorzugt 3 bis 30 C-Atomen, bevorzugt 6 bis 20 C-Atomen.

Unter Heteroaryl versteht man substituierte und unsubstituierte aromatische Reste mit bevorzugt 2 bis 20 C-Atomen, bevorzugt 3 bis 10 C-Atomen und mindestens einem Heteroatom, bevorzugt bis zu 50 % der im Heteroaromaten enthaltenen Atome. Die Heteroatome sind bevorzugt ausgewählt aus N, S und O.

In einer vorteilhaften Ausgestaltung ist R₂ ein unverzweigter, bevorzugt unsubstituierter Alkylrest, mit bevorzugt 1 bis 3 C-Atomen.

Erfindungsgemäß versteht man unter R₄ folgende Struktur: 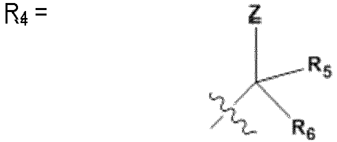
wobei Z = OH,
wobei R₅ ausgewählt ist aus H, Alkyl, Aryl, Heteroaryl, Alkylether,
   Alkylthioether, Alkylamin,
   und R₆ ausgewählt ist aus Alkyl, Aryl, Heteroaryl, Alkylether, Alkylthioether, Alkylamin,
   oder R₅ und R₆ eine Tetrahydropyranyl-Einheit bilden.

In einer bevorzugten Ausführung bilden die Reste R₅ und R₆ eine Tetrahydropyranyleinheit. Für Tetrahydropyranylderivate, die an zentraler Stelle einen Phenylring enthalten, sind ebenfalls LOX-inhibitorische Eigenschaften bekannt. Beispielhaft sei hier der Vertreter ZD2138 genannt.

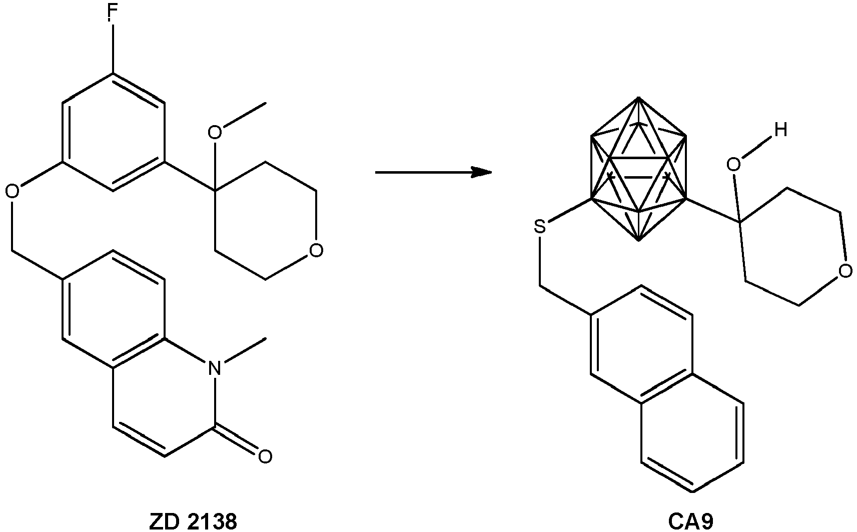

Man ersetzt in ZD 2138 den substituierten Phenylring durch einen Cluster und gelangt zu dem Clusteranalogon **CA9,** welches verbesserte pharmakokinetische Eigenschaften aufweist. Die extrem hydrophoben Cluster erlauben eine verbesserte Überwindung der Zellmembran, um somit leichter und effizienter in Zellen zu gelangen und dort ihre Wirkung auf das LOX-System zu entfalten.

Erfindungsgemäß sind die aromatischen oder heteroaromatischen Reste R₁ entweder direkt oder über einen divalenten Rest R₂ mit dem Rest R₃ verbunden.

R₃ ist direkt an den borhaltigen Cluster X gebunden und ist ausgewählt aus Heteroatomen. Erfindungsgemäß ist R₃ durch O oder S repräsentiert.

Erfindungsgemäß handelt es sich bei X um einen *nido-* oder *closo*-Borcluster.

Solche Cluster können vorteilhaft als Pharmakophore, funktionelle Gruppen und dreidimensionale Strukturelemente angewendet werden.

Generell werden Cluster analog ihrer allgemeinen Definition als "Häufung Gleicher" gesehen, sie können verschiedene Geometrien aufbauen, geladen oder ungeladen sein.

Die Geometrien der Cluster ergeben sich aus den Wade-Regeln [K. Wade, *Adv. Inorg. Chem. Radiochem.,* **1976***, 18*, 1] oder den erweiterten Wade-Mingos-Regeln.

Erfindungsgemäß handelt es sich bei X um *nido-* oder *closo*-Borcluster, welche n = 6 bis 12 Boratome, und m Wasserstoffatome enthalten, mit m = n + i wobei i eine Integer (ganze Zahl von 1 bis 10, bevorzugt 2 bis 10, besonders bevorzugt 2 bis 6) darstellt.

Von der Erfindung umfasst sind *nido-* oder *closo*-Borcluster X, wobei 1 bis 4 B-Atome innerhalb eines Clusters durch Atome von Hauptgruppenelementen ersetzt sein können bzw. wobei 0 bis 4 B-Atome eines Clusters durch Atome von Hauptgruppenelementen ersetzt sind.

Bevorzugt enthalten die *nido-* oder *closo*-Borcluster X 1 bis 4, bevorzugt 1 oder 2 Kohlenstoffatome, wobei diese kohlenstoffatom-haltigen Borcluster als Carbaborane, Dicarbaborane, Dicarbadodecaborane, Dicarba-*closo*-dodecaborane oder auch Carborane bezeichnet werden.

Von der Erfindung umfasst sind aber auch Verbindungen, in denen es sich bei X um Silaborane mit Si-Atomen an Stelle von 1 bis 4 Bor-Atomen, Phosphaborane mit P-Atomen an Stelle von 1 bis 4 Bor-Atomen, Azaborane mit N-Atomen an Stelle von 1 bis 4 Bor-Atomen, Thiaborane mit S-Atomen an Stelle von 1 bis 4 Bor-Atomen handelt.

Aber auch Verbindungen mit X = borhaltige Cluster, die Metalle enthalten, sogenannte Metallacarborane, sind von der Erfindung umfasst. Diese Metallacarborane sind durch Umsetzung eines borhaltigen Clusters mit Metallsalzen einfach zugänglich (M.F. Hawthorne, *Organomet. Chem.,* **1975,** *100,* 97).

*Closo*-Borcluster im Sinne der Erfindung bezeichnet Verbindungen, bei denen die Bor-Atome an den Ecken eines Deltaeders, also eines Polyeders, das nur von Dreiecksflächen begrenzt wird, sitzen. Bevorzugt ist der Ikosaeder, sodass der Borcluster also in Form eines Ikosaeders vorliegt.

Die Wasserstoffatome der B-H-Bindungen des *closo*-Borclusters sind kovalent an das jeweilige Boratom gebunden und zeigen radial nach außen.

*Closo*-Borcluster können einfach in *nido*-Borcluster überführt werden. Dabei ergibt sich die Struktur der *nido*-Borcluster aus der Struktur der *closo*-Borcluster, indem eine Ecke des *closo-*Clusters nicht mit einem Boratom besetzt wird. Es entsteht dabei also eine "offene" Struktur. Die Wasserstoffatome dieser Borane besetzen einerseits wieder alle radial außen liegenden Positionen an den Boratomen und zusätzliche Plätze an den geöffneten Teilen der Polyeder. (siehe auch Hey-Hawkins et al. Chem Rev. 2011, 111, 7035).

Ein closo-Carboran kann beispielsweise durch eine der gängigen Methoden in das entsprechende *nido*-Carboran überführt werden (siehe z. B. [R.A. Wiesboeck, M. F. Hawthorne, J. Am. Chem. Soc., 1964, 86, 1642, M. F. Hawthorne, D. C. Young, P. M. Garrett, D. A. Owen, S. G. Schwerin, F. N. Tebbe, P. A. Wegner, J. Am. Chem. Soc., 1968, 90, 862, L.I. Zakharkin, V.S. Kirillova, Izv. Akad. Nauk. SSSR. Ser. Khim., 1975, 11, 2596, J. L. Maurer, A. J. Serino, M. F. Hawthorne, Organometallics, 1988, 7, 2519, H. Tomita, H. Luu, T. Onak, Inorg. Chem., 1991, 30, 812, J.J. Schaeck, S. B. Kahl, Inorg. Chem., 1999, 38, 204.]).

Auch die nido-Carborane enthaltenden Verbindungen, sowohl die neutralen, monoanionischen sowie dianioschen nido-Carborane, sind von der Erfindung umfasst, wobei die dianioschen *nido*-Carborane auch als Carbollid oder Dicarbollid bezeichnet werden. Über die *nido*-Carborane, die isolobal zu Cyclopentadienylanionen sind, können ebenfalls entsprechende Metallacarborane gebildet werden. Die Transformation in die Metallacarborane wird erfindungsgemäß auch als Werkzeug gesehen, die Eigenschaften der Cluster als Pharmakophore zu modifizieren und an das jeweilige System anzupassen. Die Metallacarborane werden selbst als Pharmakophore angesehen.

Bevorzugte Cluster X sind:
a) Borane mit Hauptgruppenelementen: C₂B₈H₁₀, C₂B₁₀H₁₂, Si₂B₁₀H₁₂, P₂B₁₀H₁₀, SB₁₁H₁₁, C₂B₉H₁₁⁽⁻⁾, NB₁₁H₁₁⁽⁻⁾, PB₁₁H₁₁⁽⁻⁾, CB₆H₇⁽⁻⁾, CB₇H₈⁽⁻⁾, CB₉H₁₀⁽⁻⁾, CB₉H₁₂⁽⁻⁾, CB₁₀H₁₁⁽⁻⁾, CB₁₁H₁₂⁽⁻⁾, SiB₁₁H₁₂⁽⁻⁾, CB₁₁H₁₁⁽²⁻⁾, SiB₁₁H₁₁⁽²⁻⁾, SnB₁₁H₁₁⁽²⁻⁾, GeB₁₁H₁₁⁽²⁻⁾, C₂B₉H₁₂, C₂B₉H₁₂⁽⁻⁾; C₂B₉H₁₁⁽²⁻⁾
b) Clusterfragmente: RₐC₃BₙHₙ₊₃₋ₐ⁽⁻⁾, RₐC₂BₙHₙ₊₂₋ₐ⁽⁻⁾, C₃B₈H₁₁⁽⁻⁾, R₂C₃B₈H₉⁽⁻⁾, C₂B₉H₁₁⁽⁻⁾, C₂B₉H₁₁⁽²⁻⁾, R₂C₂B₉H₉⁽⁻⁾,(R = H, Alkyl, Aryl, Silyl).

Besonders bevorzugt ist X = 1,6-C₂B₈H₁₀, 1,10-C₂B₈H₁₀, 1,7-C₂B₁₀H₁₂, 1,12-C₂B₁₀H₁₂, 2,3-C₂B₉H₁₀, Si₂B₁₀H₁₂, P₂B₁₀H₁₀, SB₁₁H₁₁, NB₁₁H₁₁⁽⁻⁾, PB₁₁H₁₁⁽⁻⁾, CB₆H₇⁽⁻⁾, CB₇H₈⁽⁻⁾, CB₉H₁₀⁽⁻⁾, CB₉H₁₂⁽⁻⁾, CB₁₀H₁₁⁽⁻⁾, CB₁₁H₁₂⁽⁻⁾, SiB₁₁H₁₂⁽⁻⁾, CB₁₁H₁₁⁽²⁻⁾, SiB₁₁H₁₁⁽²⁻⁾, SnB₁₁H₁₁⁽²⁻⁾, GeB₁₁H₁₁⁽²⁻⁾, 7,9-C₂B₉H₁₂⁽⁻⁾, 7,9-C₂BgH₁₁²⁽⁻⁾, 2,9-C₂B₉H₁₂⁽⁻⁾, 2,9-C₂B₉H₁₁²⁽⁻⁾, RₐC₃BₙHₙ₊₃₋ₐ⁽⁻⁾, RₐC₂BₙHₙ₊₂₋ₐ⁽⁻⁾, C₃B₈H₁₁⁽⁻⁾, R₂C₃B₈H₉⁽⁻⁾, C₂B₉H₁₁⁽⁻⁾, R₂C₂B₉H₉⁽⁻⁾, (R = H, Alkyl, Aryl, Silyl).

In einer bevorzugten Ausführungsform ist X = 1,7-Dicarba-*closo*-dodecaboran (*meta-closo-*Carboran, 1,7-C₂B₁₀H₁₂). In einerweiteren bevorzugten Ausführungsform ist X = 1,12-Dicarba-*closo*-dodecaboran (para-closo-Carboran, 1,12-C₂B₁₀H₁₂).

Carborane, speziell Dicarba-*closo*-dodecaborane(12), zeichnen sich durch ihre Lipophilie, Elektronenzug, Sterik und Stabilität (thermisch und gegenüber biologischem Metabolismus) aus. Sie werden teilweise als dreidimensionale Aromaten bezeichnet, lassen sich durch den Ersatz der Wasserstoffatome sowohl an den Bor- als auch an den Kohlenstoffecken mit funktionellen Gruppen oder organischen und anorganischen Resten ausstatten.

Bevorzugte Borcluster sind Carborane, d.h. kohlenstoffhaltige Borane, die sich durch die Integration von isolobalen Kohlenstofffragmenten ergeben.

Wenn der Cluster ein Carboran ist, wird hier im Interesse der besseren graphischen Darstellbarkeit auf die Ausformulierung der BH-Einheiten im Carboran verzichtet. D. h. die Eckpunkte im Cluster stehen jeweils für eine BH-Einheit, die C-Atome werden an ihrer Position dargestellt, wie hier exemplarisch für das 1,7-C₂B₁₀H₁₂ gezeigt:

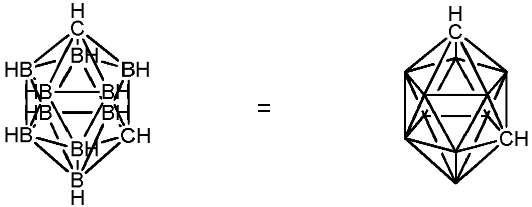

Erfindungsgemäß stehen die Reste R₃ und R₄ in *meta-* oder *para*-Position zueinander.

Handelt es sich bei X um Carborancluster, so ist X = 1,7-Dicarba-*closo*-dodecaborane(12) (X = 1,7-C₂B₁₀H₁₂) oder X = 1,12-Dicarba-*closo*-dodecaborane(12) (X= 1,12-C₂B₁₀H₁₂) oder eine mit diesen verwandte Verbindung, die sich durch die Anwendung des Isolobalkonzeptes aus diesem ergeben.

Mit verwandter Verbindung gemeint sind auch die verwandten Cluster, die synthetisch aus den Dicarba-*closo*-dodecaboranen(12) zugänglich sind.

Handelt es sich bei X um einen Borcluster anderer Hauptgruppenelemente als C (Kohlenstoff), so gilt dieselbe Definition, nur dass an Stelle des C das jeweilige Hauptgruppenelement steht.

Sowohl die geladenen als auch die ungeladenen Cluster oder Komplexe aus Clusterfragmenten werden nachfolgend und in den Ansprüchen mit X abgekürzt. X repräsentiert somit allgemein einen Bor-Cluster, vorallem Carboran-Cluster, insbesondere einen der oben oder nachfolgend aufgeführten.

Aufgrund ihrer besonderen metabolischen Stabilität werden 1,7-Dicarba-*closo*-dodecaborane (*meta*-*closo*-Carborane, 1,7-C₂B₁₀H₁₂) bevorzugt als Cluster eingesetzt, um Ringsysteme in den Leitstrukturen zu substituieren. Dabei geben die Ziffern 1 und 7 die Position der Kohlenstoff-Atome innerhalb des Clusters an. Die anderen Positionen sind BH-Einheiten, wobei die Wasserstoffatome einzelner oder aller BH-Einheiten durch Hydroxy-, Methylgruppen oder Halogene (F, Cl, Br, I), ggf. radiomarkierter Halogene, ersetzt sein können.

Von den racemischen Vertretern sind jeweils auch die enantiomerenreinen Verbindungen von der Erfindung umfasst.

Die Wasserstoffe einzelner oder mehrerer BH-Einheiten können komplett oder partiell durch Halogene oder radiomarkierte Halogene F, Cl, Br, I (vorzugsweise mit I = ¹³¹I) ersetzt sein (Beispiele 20 bis 24). Damit eignen sich die erfindungsgemäßen Verbindungen auch als Marker für diagnostische Zwecke.
Halogene können zum Beispiel über elementares Iod in Anwesenheit von Säuren oder Lewissäuren eingeführt werden [M. Tominga, Macromol. Rapid Comm., 2013, 34, 1357; M. Scholz, E. Hey-Hawkins, Chem. Rev., 2011, 111, 7035]
In einer Ausführungsform sind eine oder mehrere BH-Einheiten durch radiomarkierte B-Hal Einheiten substituiert.

Erfindungsgemäß handelt es sich bei dem Rest R₄ um Hydroxyalkylgruppen, die frei vorliegen können, aber auch verethert oder verestert mit Resten gleich oder ungleich dem erfindungsgemäßen Rest A.

Vorteilhaft zeichnen sich die erfindungsgemäßen carboran-haltigen Verbindungen dadurch aus, dass sie z.T. synthetisch leichter zugänglich sind als entsprechende Phenylderivate. Der Einsatz spezieller Syntheseschritte ermöglicht beispielsweise den Verzicht auf die Verwendung von Schutzgruppen.
Ein weiterer entscheidender Vorteil liegt in der verbesserten Löslichkeit in vielen gängigen Lösungsmitteln, der durch die Substitution der Phenyleinheit durch einen Carborancluster innerhalb der Leitstrukturen erzielt wird.

Erfindungsgemäß repräsentiert X allgemein einen Bor-Cluster, insbesondere einen Carboran-Cluster. X ist bevorzugt einer der bereits vorher definierten Clustertypen. R₁, R₂ und R₃ sind in dieser Reihenfolge miteinander verknüpft und über R₃ an den Cluster gebunden. Diese Gruppierung steht in einer bevorzugten Variante in *meta*-Position und in einer weiteren bevorzugten Variante in *para*-Position zum Rest R₄.

Am Beispiel des LOX-Inhibitors Rev-5901 kann das Prinzip des Ersatzes einer Ringstruktur in LOX-inhibierenden Verbindungen durch ein geeignetes Clustergerüst allgemein wie folgt dargestellt werden:

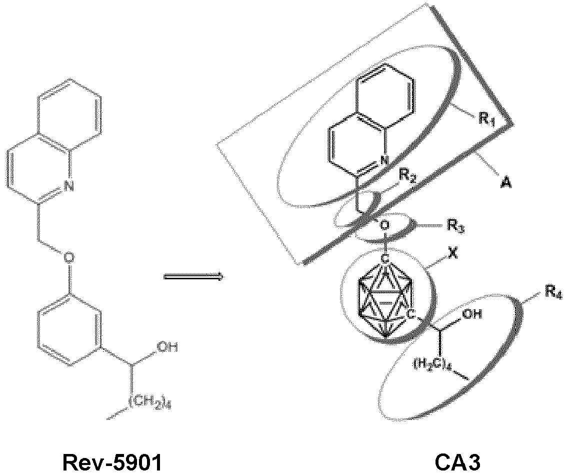

Der Einbau eines Clusters anstelle des Phenylrings in Rev-5901 führt zum Clusteranalogon **CA3** [1-(7-Chinolin-2-ylmethoxy-1,7-dicarba-*closo-*dodecaboran(12)yl)-hexan-1-ol], Ausführungsbeispiel 3.

Die Verbindung gemäß Formel **CA3** (Ausführungsbeispiel **3**) stellt das Carboranylanalogon von Rev-5901 dar, weist jedoch vorteilhaft zusätzlich die positiven Eigenschaften der Carborane auf. Der dreidimensionale hydrophobe Carborankäfig ist größer als der Phenylring und eignet sich als lipophiles, hydrophobes Pharmakophor. Er richtet die OH- und die Chinolin-Gruppe zweidimensional aus und dient selbst als funktionelle Gruppe, die modifiziert werden kann.

Die erfindungsgemäßen Verbindungen weisen vorteilhaft ein verbessertes Lösungsverhalten auf: Sie lösen sich sowohl in unpolaren Lösungsmitteln wie Pentanen, als auch polaren Lösungsmitteln wie z.B. in Ethanol.
So weist beispielsweise Rev-5901 lediglich eine Löslichkeit von 29.7 mmol/L in Ethanol bzw. 29.8 mmol/L in DMSO auf. Das Clusteranalogon **CA3** hingegen zeigt um ein Vielfaches erhöhte Löslichkeiten von 86.8 mmol/L in Ethanol bzw. 208.3 mmol/l in DMSO.
Dass Carborane außerdem eine höhere metabolische Stabilität besitzen, ist literaturbekannt (siehe z.B. F. Issa, M. Kassiou, L. M. Rendina, Chem. Rev. 2011, 111, 5701 oder M. L. Beer, J. F. Valliant, J. Med. Chem., 2010, 53, 8012.)

Vorteilhaft weisen die Carborananaloga von bekannten, phenylsubstituierten LOX-Inhibitoren in Inhibitionstests am Enzymsystem 5-Lipoxygenase/5-Lipoxygenase-aktivierendes-Protein trotz des Ersatzes des Phenylrings vergleichbare IC₅₀-Werte auf (siehe Tabelle **2**). Die Verbindungen werden durch den Ersatz des Phenylrings durch einen Carborancluster also nicht negativ in ihrer Wirkung beeinflusst. Somit ist es nunmehr möglich, die vorteilhaften Eigenschaften der Carborane, wie gute Löslichkeit, höhere metabolische Stabilität und breitere Einsatzmöglichkeiten mit den LOX-inhibitorischen Eigenschaften der phenylsubstituierten Verbindungen zu vereinen.
Überraschend weisen die Carborananaloga von bekannten, phenylsubstituierten LOX-Inhibitoren wie z.B. Verbindung **3** (ein Analogon von Rev-5901) überraschend eine deutlich gesteigerte Cytotoxizität gegenüber diversen Krebszelllinien auf im Vergleich zu den phenylbasierten Verbindungen (siehe auch Abschnitt Cytotoxizität).
Gezeigt werden kann dies an Melanom- und Darmkrebszelllinien. Vor allem die Melanomkrebszelllinie A375, welche hoch aggressiv und dafür bekannt ist, gegen Chemotherapie resistent zu sein, hat gegenüber den Carborananaloga phenylsubstituierter LOX-Inhibitoren IC₅₀-Werte im einstelligen mikromolaren Bereich, das heißt, dass bereits bei sehr niedrigen Konzentrationen an Verbindung **3** im Medium die Hälfte der untersuchten Zellen abstirbt. Dies ist insbesondere für die gezielte Therapie von Metastasen von sehr hoher Bedeutung.

Im Gegensatz dazu hat die originale, phenylbasierte Vergleichssubstanz Rev-5901 IC₅₀-Werte zwischen 25 und über 50 mikromolar, je nach verwendeter Untersuchungsmethode, und weist damit eine deutlich geringere Cytotoxizität gegenüber der Krebszelllinie auf.

Wichtig für die spätere pharmakologische Anwendbarkeit ist außerdem eine hohe Selektivität der Verbindungen bezüglich gesunder und pathologischer Zellen.

Zur Bestimmung der Selektivität wurden ebenso Cytotoxizitätsuntersuchungen an gesunden Zellen durchgeführt. Hierzu wurde die Zellinie MRC-5 herangezogen. Um die Selektivität zwischen gesunden und Krebszellen zu ermitteln, wurde der IC₅₀-Wert gesunder Zellen durch den IC₅₀-Wert der Krebszellen geteilt. Es zeigt sich, dass die phenylbasierte Vergleichssubstanz Rev-5901 gegenüber gesunden Zellen einen IC₅₀-Wert von 70 mikromolar hat, sodass die Selektivität dieser Verbindung zwischen gesunden Zellen und Krebszellen lediglich den Wert 2,8 aufweist.

Im Gegensatz dazu zeigt zum Beispiel die erfindungsgemäße Verbindung **5** (Ausführungsbeispiel 5) einen IC₅₀-Wert für A375-Krebszellen von 8 mikromolar und einen IC₅₀-Wert für MRC-5 von 82 mikromolar, sodass sich eine deutlich vorteilhafte Selektivität von 10,2 errechnen lässt. Das bedeutet, dass sich durch die Verwendung carboransubstituierter LOX-Inhibitoren das Wirkfenster dieser Wirkstoffe verdreifachen lässt.

Es zeigt außerdem, dass monofunktionalisierte Cluster, bei denen R₄ = H ist, nur ungenügende Cytotoxizitäten gegenüber Krebszellen zeigen (Vergleichsbeispiel 1 und 2, Abschnitt Cytotoxizität).

In einer Ausführungsform erfolgt die Funktionalisierung durch Deboronierungsmethoden und den isolobalen Ersatz von BH-Einheiten. Auch können die Wasserstoffe einzelner oder mehrerer BH-Einheiten beispielsweise durch Halogene oder durch radiomarkierte Halogene substituiert sein. Die Eigenschaft des Clusters kann so weiter modifiziert werden. Der Cluster kann beispielsweise auch durch die Knüpfung einer B-I-Bindung mit ¹³¹I markiert werden.

Aus dem stark hydrophoben Käfig wird durch Deboronierung eine geladene anionische Spezies.
In einer weiteren Ausführungsform erfolgt die Funktionalisierung durch Umwandlung in ein Metallacarboran, um die bestehenden Eigenschaften weiter zu modifizieren.

Die Erfindung umfasst auch ein Herstellungsverfahren für die erfindungsgemäßen Verbindungen gemäß der allgemeinen Struktur

**[A - R₃ - X - R₄]**

wobei
A = **[R₁** - **R₂]** oder **[R₁]**
R₁ = Aryl mit 6 - 20 C-Atomen oder Heteroaryl mit 2 - 20 C-Atomen
R₂ = C1-C5-Alkyl oder Carbonyl,
R₃ = O, S,
X = *closo*- oder *nido-* Borcluster 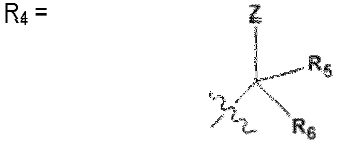
   wobei Z = OH,
   wobei R₅ ausgewählt ist aus H, Alkyl, Aryl, Heteroaryl, Alkylether,
      Alkylthioether, Alkylamin, und
      R₆ ausgewählt ist aus Alkyl, Aryl, Heteroaryl, Alkylether, Alkylthioether, Alkylamin,
      oder R₅ und R₆ eine Tetrahydropyranyl-Einheit bilden,
   und wobei R₃ und R₄ in *meta-* oder *para-* Position zueinander stehen.

Der Rest A ist verbunden mit dem Rest R₃ und wird gebildet aus R₁ oder [R₁ - R₂]. Wenn A = [R₁ - R₂], dann ist R₂ ein divalenter Rest, der R₁ und R₃ miteinander verbindet.

Das erfindungsgemäße Herstellungsverfahren umfasst die Schritte:
a) Hydroxyalkylierung des Clusters X
b) Hydroxylierung oder Thiolierung des Clusters X,
   zu einer Zwischenverbindung der allgemeinen Formel [H-R₃ - X - R₄]
   wobei
   R₃ = O, S,
   X = *nido-* oder *closo*-Borcluster 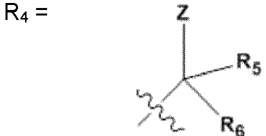
   wobei Z = OH,
   wobei R₅ ausgewählt ist aus H, Alkyl, Aryl, Heteroaryl, Alkylether, Alkylthioether, Alkylamin,
      und R₆ ausgewählt ist aus Alkyl, Aryl, Heteroaryl, Alkylether, Alkylthioether, Alkylamin,
      oder R₅ und R₆ eine Tetrahydropyranyl-Einheit bilden.
   und wobei R₃ und R₄ in *meta-* oder *para-* Position zueinander stehen, und
c) Selektive Veretherung oder Veresterung von H-R₃ zur Einführung von A,
   wobei die Schritte a) und b) beliebig vertauscht werden können,
   und gegebenenfalls nachfolgend
d) Veretherung oder Veresterung der freien Hydroxygruppe Z=OH mit einem von A verschiedenen Rest.

Der erste Schritt ist bevorzugt die Hydroxyalkylierung, der zweite Schritt ist bevorzugt die Hydroxylierung oder Thiolierung, der dritte Schritt ist bevorzugt die selektive Veretherung oder Veresterung von H-R₃. Die Reihenfolge der ersten beiden Schritte kann jedoch auch beliebig geändert werden.

### Hydroxyalkylierung

Die Hydroxyalkylierung erfolgt nach dem Stand der Technik durch Deprotonierung des Carborans durch eine geeignete Base und anschließender Reaktion mit Ketonen oder Aldehyden. [J. Cai, Chem. Lett., 1996, 791-792.] Analog dazu kann die Thioalkylierung mit der entsprechenden Thiocarbonylverbindung bzw. eine Aminoalkylierung mittels eines entsprechenden Enamins erfolgen.

Durch folgendes Hydroxyalkylierungsverfahren wird bevorzugt eine Hydroxyalkylgruppe am Carboran eingeführt:

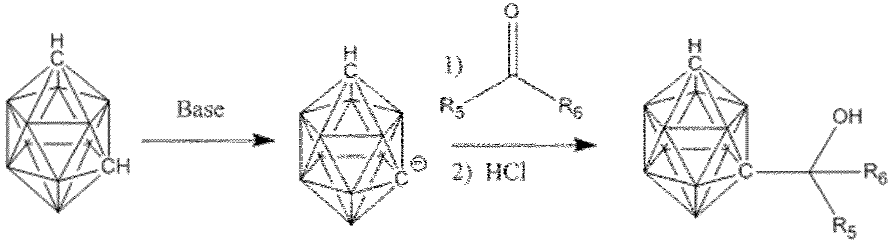

Die Reste R₅ und R₆ sind im Sinne der Erfindung vom Fachmann auszuwählen.
Der erste Schritt ist die Deprotonierung mit einer geeigneten Base, bevorzugt Alkalimetallorganyle, Metallamide, Silazane, Metallhydride oder Ammoniumverbindungen, wie z.B. n-BuLi, MeLi, Lithiumhexamethyldisilazan (LiHMDS), Natriumhexamethyldisilazan (NaHMDS), Kaliumhexamethyldisilazan (KHMDS), Lithiumdiisopropylamid (LDA), Tetrabutylammoniumfluorid (TBAF), NaH, NaNH₂ oder Verbindungen mit vergleichbarer Basizität Anschließend werden zu den entstehenden Carboranylanionen Carbonylverbindungen wie Aldehyde oder Ketone gegeben, wobei der Carbonylkohlenstoff dieser Verbindungen durch das Carboranylanion nukleophil angegriffen wird. Anschließende saure Aufarbeitung liefert die gewünschten Hydroxyalkylcarborane.

### Hydroxylierung

Die Hydroxylierung kann wie bisher nachdem Stand der Technik mittels Umsetzung des monolithiierten Clusters mit O₂, Dibenzoylperoxid, Bis(trimethylsilyl)peroxid oder mit einem Trialkylborat und anschließender Oxidation erfolgen. Bevorzugt erfolgt die Hydroxylierung jedoch nach dem Stand der Technik durch Deprotonierung des Clusters mit einer geeigneten Base, Knüpfung einer heteronuklearen Bindung mit einem Trialkylborat oder Bortrihalogenid und anschließender Oxidation. [K. Ohta, Inorg. Chem., 2007, 46, 3966.]

Durch folgendes Hydroxylierungsverfahren wird bevorzugt eine Hydroxygruppe am Carboran (an einem C-Atom des Carboran-Clusters) eingeführt, die dann später weiter modifiziert, z.B. verestert oder verethert, werden kann: 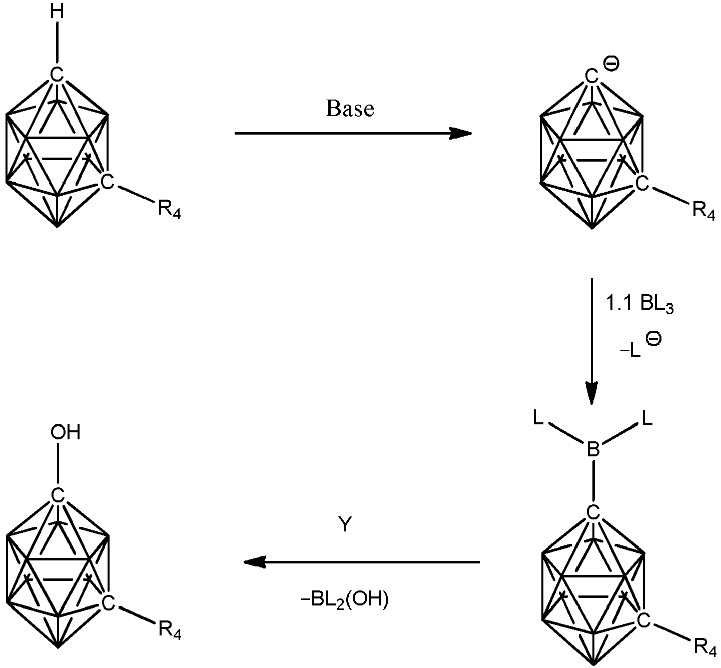
L = OEt, OPr, OBu, F, Cl, Br, I
Y = Peroxid, Persäure

Der erste Schritt ist die Deprotonierung des C-Atoms mit einer geeigneten Base, bevorzugt Alkalimetallorganyle, Metallamide, Silazane, Metallhydride oder Ammoniumverbindungen, wie z.B. n-BuLi, MeLi, Lithiumhexamethyldisilazan (LiHMDS), Natriumhexamethyldisilazan(NaHMDS), Kaliumhexamethyldisilazan (KHMDS), Lithiumdiisopropylamid (LDA), Tetrabutylammoniumfluorid (TBAF), NaH, NaNH₂ oder Verbindungen mit vergleichbarer Basizität. Der zweite und zentrale Schritt des Verfahrens ist die Knüpfung einer heteronuklearen Bindung. B repräsentiert das Element Bor, es sind aber auch andere Elemente denkbar (z.B. P, As). L stellt ein Halogenid (F, Cl, Br, I), ein Alkoholat (OR) oder einen Alkylrest (R) dar. R ist ein linearer oder verzweigter, gesättigter oder ungesättigter, aromatischer oder nicht aromatischer, chiraler oder achiraler kohlenstoffhaltiger Rest. L kann innerhalb von BL₃ verschieden sein und auch in der Peripherie verknüpft sein. Die heteronukleare Bindung enthaltende Spezies kann isoliert werden, sie kann aber auch direkt *in situ* weiter umgesetzt werden. Der letzte Schritt in der Darstellung stellt die Umsetzung mit einer Peroxospezies dar. Dies kann entweder vornehmlich eine Persäure (z.B. Peressigsäure) oder ein Peroxid (Wasserstoffperoxid, Alkylperoxid) oder anderes übliches Oxidationsmittel sein.

Die Reinigung der Verbindung erfolgt durch die Wahl einer geeigneten, wässrigen Base, bevorzugt ausgewählt aus NaOH und KOH.
Durch die Base wird das Hydroxycarboran als Anion vorteilhaft in die wässrige Phase überführt und so vom Ausgangsstoff, der in protonierter Form in der organischen Phase vorliegt, durch Phasentrennung isoliert. Vorteilhaft kann somit mittels Extraktion unumgesetzter Ausgangsstoff zurückgewonnen werden. Eine lösungsmittel- und daher kostenintensivere säulenchromatographische Reinigung ist somit nicht notwendig.
Bevorzugt wird bei der erfindungsgemäßen Reinigung das Rohprodukt in einer wässrigen basischen Lösung (vorzugsweise verdünnte KOH- oder NaOH-Lösung) aufgeschlämmt (oder mehrmals extrahiert, falls das Rohprodukt noch in Lösung vorliegt) und dann mit einem organischen Lösungsmittel (vorzugsweise einem apolaren Lösungsmittel, wie z. B. Diethylether) mehrmals extrahiert. In der organischen Phase befindet sich der Ausgangsstoff. Die wässrigen basischen Phasen werden dann angesäuert (vorzugsweise mit HCl bis etwa pH=1). Hierbei zeigt sich das Produkt als weißer Feststoff. Dieser kann erneut mit einem organischen Lösungsmittel (vorzugsweise einem apolaren Lösungsmittel, wie z. B. Diethylether) extrahiert werden. Nach Entfernen des Lösungsmittels erhält man das entsprechend substituierte Hydroxycarboran.
Sollte das Produkt in ungenügender Reinheit erhalten werden, ist ein Sublimationsschritt möglich.
Durch dieses Verfahren sind alle Hydroxycarborane und andere hydroxylierte borhaltige Cluster zugänglich.

### Thiolierung

Die Thiolierung erfolgt nach dem Stand der Technik durch Deprotonierung des Carborans (eines C-Atoms des Carboran-Clusters) durch eine geeignete Base und anschließende Reaktion mit elementarem Schwefel. [J. Plešek, S. Heřmánek, Collect. Czech. Chem. Commun., 1981, 46, 687-692.] Die Reaktionssequenz ist wie folgt schematisch dargestellt:

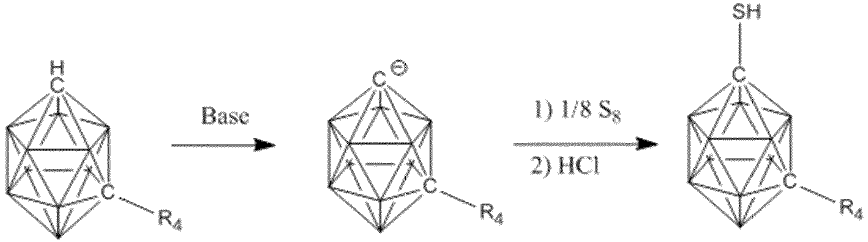

Der erste Schritt ist die Deprotonierung eines C-Atoms mit einer geeigneten Base, bevorzugt Alkalimetallorganyle, Metallamide, Silazane, Metallhydride oder Ammoniumverbindungen, wie z.B. *n*-BuLi, MeLi, Lithiumhexamethyldisilazan (LiHMDS), Natriumhexamethyldisilazan(NaHMDS), Kaliumhexamethyldisilazan (KHMDS), Lithiumdiisopropylamid (LDA), Tetrabutylammoniumfluorid (TBAF), NaH, NaNH₂ oder Verbindungen mit vergleichbarer Basizität. Der zweite Schritt des Verfahrens ist die nukleophile Substitution elementaren Schwefels durch diese Carboranylanionen. Nach saurer wässriger Aufarbeitung werden die entsprechenden Thiocarborane erhalten. Erfindungsgemäß erfolgt die Reinigung der Verbindung durch die Wahl einer geeigneten, wässrigen Base, bevorzugt ausgewählt aus NaOH und KOH.
Durch die Base wird das Thiocarboran als Anion vorteilhaft in die wässrige Phase überführt und so vom Ausgangsstoff, der in protonierter Form in der organischen Phase vorliegt, durch Phasentrennung isoliert. Vorteilhaft kann somit mittels Extraktion unumgesetzter Ausgangsstoff zurückgewonnen werden. Eine lösungsmittel- und daher kostenintensivere säulenchromatographische Reinigung ist somit nicht notwendig.
Bevorzugt wird bei der Reinigung das Rohprodukt in einer wässrigen basischen Lösung (vorzugsweise verdünnte KOH- oder NaOH-Lösung) aufgeschlämmt (oder mehrmals extrahiert, falls das Rohprodukt noch in Lösung vorliegt) und dann mit einem organischen Lösungsmittel (vorzugsweise einem apolaren Lösungsmittel, wie z. B. Diethylether) mehrmals extrahiert. In der organischen Phase befindet sich der Ausgangsstoff. Die wässrigen basischen Phasen werden dann angesäuert (vorzugsweise mit HCl bis etwa pH=1). Hierbei zeigt sich das Produkt als weißer Feststoff. Dieser kann erneut mit einem organischen Lösungsmittel (vorzugsweise einem apolaren Lösungsmittel, wie z. B. Diethylether) extrahiert werden. Nach Entfernen des Lösungsmittels erhält man das entsprechend substituierte Thiocarboran. Sollte das Produkt in ungenügender Reinheit erhalten werden, ist ein Sublimationsschritt möglich.
Durch dieses Verfahren sind alle Thiocarborane und thiolierten borhaltigen Cluster zugänglich.

Vorteilhaft können die beiden Schritte a und b in beliebiger Reihenfolge erfolgen. Auch kann auf eine aufwändige Schutzgruppenstrategie verzichtet werden. Wird z.B. erst eine Hydroxyfunktion eingeführt, so ist es nicht nötig, diese mit einer Schutzgruppe zu versehen, bevor die Verbindung der Hydroxyalkylierung zugeführt wird.
Andersherum ist es auch nicht nötig, die freie Hydroxyfunktion am Rest R4zu schützen, bevor hydroxyliert oder thioliert wird. Damit reduziert sich die Anzahl der Syntheseschritte bedeutend.

### Selektive Veretherung

Ein Bestandteil des erfindungsgemäßen Verfahrens ist neben der möglichen Veresterung auch die selektive Veretherung der direkt an den Cluster gebundenen Hydroxy- oder Thiofunktion H-R₃.

Die Reaktion der Verbindungen aus den beiden Verfahrensschritten a) und b) mit Benzyl- oder Alkylhalogeniden, -tosylaten, -triflaten, und anderen gängigen Alkylierungsreagenzien führt überraschend ausschließlich zur Funktionalisierung der Hydroxy- oder Mercaptofunktion, welche direkt an das Carboran gebunden ist. Hydroxyalkylgruppen Thioalkylgruppen oder Aminoalkylgruppen werden bei diesem Verfahren vorteilhaft nicht funktionalisiert. Dadurch kann auf die aufwändige und kostenintensive Verwendung von Schutzgruppen verzichtet werden. Vorteilhaft können ebenso verschiedene Basen zur Deprotonierung der OH- oder SH-Funktion eingesetzt werden. Auch dann werden die carborangebundenen Hydroxyalkylgruppen nicht verethert. Durch folgendes Veretherungsverfahren wird bevorzugt eine Ether- oder Thioethergruppe am Carboran eingeführt: 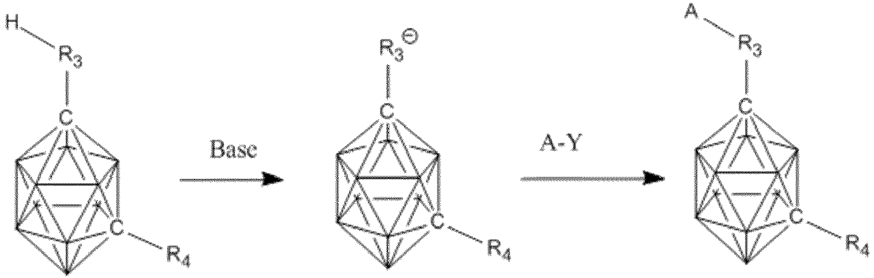 Y = Abgangsgruppe, z.B. F, Cl, Br, I, OTs, OTf

Der erste Schritt ist die Deprotonierung mit einer geeigneten Base, bevorzugt Alkalimetallorganyle, Metallamide, Silazane, Metallhydride oder Ammoniumverbindungen, wie z.B. n-BuLi, MeLi, Lithiumhexamethyldisilazan (LiHMDS), Natriumhexamethyldisilazan (NaHMDS), Kaliumhexamethyldisilazan (KHMDS), Kaliumcarbonat, Lithiumdiisopropylamid (LDA), Tetrabutylammoniumfluorid (TBAF), NaH, NaNH₂ oder Verbindungen mit vergleichbarer Basizität. Der zweite Schritt des Verfahrens ist die nukleophile Substitution von Alkyl- oder Arylhalogeniden durch diese Thio- bzw. Hydroxycarboranylanionen. Erfindungsgemäß erfolgt die Reinigung durch Zugabe protischer Lösungsmittel wie Wasser (dabei können auch Basen, vornehmlich KOH oder NaOH, zugegeben werden) und anschließender Extraktion mit Lösungsmitteln wie Diethylether. Dabei verbleibt der Ausgangsstoff in der wässrigen Phase und das Produkt in der etherischen Phase. Dadurch ist eine teure säulenchromatographische Aufreinigung nicht unbedingt nötig. Nach Entfernen des Lösungsmittels erhält man die Produkte als Feststoffe.

Bislang konnten auf diese Weise nur Thioether des ortho-Carborans sehr aufwändig durch die Verwendung von symmetrisch disubstituierten Disulfiden synthetisiert werden [F. Teixidor, M. A. Flores, C. Viñas, Organometallics, 1999, 18, 5409-5411]. Die hier dargestellte Methode vereinfacht diese Reaktion grundlegend.

Insbesondere umfasst die Erfindung das Verfahren zur selektiven Veretherung von Thio- und Hydroxy-Carboranen in Anwesenheit von carboranylgebundenen freien Hydroxyalkylgruppen. Beispielhaft sei dies anhand einer Hydroxyalkylverbindung dargestellt. 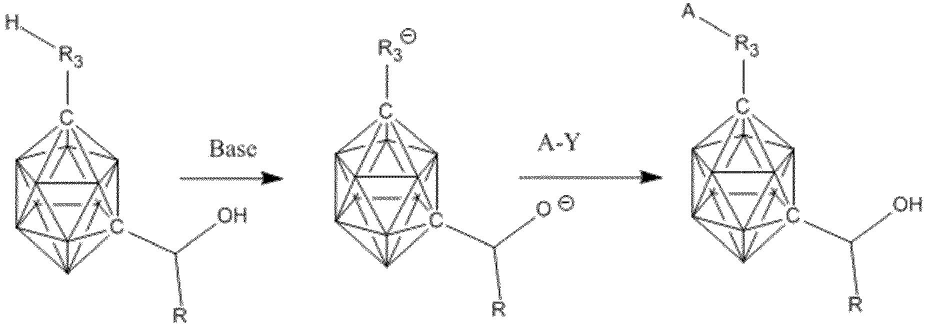 Y = Abgangsgruppe, z.B. F, Cl, Br, I, OTs, OTf und andere

Der erste Schritt ist die Deprotonierung mit einer geeigneten Base, bevorzugt Alkalimetallorganyle, Metallamide, Silazane, Metallhydride oder Ammoniumverbindungen, wie z.B. n-BuLi, MeLi, Lithiumhexamethyldisilazan (LiHMDS), Natriumhexamethyldisilazan (NaHMDS), Kaliumhexamethyldisilazan (KHMDS), Kaliumcarbonat, Lithiumdiisopropylamid (LDA), Tetrabutylammoniumfluorid (TBAF), NaH, NaNH₂ oder Verbindungen mit vergleichbarer Basizität. Der zweite Schritt des Verfahrens ist die nukleophile Substitution von Alkyl- oder Arylhalogeniden, -triflaten, -tosylaten oder anderen Alkylierungsreagenzien durch die Thio- bzw. Hydroxycarboranylanionen. Erfindungsgemäß erfolgt die Reinigung durch Zugabe protischer Lösungsmittel wie Wasser (dabei können auch Säuren, vornehmlich HCl, zugegeben werden) und anschließender Extraktion mit Lösungsmitteln wie Diethylether. Dabei verbleibt das Produkt in der etherischen Phase. Dadurch ist eine teure säulenchromatographische Aufreinigung nicht unbedingt nötig. Nach Entfernen des Lösungsmittels erhält man die Produkte.

Durch dieses Verfahren werden vorteilhaft nur die carboranylgebundenen Thio- bzw. Hydroxygruppen verethert, während carboranylgebundene Hydroxyalkylfunktionen nicht reagieren.

Die erfindungsgemäßen Verbindungen der Struktur

**[A - R₃ - X - R₄]**

mit A = [R₁ - R₂] oder [R₁]
sind damit vorteilhaft und neu gegenüber dem Stand der Technik in maximal drei Reaktionsstufen zugänglich.

Beispielhaft sei das Gesamtverfahren anhand der Synthese der Verbindung CA3 (Ausführungsbeispiel 3), des Carborananalogons von Rev-5901, wie folgt dargestellt:

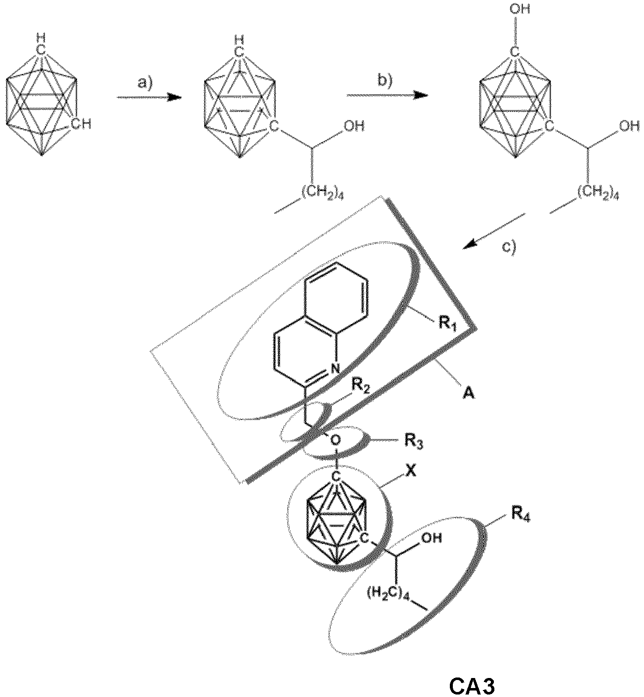

Es sei betont, dass in den erfindungsgemäßen Verbindungen die Hydroxy-, Thio- und Hydroxyalkylgruppen auch an den Boratomen sitzen können.
Vorteilhaft kann nach der Einführung der Reste R3 und R4 auch eine weitere Substitution eines oder mehrerer B-H Wasserstoffatome erfolgen.

Ebenso kann sich einer der Reste R3 oder R4 an einem C-Atom und der jeweils andere an einem Boratom befinden. Beide Reste stehen zueinander in *meta- oder para* - Position.

Erfindungsgemäß kann Z, die freie Hydroxygruppe des Restes R₄, in einem dem erfindungsgemäßen Verfahren nachfolgenden Schritt durch gängige Verfahren mit einem von A verschiedenen Rest verethert oder verestert werden. Auch dies ist Gegenstand der Erfindung.

### Verwendung

Gegenstand der Erfindung ist auch die Verwendung der erfindungsgemäßen Verbindungen für pharmakologische und medizinische Anwendungen.

Die zentrale Position des Clusters in den erfindungsgemäßen Verbindungen nimmt explizit Einfluss auf die metabolischen sowie pharmakokinetischen Eigenschaften der Verbindungen. Die carboransubstituierten Verbindungen weisen eine verbesserte Löslichkeit und höhere metabolische Stabilität bei gleichbleibender inhibitorischer Aktivität gegenüber den phenylsubstituierten Verbindungen auf.
Meta-substituierte Cluster verfügen dabei über eine nochmals gesteigerte metabolische Stabilität als ihre *ortho-* oderpara-Analoga (E. Svantesson, J. Pettersson, A. Olin, K. Markides, S. Sjöberg, Acta Chem. Scand. 1999, 53, 731].).
Dadurch stehen die Verbindungen länger in ihrer aktiven Form im Körper zur Verfügung und besitzen eine verlängerte Wirkdauer, ohne Verlust der inhibitorischen Aktivität.

Weiterhin besitzen die erfindungsgemäßen carboransubstituierten Verbindungen eine deutlich gesteigerte Cytotoxizität gegenüber diversen Krebszelllinien und haben ein deutlich verbreitertes Wirkfenster. Das bedeutet, dass auch deutlich höhere Konzentrationen verwendet werden können, als für das Absterben der Krebszellen eigentlich benötigt würde, ohne gesunde Zellen in Mitleidenschaft zu ziehen.

Die Verwendung dieser Verbindungen oder deren Salze zur Inhibition oder Modulation des LOX-Systems, insbesondere 5-LOX bzw. das 5-Lipoxygenase aktivierende Protein, im Folgenden als FLAP bezeichnet, ist Gegenstand der Erfindung. Hierbei sollen die Größe, Lipophilie und Elektronenzug des Clusters genutzt werden, um die biologischen Eigenschaften der Derivate mitzubestimmen. Die lipophile Natur des Clusters prädestiniert die Verbindung zur Interaktion mit dem membranverankerten LOX-System.

Gegenstand der Erfindung ist ebenfalls die Verwendung der erfindungsgemäßen Verbindungen oder deren Salze für die Imitation von Arachidonsäure und deren Abkömmlingen in biologischen Systemen außerhalb des menschlichen oder tierischen Körpers.
Die Erfindung umfasst die Verwendung der erfindungsgemäßen Verbindungen zur Imitation von Arachidonsäure (AA) und deren Abkömmlingen in biologischen Systemen. Sie umfasst also auch die Verwendung dieser Verbindungen in allen LOX unabhängigen, aber mit AA assoziierten Prozessen oder Systemen. Hierzu zählt unter anderem die Wirkung auf andere Enzyme und Rezeptoren. Bei den Enzymen ist die Cyclooxygenase hervorgehoben, bei den Rezeptoren ist die Familie der GPCRs (G-Protein gekoppelten Rezeptoren), CBs (Cannabinoidrezeptoren), CRTH2 (Chemo attractant Receptor-homologous molecules expressed on TH2 cells) besonders hervorgehoben. Auch nukleare Faktoren, wie z. B. PPARs (Peroxisom-Proliferator-aktivierte Rezeptoren), C/EBP stellen biologische Anwendungsgebiete der beschriebenen Inhibitoren dar.

Mit der Einführung eines Clusters hat man außerdem die Möglichkeit, bekannte, phenylsubstituierte Verbindungen so zu modifizieren, dass auch die biologischen Angriffsziele dieser Verbindungen erweitert werden. Hier ergibt sich nunmehr die Möglichkeit, dass das Cluster-Analogon nicht nur am LOX-System angreift, sondern dass auch andere Organismusbestandteile (Enzyme, Rezeptoren, Nukleinsäuren...) Angriffsstellen bieten.

Gegenstand der Erfindung sind auch die erfindungsgemäßen Verbindungen oder deren Salze zur Verwendung als Arzneimittel, beispielsweise für die Behandlung von mit Arachidonsäure assoziierten Krankheiten.

Gegenstand der Erfindung sind auch die erfindungsgemäßen Verbindungen oder deren Salze zur Verwendung als Arzneimittel, beispielsweise zur Behandlung oder Vorbeugung von Asthma, von Krankheiten des ZNS (Zentrales Nervensystem), Allergien, Rhinitis, Herz-Kreislauf-Erkrankungen, bei der Schmerzvermittlung, der Alzheimer Krankheit, des Sehprozesses, bei Krebsleiden, und Beschwerden in Magen-Darm-, Nieren-, Gefäßbereich und Schwangerschaftsstörungen.

Gegenstand der Erfindung sind auch die erfindungsgemäßen Verbindungen oder deren Salze zur Verwendung in der Behandlung der oben genannten Krankheiten.

Ebenfalls Gegenstand der Erfindung ist die Verwendung der erfindungsgemäßen Verbindungen oder deren Salze zur Herstellung eines Arzneimittels oder Diagnostikum zur Behandlung der oben genannten Krankheiten.
Auch die Verwendung der erfindungsgemäßen Verbindungen für Imagingverfahren sowie die erfindungsgemäßen Verbindungen zur Verwendung für diagnostische oder therapeutische Verfahren, insbesondere in der biologischen Forschung, der Radiologie oder der Nuklearmedizin, sind Gegenstand der Erfindung. Insbesondere seien hier erwähnt:

### BNCT (boron neutron capture therapy)

Die Tatsache, dass die Inhibitoren mit Borclustern einen hohen Borgehalt besitzen, ermöglicht die Anwendung der Verbindungen in der BNCT (boron neutron capture therapy). Die BNCT konzentriert sich bisweilen auf die Behandlung von Krebsleiden. Da das LOX-System bei Krebserkrankungen bedeutende Funktionen besitzt, eignen sich die erfindungsgemäßen Substanzen nicht nur als BNCT- und Tumorimaging-Agentien, sondern auch als Krebsmedikamente. Auch andere Vertreter, die krebsunabhängige Angriffsstellen besitzen, sind mögliche Kandidaten, um mit thermischen Neutronen beschossen zu werden. Generell eigenen sich alle hier erwähnten borhaltigen Verbindungen für die BNCT.

### Weiterer Einsatz der Verbindungen:

Die erfindungsgemäßen Verbindungen zur Verwendung für die BNCS (boron neutron capture synovectomy), bei MRI (magnetic resonance imaging), PET (positron emission tomography), SPECT (single-photon emission computed tomography), PIGE (Particle induced γ-ray emission) und AFM-NIAR (atomic force microscopy with neutron-induced alphaautoradiography) sind ebenfalls Teil der Erfindung.

Des Weiteren ist die Verwendung der erfindungsgemäßen Verbindungen, insbesondere der abgeleiteten *nido-* und Metallacarborane, sowie der radiomarkierten Derivate für Imagingzwecke bzw. diese Verbindungen zur Verwendung für Diagnosezwecke beansprucht.

Gegenstand der Erfindung ist auch eine pharmazeutische Zusammensetzung, enthaltend eine oder mehrere der erfindungsgemäßen Verbindungen bzw. deren Salze. Die aktiven Inhaltsstoffe können mit einem oder mehreren üblichen Trägern, Lösungsmitteln, Verdünnungsmitteln und/oder Grundstoffen vorliegen, um übliche Zubereitungen, wie Tabletten, Pillen, Pulver, Pastillen, Kissen, Kapseln, Elixiere, Suspensionen, Emulsionen, Lösungen, Sirupe, Aerosole (als Feststoff oder in einem flüssigen Medium), Salben, Weich- und Hartgelatinekapseln, Zubereitungen für örtliche Anwendung, steril verpackte Pulver, Mundwässer oder Mundspülungen und dergleichen herzustellen.

Beispiele für geeignete Träger, Grundstoffe und Verdünnungsmittel sind Lactose, Glucose, Saccharose, Sorbitol, Mannitol, Stärken, Akaziengummi, Calciumphosphat, Alginate, Traganth, Gelatine, Calciumsilicat, mikrokristalline Cellulose, Polyvinylpyrrolidon, Cellulose, Wassersirup, Wasser, Wasser/Ethanol, Ethanol, Wasser/Glycol, Wasser/Polyethylen, Glycol, Propylenglycol, Methylcellulose, Methylhydroxybenzoate, Propylhydroxybenzoate, Talkum, Magnesiumstearat, Mineralöl oder fettartige Substanzen, wie Hartfett, oder geeignete Mischungen daraus. Die Zusammensetzungen können zusätzlich Gleit- bzw. Schmiermittel, Netzmittel, Emulgatoren, Suspensionsmittel, Konservierungsstoffe, Süßstoffe, Aroma- bzw. Geschmacksstoffe und dergleichen enthalten. Das Medikament kann so formuliert sein, dass es nach Verabreichung an den Patienten eine schnelle, anhaltende oder verzögerte Freisetzung der aktiven Inhaltsstoffe bereitstellt.

### Ausführungsbeispiele:

Die Erfindung wird nachfolgend durch Ausführungsbeispiele näher erläutert, ohne die Erfindung auf diese zu beschränken.

Hier soll zunächst die Synthese der Clusteranaloga der Ausführungsbeispiele 3 bis 23 sowie der übrigen Vergleichsbeispiele beschrieben sein:
Alle nachfolgend erwähnten Reaktionen wurden in Schlenkgefäßen unter einer Stickstoffschutzatmosphäre durchgeführt. Die Lösungsmittel (Diethylether, THF, CH₂Cl₂, n-Hexan) wurden in einer Lösungsmitteltrocknungsanlage, SPS-800 Series (MBRAUN GmbH) gereinigt, Tetrahydrofuran (THF) wurde über Na/Benzophenon destilliert. Der Essigester und das n-Hexan für die Chromatographie wurden ungereinigt verwendet, wobei n-Hexan als Isomerengemisch verwendet wurde. Die kommerziellen, deuterierten Lösungsmittel (CDCl₃, CD₃COCD₃, CD₃OD und D₂O) wurden unverändert verwendet. Trimethylborat wurde vor Gebrauch destilliert. Meta-Carboran, n-BuLi, LiHMDS, NaHMDS, Kaliumcarbonat und Peressigsäure (Wofasteril®, KESLA PHARMA WOLFEN GmbH) und alle anderen Chemikalien wurden unverändert verwendet. Die IR-Spektren wurden an einem PerkinElmer System 2000 FT-IR Spektrometer in KBr und an einem Autolmage-Mikrosystem mit MCT-Detektor (PerkinElmer) gemessen. Die ¹H-, ¹³C- und ¹¹B-NMR-Spektren wurden an einem AVANCE DRX 400 Spektrometer (Bruker) aufgenommen. Die chemischen Verschiebungen in den ¹H-, ¹³C-, und ¹¹B-NMR-Spektren sind in parts per million (ppm) bei 400,13 MHz, 100,63 MHz, und 161,97 MHz dargestellt. Tetramethylsilan dient als interner Standard für die ersten beiden und BF₃(OEt₂) als externer Standard für die ¹¹B-NMR-Spektren. Die Massenspektren wurden an einem FT-ICR-MS-Bruker-Daltonics ESI Massenspektrometer (APEX II, 7 Tesla) aufgenommen, die Elementaranalysen an einem VARIO EL (Heraeus). Die Schmelzpunkte wurden in Kapillaren (GALLENKAMP) bestimmt. Die Daten für die Röntgenkristallstrukturanalysen wurden auf einem Gemini Diffraktometer (Agilent Technologies) aufgenommen unter Verwendung von Mo-K_{α} Strahlung (λ = 71.073 pm) und ω-scan Rotation. Die Datenreduktion wurde mit CrysAlisPro (CrysAlisPro: Data collection and data reduction software package, Agilent Technologies) sowie dem Programm SCALE3 ABSPACK (SCALE3 ABSPACK: Empirische Absorptionskorrektur unter Verwendung von spherical harmonics) zur empirischen Absorptionskorrektur durchgeführt. Die Strukturen wurden durch direkte Methoden mit SIR92 gelöst. Die Verfeinerung aller Nichtwasserstoffatome wurde mit SHELXL-97 durchgeführt. Die Strukturen wurden mit Diamond und Ortep generiert.

Für die Zeichnungen gilt:
• = B oder BH
∘ = C
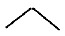 = CH₂ bzw. CR₂

Die Abbildungen der Röntgenstrukturanalysen zeigen jeweils das Ortepbild der Verbindung. mit Schwingungsellipsoiden bei 30% Aufenthaltswahrscheinlichkeit.

### Vergleichsbeispiel 1:

### 1-(1,7-Dicarba-closo-dodecaboran(12)yl)-hexan-1-ol

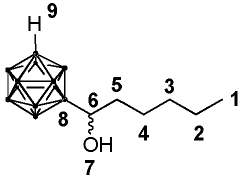

Zu einer Lösung von 4,03 g (27,6 mmol, 1,0 Äq.) 1,7-Dicarba-*closo*-dodecaboran(12) in 80 ml Diethylether werden bei 0 °C langsam 17,7 ml (1,64 M in *n*-Hexan, 29,0 mmol, 1.05 Äq.) einer *n*-Butyllithiumlösung getropft. Nach zwei Stunden werden zu der klaren, farblosen Lösung 3,31 ml (2,76 g, 27,6 mmol, 1,0 Äq.) Hexanal gelöst in 10 ml Diethylether gegeben. Nach 30 Minuten wird die Lösung auf Raumtemperatur erwärmt. Nach 24 Stunden wird die Lösung mit 50 ml destilliertem Wasser versetzt und dreimal mit je 50 ml Diethylether extrahiert. Die organischen Phasen werden vereinigt und mit gesättigter Natriumchloridlösung gewaschen. Es wird über Natriumsulfat getrocknet, das Lösungsmittel unter vermindertem Druck entfernt und der Rückstand über Kieselgel filtriert (Eluent: *n*-Hexan/Ethylacetat, 5:1 v/v). Die erhaltene hochviskose Flüssigkeit wird aus *n*-Hexan umkristallisiert und das Produkt in Form farbloser Plättchen erhalten.

| | |
|---|---|
| Ausbeute: | 6,34 g (25,8 mmol, 94 %) |
| R_{f}-Wert: | 0,64 (Eluent: n-Hexan/Ethylacetat 5:1 *v*/*v*) |
| Schmelzpunkt: | 57,6 - 58,3 °C |

¹H-NMR (CDCl₃): δ = 0,89 (t, 3H, 1-C**H₃**, ³*J*_{HH} = 6,5 Hz), 1,12 - 2,95 (br m, 10H, BH), 1,28 (m, 6H, 2,3,4-C**H₂**), 1,56 (m, 2H, 5-C**H₂**), 1,82 (br s, 1H, 7-OH), 2,92 (br s, 1H, 9-C**H**), 3,75 (br d, 1H, 6-C**H**, ³*J*_{HH} = 10,0 Hz) ppm.

¹¹B{¹H}-NMR (CDCl₃): δ = -16,2 (s, 2B, **B**H), -13,6 (s, 2B, **B**H), -12,2 (s, 2B, **B**H), -11,1 (s, 2B, **B**H), -8,7 (s, 1B, **B**H), -5,1 (s, 1B, **B**H) ppm.

¹³C{¹H}-NMR (CDCl₃): δ = 13,9 (s, 1-**C**H₃), 22,4 (s, 2-**C**H₂), 26,1 (s, 3-**C**H₂), 31,3 (s, 4-**C**H₂), 37,3 (s, 5-**C**H₂), 54,3 (s, 9-**C**H), 72,6 (s, 6-**C**H), 82,7 (s, 8-**C**_{q}) ppm.

Massenspektrometrie (ESI-neg., CH₂Cl₂/CH₃OH):

| | |
|---|---|
| Berechnet: | *m*/*z* = 244,3 |
| Ermittelt: | *m*/*z* = 243,2 (100%, [M - H]⁻), 216,2 (22%, [M- C₂H₅]⁻), 143,0 (5%, [M-C₆H₁₃O]⁻). |

IR-Spektroskopie (KBr, ṽ in cm⁻¹): 3387 (s), 2957 (s), 2928 (s), 2859 (m), 2602 (s), 1461 (w), 1253 (w), 1137 (s), 842 (m), 732 (w).

Elementaranalyse:

| | | |
|---|---|---|
| Berechnet für C₈H₂₄B₁₀O₁: | C = 39,32 % | H = 9,90 % |
| Gefunden: | C = 40,10 % | H = 9,74 % |

### Vergleichsbeispiel 2:

### 1-((7-Hydroxy)-1,7-dicarba-closo-dodecaboran(12)yl)-hexan-1-ol

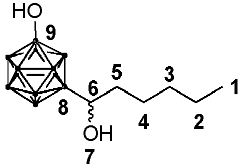

Zu einer Lösung von 0,50 g (2,03 mmol, 1,0 Äq.) 1-(1,7-Dicarba-*closo*-dodecaboran(12)yl)-hexan-1-ol in 20 ml Diethylether werden bei 0 °C 2,7 ml (1,64 M in *n*-Hexan, 4,47 mmol, 2,2 Äq.) einer *n*-Butyllithiumlösung vorsichtig getropft. Die klare Lösung wird anschließend auf Raumtemperatur erwärmt. Nach drei Stunden wird auf -30 °C abgekühlt und 0,28 ml (0,25 g, 2,44 mmol, 1,2 Äq.) Borsäuretrimethylester zugetropft. Die Lösung wird auf Raumtemperatur erwärmt. Innerhalb von 24 Stunden entsteht eine farblose, hochviskose Substanz. Es wird auf 0 °C abgekühlt und mit 2,0 ml (32% in Essigsäure, 0,64 g, 8,41 mmol, 4,1 Äq.) Peressigsäure versetzt. Nach 24 Stunden bei Raumtemperatur werden 2,0 ml konzentrierter Natronlauge zugetropft. Die entstehende klare Lösung wird nach zwei Stunden mit 20 ml destilliertem Wasser versetzt. Es wird dreimal mit je 20 ml Diethylether extrahiert und die vereinigten organischen Phasen mit 20 ml gesättigter Natriumchloridlösung gewaschen. Anschließend wird über Magnesiumsulfat getrocknet und das Lösungsmittel unter vermindertem Druck entfernt. Der Rückstand wird säulenchromatographisch gereinigt (Eluent: *n*-Hexan/Ethylacetat 5:1 *v*/*v*). Es wird ein farbloser Feststoff erhalten.

| | |
|---|---|
| Ausbeute: | 0,27 g (1,05 mmol, 52 %) |
| R_{f}-Wert: | 0,29 (Eluent: *n*-Hexan/Ethylacetat 5:1 *v*/*v*) |
| Schmelzpunkt: | 68,3 - 68,8 °C |

¹H-NMR (CDCl₃): δ = 0,89 (t, 3H, 1-C**H₃**, ³*J*_{HH} = 6,7 Hz), 1,17 - 3,40 (br m, 10H, B**H**), 1,27 (m, 8H, 2,3,4-C**H₂**), 1,55 (m, 2H, 5-C**H₂**), 1,94 (d, 1H, 6-O**H**, ³*J*_{HH} = 5,9 Hz), 3,74 (d, 1H, 6-C**H**, ³*J*_{HH} = 7,8 Hz), 3,91 (s, 1H, 7-O**H**) ppm.

¹¹B{¹H}-NMR (CDCl₃): δ = -14,8 (s, 4B, **B**H), -13,6 (s, 3B, **B**H), -11,6 (s, 2B, **B**H), -6,5 (s, 1B, **B**H) ppm.

¹³C{¹H}-NMR (CDCl₃): δ = 14,0 (s, 1-**C**H₃), 22,5 (s, 2-**C**H₂), 26,1 (s, 4-**C**H₂), 31,3 (s, 3-**C**H₂), 37,3 (s, 5-**C**H₂), 72,5 (s, 6-CH), 79,1 (s, 8-**C**_{q}), 100,9 (s, 9-**C**_{q}) ppm.

Massenspektrometrie (ESI neg., CH₂Cl₂/CH₃OH):

| | |
|---|---|
| Berechnet: | *m*/*z* = 260,3. |
| Ermittelt: | *m*/*z* = 259,2 (100%, [M - H]⁻). |

IR-Spektroskopie (KBr, ṽ in cm⁻¹): 3424 (s), 2959 (m), 2929 (m), 2603 (s), 1635 (m), 1459 (w), 1261 (m), 1205 (m), 1082 (m) 1025 (m), 803 (m).

Elementaranalyse:

| | | |
|---|---|---|
| Berechnet für C₈H₂₄B₁₀O₂: | C = 36,90 % | H = 9,29 % |
| Gefunden: | C = 37,00 % | H = 9,33 % |

### Ausführungsbeispiel 3:

### 1-((7-Chinolin-2-ylmethoxy)-1,7-dicarba-closo-dodeca-boran(12)yl)-hexan-1-ol-Verbindung 1

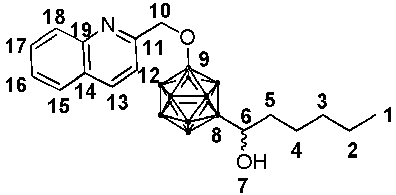

Zu einer Lösung von 0,26 g (1,0 mmol, 1,0 Äq.) 1-(7-Hydroxy-1,7-dicarba-*closo-*dodecaboran(12)yl)-hexan-1-ol in 20 ml trockenem Tetrahydrofuran werden 24,2 mg (1,0 mmol, 1,0 Äq.) Natriumhydrid bei Raumtemperatur gegeben. Die klare Lösung wird nach vier Stunden mit 0,25 g (1,1 mmol, 1,1 Äq.) Chinaldinbromid versetzt und die Lösung 24 Stunden auf 55 °C erwärmt. Anschließend werden 20 ml destilliertes Wasser zugegeben und dreimal mit je 20 ml Diethylether extrahiert. Die vereinigten organischen Phasen werden mit 20 ml gesättigter Natriumchloridlösung gewaschen und über Magnesiumsulfat getrocknet. Das Lösungsmittel wird unter vermindertem Druck entfernt und der hochviskose Rückstand säulenchromatographisch gereinigt (Eluent: *n*-Hexan/Ethylacetat, 5:1, *v*/*v*). Es wird ein farbloser Feststoff erhalten.

| | |
|---|---|
| Ausbeute: | 122 mg (34 %) |
| R_{f}-Wert: | 0,48 (Eluent: *n*-Hexan/Ethylacetat 5:1 *v*/*v*) |
| Schmelzpunkt: | 86,6 - 87,5 °C |

¹H-NMR (CDCl₃): δ = 0,87 (t, 3H, 1-C**H₃**, ³*J*_{HH} = 6,7 Hz), 1,18 - 3,62 (br m, 10H, BH), 1,27 (m, 2H, 3-C**H₂**), 1,28 (m, 2H, 2-C**H₂**), 1,39 (m, 2H, 5-C**H₂**), 1,56 (m, 2H, 4-C**H**₂), 2,47 (brs, 1H, 7-O**H**), 3,75 (br d, 1H, 6-C**H**, ³*J*_{HH} = 8,9 Hz), 4,84 (s, 2H, 10-C**H₂**), 7,44 (d, 1H, 12-C**H**, ³*J*_{HH} = 8,5 Hz), 7,55 (t, 1H, 16-C**H**, ³*J*_{HH} = 8,6 Hz), 7,71 (t, 1H, 17-C**H**, ³*J*_{HH} = 8,5 Hz), 7,80 (d, 1H, 15-C**H**, ³*J*_{HH} = 8,0 Hz), 8,02 (d, 1H, 18-C**H**, ³*J*_{HH} = 8,9 Hz), 8,16 (d, 1H, 13-C**H**, ³*J*_{HH} = 8,5 Hz) ppm.

¹¹B{¹H}-NMR (CDCl₃): δ = -14,9 (s, 4B, **B**H), -13,7 (s, 3B, **B**H), -12,7 (s, 2B, **B**H), -7,8 (s, 1B, **B**H) ppm.

¹³C{¹H}-NMR (CDCl₃): δ = 14,0 (s, 1-**C**H₃), 22,5 (s, 2-**C**H₂), 26,2 (s, 4-**C**H₂), 31,3 (s, 3-**C**H₂), 37,3 (s, 5-**C**H₂), 72,5 (s, 6-CH), 76,5 (s, 10-**C**H₂), 78,4 (s, 8-**C**_{q}), 106,3 (s, 9-**C**_{q}), 119,0 (s, 12-**C**H), 126,7 (s, 16-**C**H), 127,6 (s, 14-**C**_{q}), 127,7 (s, 15-**C**H), 128,9 (s, 18-**C**H), 129,9 (s, 17-**C**H), 137,0 (s, 13-**C**H), 147,2 (s, 19-**C**_{q}), 156,1 (s, 11-**C**_{q}) ppm.

Massenspektrometrie (ESI pos., CH₂Cl₂/CH₃OH):
Berechnet: *m*/*z* = 401,3.
Ermittelt: *m*/*z* = 402,4 (100%, [M + H]⁺), 423,4 (21%, [M + Na]⁺), 440,3 (24%, [M + K]⁺).

IR-Spektroskopie (KBr, ṽ in cm⁻¹): 3165 (m), 2955 (m), 2933 (m), 1601 (w), 1508 (m), 1464 (w), 1429 (w), 1380 (w), 1319 (w), 1197 (s), 1142 (m), 1036 (s), 824 (m), 762 (m), 740 (m), 477 (w).

Elementaranalyse:

| | | |
|---|---|---|
| Berechnet für C₁₈H₃₁B₁₀N₁O₂: | C = 53,84 % | H = 7,78 % N = 3,49 % |
| Gefunden: | C = 54,80 % | H = 7,91 % N = 3,44 % |

Röntgenkristallstrukturanalyse

| | | |
|---|---|---|
| Summenformel | C₁₈ H₃₁ B₁₀ N₁ O₂ | |
| Formelgewicht | 401,54 | |
| Temperatur | 130(2) K | |
| Wellenlänge | 71,073 pm | |
| Kristallsystem | Triklin | |
| Raumgruppe | P -1 | |
| Gitterkonstanten | a = 722,51(7) pm | α = 71,713(7)° |
| | b = 1268,63(8) pm | β = 81,786(7)° |
| | c = 1311,04(11) pm | γ = 83,030(7)° |
| Zellvolumen | 1,12555(17) nm³ | |
| Zahl der Formeleinheiten | 2 | |
| Dichte (berechnet) | 1,185 Mg/m³ | |
| Absorptions koeffizient | 0,067 mm⁻¹ | |
| F(000) | 424 | |
| Größe des Kristalls | 0,7 x 0,2 x 0,05 mm³ | |
| Messbereich von θ | 1.975 bis 32,522°. | |
| Index Bereiche | -10≤h≤10, -18≤k≤18, -19≤ l≤19 | |
| Gemessene Reflexe | 17867 | |
| Unabhängige Reflexe | 7406 [R(int) = 0,0450] | |
| Vollständigkeit bis θ = 30.510° | 100,0 % | |
| Absorptionskorrektur | Semi-empirisch von Äquivalenten | |
| Max. und min. Transmission | 1 und 0,82496 | |
| Verfeinerungsmethode | Full-matrix least-squares on F² | |
| Data / restraints / parameters | 7406 / 76 / 386 | |
| Goodness-of-fit on F² | 1,016 | |
| R Werte [l>2σ(l)] | R1 = 0,0635, wR2 = 0,1412 | |
| R Werte (alle Reflexe) | R1 = 0,1189, wR2 = 0,1686 | |
| Max. und min. Restelektronendichte | 0,296 und -0,237 e.Å⁻³ | |

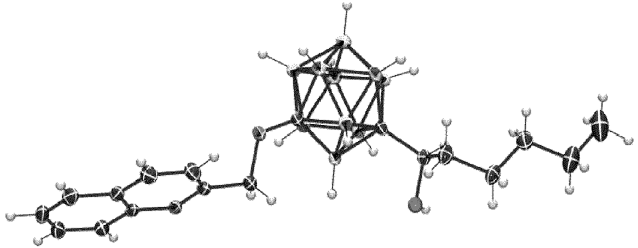

### Vergleichsbeispiel 4:

### 1-(7-(Hexan-1-olyl)-1,7-dicarba-closo-dodecaboran(12)yl)-thiol

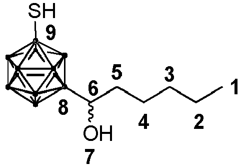

Zu einer Lösung von 0,50 g (2,03 mmol, 1,0 Äq.) 1-(1,7-Dicarba-*closo*-dodecaboran(12)yl)-hexan-1-ol in 20 ml Diethylether werden bei 0 °C 2,7 ml (1,64 M in *n*-Hexan, 4,47 mmol, 2,2 Äq.) einer *n*-Butyllithiumlösung vorsichtig getropft. Die klare Lösung wird anschließend auf Raumtemperatur erwärmt. Nach drei Stunden wird erneut auf 0 °C abgekühlt und 71,5 mg (2,23 mmol, 1,1 Äq.) Schwefel zugegeben. Die Lösung wird auf Raumtemperatur erwärmt und nach 24 Stunden mit 20 ml destilliertem Wasser versetzt. Es wird dreimal mit je 20 ml Diethylether extrahiert und die vereinigten organischen Phasen mit 20 ml gesättigter Natriumchloridlösung gewaschen. Anschließend wird über Magnesiumsulfat getrocknet und das Lösungsmittel unter vermindertem Druck entfernt. Der entstehende hochviskose Rückstand wird aus n-Hexan umkristallisiert und das Produkt als farbloser Feststoff erhalten.

| | |
|---|---|
| Ausbeute: | 0,55 g (98 %) |
| R_{f}-Wert: | 0,65 (Eluent: n-Hexan/Ethylacetat 5:1 *v*/*v*) |
| Schmelzpunkt: | 51,8 - 52,3 °C |

¹H-NMR (CDCl₃): δ = 0,89 (t, 3H, 1-C**H₃**, ³*J*_{HH} = 7,0 Hz),1,10 - 3,20 (m, 10H, B**H**), 1,28 (m, 6H, 2,3,4-C**H₂**), 1,55 (m, 2H, 5-C**H₂**), 1,82 (d, 1H, 7-O**H**, ³*J*_{HH} = 6,9 Hz), 3,39 (s, 1H, 9-S**H**), 3,75 (br t, 1H, 6-C**H**, ³*J*_{HH} = 7,3 Hz) ppm.

¹¹B{¹H}-NMR (CDCl₃): δ = -12,9 (s, 2B, **B**H), -12,1 (s, 2B, **B**H), -10,6 (s, 2B, **B**H), -9,1 (s, 2B, **B**H), -4,0 (s, 1B, **B**H) ppm.

¹³C{¹H}-NMR (CDCl₃): δ = 14,0 (s, 1-**C**H₃), 22,5 (s, 2-**C**H₂), 26,12 (s, 4-**C**H₂), 31,3 (s, 3-**C**H₂), 37,3 (s, 5-**C**H₂), 63,5 (s, 8-**C_{q}**), 72,7 (s, 6-**C**H), 84,2 (s, 9-**C_{q}**) ppm.

Massenspektrometrie (ESI neg., CH₂Cl₂/CH₃OH):

| | |
|---|---|
| Berechnet: | *m*/*z* = 276,3 |
| Ermittelt: | *m*/*z* = 275,3 (100%, [M - H]⁻). |

IR-Spektroskopie (KBr, ṽ in cm⁻¹): 3407 (s), 2957 (m), 2928 (m), 2602 (s), 1639 (m), 1156 (m), 1125 (m), 1078 (m), 735 (m), 611 (w).

Elementaranalyse:

| | | |
|---|---|---|
| Berechnet für C₈H₂₄B₁₀O₁S₁: | C = 34,76 % | H = 8,75 % |
| Gefunden: | C = 34,89 % | H = 8,47 % |

### Ausführungsbeispiel 5

### 1-(7-Thiomethylchinolin-2-yl-1,7-dicarba-closo-dodecaboran-(12)yl)-hexan-1-ol

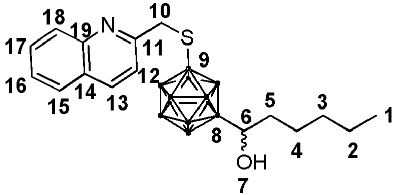

Zu einer Lösung von 220 mg (0,79 mmol, 1,0 Äq.) 1-(7-(Hexan-1-olyl)-1,7-dicarba-*closo-*dodecaboran(12)yl)-thiol in 20 ml Diethylether werden unter inerten Bedingungen bei 0 °C 0,48 ml (1,64 M in *n*-Hexan, 0,79 mmol, 1,0 Äq.) einer *n*-Butyllithiumlösung vorsichtig getropft. Nach 30 Minuten wird die Lösung auf Raumtemperatur erwärmt. Nach drei Stunden wird die Lösung auf -50 °C abgekühlt und mit 175,7 mg (0,79 mmol, 1,0 Äq.) 2-Brommethylchinolin versetzt. Die Lösung wird anschließend auf Raumtemperatur erwärmt. Nach 24 Stunden hat sich ein gelber Niederschlag gebildet. Die Suspension wird mit 20 ml destilliertem Wasser versetzt und anschließend dreimal mit je 20 ml Diethylether extrahiert. Die vereinigten organischen Phasen werden mit 20 ml gesättigter Natriumchloridlösung gewaschen, über Magnesiumsulfat getrocknet und das Lösungsmittel anschließend unter vermindertem Druck entfernt. Der Rückstand wird säulenchromatographisch gereinigt (Eluent: *n*-Hexan/Ethylacetat 5:1 *v*/*v*). Es wird eine leicht gelbliche Flüssigkeit erhalten.

| | |
|---|---|
| Ausbeute: | 166 mg (51 %) |
| R_{f}-Wert: | 0,36 (Eluent: *n*-Hexan/Ethylacetat 5:1 *v*/*v*) |

¹H-NMR (CDCl₃): δ = 0,87 (t, 3H, 1-C**H₃**, ³*J*_{HH} = 7,0 Hz), 1,25 (br m, 4H, 3,4-C**H₂**), 1,26 - 3,47 (br m, 10H, B**H**), 1,34 (m, 2H, 2-C**H₂**), 1,51 (m, 2H, 5-C**H₂**), 2,52 (br s, 2H, 7-O**H**), 3,74 (br t, 1H, 6-C**H**, ³*J*_{HH} = 8,7 Hz), 4,30 (s, 2H, 10-C**H₂**), 7,45 (d, 1H, 12-C**H**, ³*J*_{HH} = 8,7 Hz), 7,52 (t, 1H, 17-C**H**, ³*J*_{HH} = 8,0 Hz), 7,69 (t, 1H, 16-C**H**, ³*J*_{HH} = 8,0 Hz), 7,77 (d, 1H, 15-C**H**, ³*J*_{HH} = 8,7 Hz), 8,04 (d, 1H, 18-C**H**, ³*J*_{HH} = 8,0 Hz), 8,10 (d, 1H, 13-C**H**, ³*J*_{HH} = 8,0 Hz) ppm.

¹¹B{¹H}-NMR (CDCl₃): δ = -13,6 (s, 2**B**, **B**H), -12,2 (s, 2**B**, **B**H), -11,0 (s, 4**B**, **B**H), -7,8 (s, 1**B**, **B**H), -5,4 (s, 1**B**, **B**H) ppm.

¹³C{¹H}-NMR (CDCl₃): δ = 14,0 (s, 1-**C**H₃), 22,5 (s, 2-**C**H₂), 26,2 (s, 4-**C**H₂), 31,1 (s, 3-**C**H₂), 37,3 (s, 5-**C**H₂), 43,2 (s, 10-**C**H₂), 71,2 (s, 9-**C**_{q}), 72,6 (s, 6-**C**H), 83,3 (s, 8-**C**_{q}), 120,8 (s, 12-**C**H), 126,8 (s, 17-**C**H), 127,1 (s, 19-**C**_{q}), 127,6 (s, 15-**C**H), 128,9 (s, 18-**C**H), 129,9 (s, 16-**C**H), 137,0 (s, 13-**C**H), 147,6 (s, 14-**C**H), 155,5 (s, 11-**C**H) ppm.

Massenspektrometrie (ESI pos., CH₂Cl₂/CH₃OH):

| | |
|---|---|
| Berechnet: | *m*/*z* = 417,3. |
| Ermittelt: | *m*/*z* = 418,4 (100%, [M + H]⁺), 441,4 (50%, [M + Na]⁺), 456,3 (45%, [M + K]⁺) |

IR-Spektroskopie (KBr, ṽ in cm⁻¹): 3434 (s), 2963 (m), 2600 (w), 1631 (w), 1262 (s), 1096 (s), 1022 (s), 865 (w), 802 (s), 704 (w).

Elementaranalyse:

| | | |
|---|---|---|
| Berechnet für C₇H₂₀B₁₀O₂: | C = 51,77 % | H = 7,48 % N = 3,35 % |
| Gefunden: | C = 51,60 % | H = 7,25 % N = 3,13 % |

### Vergleichsbeispiel 6:

### 1-(Tetrahydro-2H-pyran-4-olyl)-1,7-dicarba-closo-dodecaboran(12)

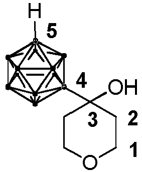

Es werden 2,0 g (13,7 mmol, 1,0 Äq.) 1,7-Dicarba-*closo*-dodecaboran(12) in 60 ml Diethylether gelöst und bei 0 °C mit 9,78 ml (1,54 M in *n*-Hexan, 15,1 mmol, 1,1 Äq.) *n-*Butyllithium versetzt und anschließend auf Raumtemperatur erwärmt. Nach drei Stunden wird die Lösung auf 0 °C abgekühlt und mit 1,27 ml (1,37 g, 13,7 mmol, 1,0 Äq.) Tetrahydro-2H-pyranon versetzt. Anschließend wird auf Raumtemperatur erwärmt. Nach 24 Stunden wird die erhaltene weiße Suspension mit 30 ml destilliertem Wasser versetzt und die entstehenden Phasen getrennt. Die organische Phase wird mit 20 ml 10%iger Natronlauge extrahiert, die vereinigten wässrigen Phasen mit konzentrierter Salzsäure angesäuert und dreimal mit je 20 ml Diethylether extrahiert. Die vereinigten organischen Phasen werden über Magnesiumsulfat getrocknet und das Lösungsmittel unter vermindertem Druck entfernt. Der Rückstand wird aus *n*-Hexan umkristallisiert und das Produkt als farbloser Feststoff erhalten.

| | |
|---|---|
| Ausbeute: | 2,86 g (11,6 mmol, 85 %) |
| R_{f}-Wert: | 0,32 (Eluent: n-Hexan/Ethylacetat 5:2 *v*/*v*) |
| Schmelzpunkt: | 109,0 - 110,2 °C |

¹H-NMR (CDCl₃): δ = 1,50-3,20 (br m, 10H, B**H**), 1,53 (d, 2H, Sessel-2-C**H₂**, ³*J*_{HH} = 13,3 Hz), 1,82 (dt, 2H, Wanne-2-C**H₂**, ³*J*_{HH} = 13,0 Hz), 2,92 (brs, 1H, 5-C**H**), 3,66 (t, 2H, Sessel-1-C**H₂**, ³*J*_{HH} = 12,2 Hz), 3,80 (dd, 2H, Wanne-1-C**H₂**, ³*J*_{HH} = 11,0 Hz) ppm.

¹¹B{¹H}-NMR (CDCl₃): δ = -15,8 (s, 5B, **B**H), -13,1 (s, 2B, **B**H), -11,3 (s, 2B, **B**H), -4,6 (s, 1B, **B**H) ppm.

¹³C{¹H}-NMR (CDCl₃): δ = 39,3 (s, 2-**C**H₂), 53,9 (s, 5-CH), 63,8 (s, 1-**C**H₂), 69,3 (s, 3-**C**_{q}), 69,4 (s, 5-**C**_{q}) ppm.

Massenspektrometrie (ESI neg., Aceton):

| | |
|---|---|
| Berechnet: | *m*/*z* = 244,3 |
| Ermittelt: | *m*/*z* = 243,2 (100%, [M - H]⁻), 216,2 (22%, [M - C₂H₅]⁻). |

IR-Spektroskopie (KBr, ṽ in cm⁻¹): 3430 (s), 3062 (w), 2964 (w), 2934 (w), 2864 (w), 2600 (s), 1468 (w), 1387 (w), 1356 (w), 1159 (m), 1123 (m), 1022 (m) 852 (w), 732 (w) 550 (m).

Elementaranalyse:

| | | |
|---|---|---|
| Berechnet für C₇H₂₀B₁₀O₂: | C = 34,41 % | H = 8,25 % |
| Gefunden: | C = 34,28 % | H = 8,27 % |

### Vergleichsbeispiel 7:

### 1-Mercapto-7-(tetrahydro-2H-pyran-4-olyl)-1,7-dicarba-closo-dodecaboran(12)

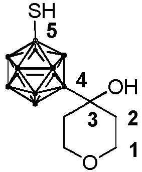

Es werden 1,5 g (8,42 mmol, 1,0 Äq.) 1-Thio-1,7-dicarba-*closo*-dodecaboran(12) in 50 ml Diethylether gelöst und bei 0 °C mit 11,50 ml (1,54 M in *n*-Hexan, 17,71 mmol, 2,1 Äq.) *n-*Butyllithium versetzt und anschließend auf Raumtemperatur erwärmt. Nach drei Stunden wird die erhaltene Suspension auf 0 °C abgekühlt und mit 0,78 ml (0,84 g, 8,42 mmol, 1,0 Äq.) Tetrahydro-2*H*-pyranon versetzt. Anschließend wird auf Raumtemperatur erwärmt. Nach 24 Stunden wird die erhaltene weiße Suspension mit 20 ml destilliertem Wasser versetzt, mit konzentrierter Salzsäure angesäuert und dreimal mit je 20 ml Diethylether extrahiert. Die vereinigten organischen Phasen werden über Magnesiumsulfat getrocknet und das Lösungsmittel unter vermindertem Druck entfernt. Der Rückstand wird auf Kieselgel aufgetragen und das Edukt abgetrennt (Eluent: n-Hexan/Ethylacetat 10:1 *v*/*v*). Anschließend wird das Produkt mit Ethylacetat eluiert, das Lösungsmittel unter vermindertem Druck entfernt und der Rückstand aus *n*-Hexan/Methanol (20:1 *v*/*v*) umkristallisiert. Es wird ein farbloser Feststoff erhalten.

| | |
|---|---|
| Ausbeute: | 1,15 g (4,13 mmol, 49 %) |
| R_{f}-Wert: | 0,32 (Eluent: n-Hexan/Ethylacetat 5:2 *v*/*v*) |
| Schmelzpunkt: | 91,2 - 92,3 °C |

¹H-NMR (CDCl₃): δ = 1,45-3,80 (br m, 10H, B**H**), 1,53 (d, 2**H**, Sessel-2-C**H₂**, ³*J*_{HH} = 13,8 Hz), 1,61 (br s, 1H, 5-S**H**), 1,83 (dt, 2H, Wanne-2-C**H₂**, ³*J*_{HH} = 13,1 Hz), 3,66 (dt, 2H, Sessel-1-C**H₂**, ³*J*_{HH} = 11,6 Hz), 3,81 (dd, 2H, Wanne-1-C**H₂**, ³*J*_{HH} = 11,6 Hz) ppm.

¹¹B{¹H}-NMR (CDCl₃): δ = -12,8 (s, 2B, **B**H), -12,4 (s, 2B, **B**H), -10,5 (s, 2B, **B**H),-9,0 (s, 3B, **B**H),-3,2 (s, 1B, **B**H) ppm.

¹³C{¹H}-NMR (CDCl₃): δ = 39,3 (s, 2-**C**H₂), 42,8 (s, 5-**C**_{q}), 63,7 (s, 1-**C**H₂), 67,8 (s, 4-**C**_{q}), 69,5 (s, 3-**C**_{q}) ppm.

Massenspektrometrie (ESI neg., CHCl₃, CH₃OH):

| | |
|---|---|
| Berechnet: | *m*/*z* = 276,2 |
| Ermittelt: | *m*/*z* = 275,2 (100%, [M -H]⁻). |

IR-Spektroskopie (KBr, ṽ in cm⁻¹): 3347 (s), 2974 (m), 2952 (s), 2941 (m), 2865 (s), 2634 (s), 2571 (s), 2557 (s), 1406 (m), 1388 (m), 1355 (m), 1305 (m), 1249 (m), 1161 (s), 1138 (s), 1102 (s), 1019 (s), 973 (m), 849 (m), 739 (w), 553 (s).

Elementaranalyse:

| | | |
|---|---|---|
| Berechnet für C₇H₂₀B₁₀O₂S₁: | C = 30,42 % | H = 7,29 % |
| Gefunden: | C = 30,68 % | H = 7,31 % |

Röntgenkristallstrukturanalyse

| | | |
|---|---|---|
| Summenformel | C₈ H₂₄ B₁₀ O₃ S₁ | |
| Formelgewicht | 308,43 | |
| Temperatur | 130(2) K | |
| Wellenlänge | 71,073 pm | |
| Kristallsystem | Triklin | |
| Raumgruppe | P -1 | |
| Gitterkonstanten | a = 760,23(5) pm | α = 85,472(4)° |
| | b = 969,71(5) pm | β = 84,192(4)° |
| | c = 2324,27(10) pm | γ = 73,010(5)° |
| Zellvolumen | 1,62807(16) nm³ | |
| Zahl der Formeleinheiten | 4 | |
| Dichte (berechnet) | 1,258 Mg/m³ | |
| Absorptions koeffizient | 0,197 mm⁻¹ | |
| F(000) | 648 | |
| Größe des Kristalls | 0,4 x 0,1 x 0,1 mm³ | |
| Messbereich von θ | 2,199 bis 30,512°. | |
| Index Bereiche | -10≤h≤10, -13≤k≤13, -31≤ | l≤32 |
| Gemessene Reflexe | 29901 | |
| Unabhängige Reflexe | 8982 [R(int) = 0,0531] | |
| Vollständigkeit bis θ = 28.285° | 100,0 % | |
| Absorptionskorrektur | Semi-empirisch von Äquivalenten | |
| Max. und min. Transmission | 1 und 0,97336 | |
| Verfeinerungsmethode | Full-matrix least-squares on F² | |
| Data / restraints / parameters | 8982 / 0 / 589 | |
| Goodness-of-fit on F² | 1,019 | |
| R Werte [l>2σ(l)] | R1 = 0,0505, wR2 = 0,1025 | |
| R Werte (alle Reflexe) | R1 = 0,0855, wR2 = 0,1155 | |
| Extinction coefficient | n/a | |
| Max. und min. Restelektronendichte | 0,354 und -0,307 e.Å⁻³ | |

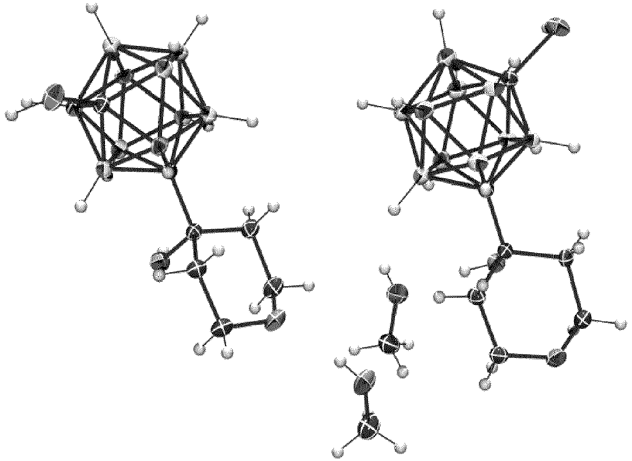

### Ausführungsbeispiel 8:

### 1-(2-Mercaptomethyl)chinolyl-7-(tetrahydro-2H-pyran-4-olyl)-1,7-dicarba-closo-dodecaboran(12)

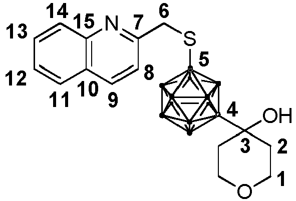

Zu einer Lösung von 1-Mercapto-7-(tetrahydro-2*H*-pyran-4-olyl)-1,7-dicarba-*closo-*dodecaboran(12) (0,3 g, 1,08 mmol, 1,0 Äq.) und 2-Brommethylchinolin (240,0 mg, 1,08 mmol, 1,0 Äq.) in 30 ml Aceton werden bei Raumtemperatur 450,0 mg (3,24 mmol, 3,0 Äq.) Kaliumcarbonat gegeben und anschließend 24 Stunden unter Rückfluss erhitzt. Nach Abkühlen auf Raumtemperatur werden 20 ml destilliertes Wasser sowie 10 ml gesättigte Natriumchloridlösung zugegeben. Es wird dreimal mit je 20 ml Diethylether extrahiert, die organischen Phasen vereinigt, über Magnesiumsulfat getrocknet und das Lösungsmittel unter vermindertem Druck entfernt. Das Rohprodukt wird säulenchromatographisch gereinigt (Eluent: *n*-Hexan/Ethylacetat, 5:1 *v*/*v*). Das Produkt wird als leicht gelblicher Feststoff erhalten.

| | |
|---|---|
| Ausbeute: | 246 mg (0,59 mmol, 54 %) |
| R_{f}-Wert: | 0,20 (Eluent: *n*-Hexan/Ethylacetat 5:1 *v*/*v*) |
| Schmelzpunkt: | 91,8 - 92,5 °C |

¹H-NMR (CDCl₃): δ = 1,20-3,70 (br m, 10H, B**H**), 1,49 (d, 2H, Sessel-2-C**H₂**, ³*J*_{HH} = 13,3 Hz), 1,76 (dt, 2H, Wanne-2-C**H₂**, ³*J*_{HH} = 12,8 Hz), 1,93 (s, 1H, 3-O**H**), 3,63 (t, 2H, Sessel-1-C**H₂**, ³*J*_{HH} = 11,1 Hz), 3,76 (dd, 2H, Wanne-1-C**H₂**, ³*J*_{HH} = 11,6 Hz), 4,30 (s, 2H, 6-C**H₂**), 7,46 (d, 1H, 8-C**H**, ³*J*_{HH} = 8,5 Hz), 7,53 (t, 1H, 12-C**H**, ³*J*_{HH} = 8,0 Hz), 7,71 (t, 1H, 13-C**H**, ³*J*_{HH} = 7,4 Hz), 7,79 (d, 2H, 11-C**H**, ³*J*_{HH} = 8,4 Hz), 8,05 (d, 1H, 14-C**H**, ³*J*_{HH} = 8,4 Hz), 8,13 (d, 1H, 9-C**H**, ³*J*_{HH} = 8,5 Hz) ppm.

¹¹B{¹H}-NMR (CDCl₃): δ = -13,6 (s, 2B, **B**H), -12,6 (s, 2B, **B**H), -11,0 (s, 4B, **B**H), -8,2 (s, 1B, **B**H), - 4,6 (s, 1B, **B**H) ppm.

¹³C{¹H}-NMR (CDCl₃): δ = 39,3 (s, 2-**C**H₂), 43,2 (s, 6-**C**H₂), 63,8 (s, 1-**C**H₂), 69,4 (s, 3-**C**_{q}), 70,9 (s, 5-**C**_{q}), 88,4 (s, 4-**C**_{q}), 120,8 (s, 8-**C**H), 126,8 (s, 12-**C**H), 127,2 (s, 10-**C**_{q}), 127,6 (s, 11-**C**H), 129,0 (s, 14-**C**H), 129,9 (s, 13-**C**H), 137,0 (s, 9-**C**H), 147,6 (s, 15-**C**_{q}), 155,6 (s, 7-**C**_{q}) ppm.

Massenspektrometrie (ESI neg., CH₂Cl₂, CH₃CN):

| | |
|---|---|
| Berechnet: | *m*/*z* = 417,3 |
| Ermittelt: | *m*/*z* = 416,3 (100%, [M - H]⁻). |

IR-Spektroskopie (KBr, ṽ in cm⁻¹): 3427 (s), 2962 (w), 2870 (w), 2606 (s), 1619 (m), 1599 (m), 1505 (m), 1427 (m), 1304 (w), 1244 (w), 1160 (m), 1019 (m), 822 (m), 766 (m), 547 (m).

Elementaranalyse:

| | | | |
|---|---|---|---|
| Berechnet für C₁₇H₂₇B₁₀N₁O₂S₁: | C = 48,90 % | H = 6,52 % | N = 3,35 % |
| Gefunden: | C = 50,64 % | H = 6,53 % | N = 3,60 % |

Röntgenkristallstrukturanalyse

| | | |
|---|---|---|
| Summenformel | C₁₇ H₂₇ B₁₀ N₁ O₂ S₁ | |
| Formelgewicht | 417,55 | |
| Temperatur | 130(2) K | |
| Wellenlänge | 71,073 pm | |
| Kristallsystem | Monoklin | |
| Raumgruppe | P 21/n | |
| Gitterkonstanten | a = 964,84(4) pm | α = 90°. |
| | b = 1816,00(6) pm | β = 102,951(3)° |
| | c = 1270,03(4) pm | γ = 90°. |
| Zellvolumen | 2,16868(14) nm³ | |
| Zahl der Formeleinheiten | 4 | |
| Dichte (berechnet) | 1,279 Mg/m³ | |
| Absorptions koeffizient | 0,165 mm⁻¹ | |
| F(000) | 872 | |
| Größe des Kristalls | 0,4 x 0,15 x 0,1 mm³ | |
| Messbereich von θ | 1,991 bis 32,567°. | |
| Index Bereiche | -14≤h≤14, -27≤k≤26, -19≤l≤18 | |
| Gemessene Reflexe | 47840 | |
| Unabhängige Reflexe | 7494 [R(int) = 0,0477] | |
| Vollständigkeit bis θ = 30.510° | 100,0 % | |
| Absorptionskorrektur | Semi-empirisch von Äquivalenten | |
| Max. und min. Transmission | 1 und 0,96721 | |
| Verfeinerungsmethode | Full-matrix least-squares on F² | |
| Data / restraints / parameters | 7494/0/388 | |
| Goodness-of-fit on F² | 1,013 | |
| R Werte [l>2σ(l)] | R1 = 0,0436, wR2 = 0,0932 | |
| R Werte (alle Reflexe) | R1 = 0,0663, wR2 = 0,1018 | |
| Max. und min. Restelektronendichte | 0,307 und -0,384 e.Å⁻³ | |

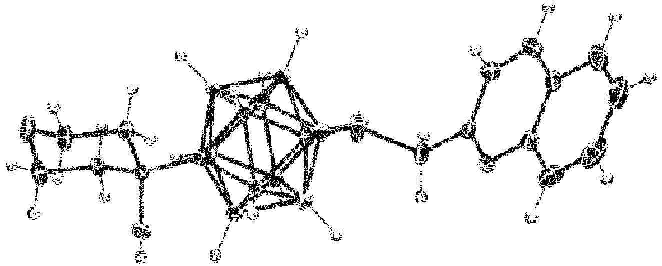

### Ausführungsbeispiel 9:

### 1-(2-Mercaptomethyl)naphthyl-7-(tetrahydro-2H-pyran-4-olyl)-1,7-dicarba-closo-dodecaboran(12) - Verbindung 2

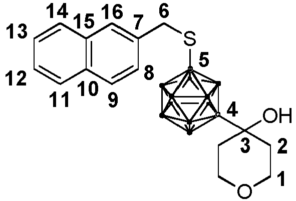

Zu einer Lösung von 1-Mercapto-7-(tetrahydro-2*H*-pyran-4-olyl)-1,7-dicarba-*closo-*dodecaboran(12) (0,25 g, 0,90 mmol, 1,0 Äq.) und 2-Brommethylnaphthalin (197,0 mg, 0,90 mmol, 1,0 Äq.) in 50 ml Aceton warden bei Raumtemperatur 373,0 mg (2,70 mmol, 3,0 Äq.) Kaliumcarbonat gegeben und anschließend 24 Stunden unter Rückfluss erhitzt. Nach Abkühlen auf Raumtemperatur werden 20 ml destilliertes Wasser sowie 10 ml gesättigte Natriumchloridlösung zugegeben. Es wird dreimal mit je 20 ml Diethylether extrahiert, die organischen Phasen vereinigt, über Magnesiumsulfat getrocknet und das Lösungsmittel unter vermindertem Druck entfernt. Das Rohprodukt wird säulenchromatographisch gereinigt (Eluent: *n*-Hexan/Ethylacetat, 5:1 *v*/*v*). Das Produkt wird als farbloser Feststoff erhalten.

| | |
|---|---|
| Ausbeute: | 184 mg (0,44 mmol, 49 %) |
| R_{f}-Wert: | 0,44 (Eluent: *n*-Hexan/Ethylacetat 5:2 *v*/*v*) |
| Schmelzpunkt: | 100,5 - 101,3 °C |

¹H-NMR (CDCl₃): δ = 1,10-3,70 (br m, 10H, B**H**), 1,52 (d, 2H, Sessel-2-C**H₂**, ³*J*_{HH} = 13,4 Hz), 1,63 (br s, 1H, 3-O**H**), 1,82 (dt, 2H, Wanne-2-C**H₂**, ³*J*_{HH} = 13,1 Hz), 3,67 (dt, 2H, Sessel-1-C**H₂**, ³*J*_{HH} = 11,6 Hz), 3,81 (dd, 2H, Wanne-1-C**H₂**, ³*J*_{HH} = 11,6 Hz), 4,14 (s, 2H, 6-C**H₂**), 7,39 (d, 1H, 8-C**H**, ³*J*_{HH} = 8,5 Hz), 7,48 (t, 1H, 13-C**H**, ³*J*_{HH} = 9,0 Hz), 7,49 (t, 1H, 12-C**H**, ³*J*_{HH} = 9,7 Hz), 7,75 (s, 1H, 16-C**H**), 7,80 (d, 1H, 9-C**H**, ³*J*_{HH} = 8,5 Hz), 7,81 (d, 1H, 14-C**H**, ³*J*_{HH} = 8,4 Hz), 7,82 (d, 1H, 11-C**H**, ³*J*_{HH} = 8,5 Hz) ppm.

¹¹B{¹H}-NMR (CDCl₃): δ = -13,6 (s, 2B, **B**H), -12,6 (s, 2B, **B**H), -10,9 (s, 4B, **B**H), -8,2 (s, 1B, **B**H), -4,5 (s, 1B, **B**H) ppm.

¹³C{¹H}-NMR (CDCl₃): δ = 39,3 (s, 2-**C**H₂), 41,1 (s, 6-**C**H₂), 63,7 (s, 1-**C**H₂), 69,4 (s, 3-**C**_{q}), 71,3 (s, 5-**C**_{q}), 88,0 (s, 4-**C**_{q}), 126,2 (s, 12-**C**H), 126,3 (s, 13-**C**H), 126,8 (s, 8-**C**H), 127,6 (s, 9-**C**H, 14-**C**H), 128,2 (s, 16-**C**H), 128,5 (s, 11-**C**H), 131,6 (s, 7-**C**_{q}), 132,7 (s, 10-**C**_{q}), 133,2 (s, 15-**C**_{q}) ppm.

Massenspektrometrie (ESI neg., Aceton):

| | |
|---|---|
| Berechnet: | *m*/*z* = 416,3 |
| Ermittelt: | *m*/*z* = 415,3 (100%, [M - H]⁻), 452,2 (50%, [M + Cl]⁻). |

IR-Spektroskopie (KBr, ṽ in cm⁻¹): 3472 (s), 2957 (s), 2979 (m), 2957 (m), 2918 (m), 2865 (m), 2598 (s), 1508 (w), 1464 (w), 1437 (w), 1390 (m), 1361 (m), 1303 (m), 1243 (m), 1159 (s), 1124 (s), 1102 (s) 1019 (s), 973 (m), 848 (m), 822 (s), 755 (s), 549 (m), 472 (m).

Elementaranalyse:

| | | |
|---|---|---|
| Berechnet für C₁₈H₂₈B₁₀O₂S₁: | C = 51,90 % | H = 6,77 % |
| Gefunden: | C = 53,23 % | H = 6,92 % |

Röntgenkristallstrukturanalyse

| | | |
|---|---|---|
| Summenformel | C₁₈ H₃₁ B₁₀ O_{3,5} S₁ | |
| Formelgewicht | 443,59 | |
| Temperatur | 130(2) K | |
| Wellenlänge | 71,073 pm | |
| Kristallsystem | Monoklin | |
| Raumgruppe | P 21/c | |
| Gitterkonstanten | a = 1428,01 (4) pm | α = 90°. |
| | b = 660,10(2) pm | β = 94,490(3)° |
| | c = 5004,4(2) pm | γ = 90°. |
| Zellvolumen | 4,7028(3) nm³ | |
| Zahl der Formeleinheiten | 8 | |
| Dichte (berechnet) | 1,253 Mg/m³ | |
| Absorptions koeffizient | 0,160 mm⁻¹ | |
| F(000) | 1864 | |
| Größe des Kristalls | 0,4 x 0,1 x 0,02 mm³ | |
| Messbereich von θ | 2,085 bis 27,024°. | |
| Index Bereiche | -18≤h≤16, -8≤k≤8, -61≤l≤62 | |
| Gemessene Reflexe | 37852 | |
| Unabhängige Reflexe | 9424 [R(int) = 0,0725] | |
| Vollständigkeit bis θ = 25.350° | 99,9 % | |
| Absorptionskorrektur | Semi-empirisch von Äquivalenten | |
| Max. und min. Transmission | 1 und 0,93162 | |
| Verfeinerungsmethode | Full-matrix least-squares on F² | |
| Data / restraints / parameters | 9424 / 96 / 696 | |
| Goodness-of-fit on F² | 1,030 | |
| R Werte [l>2σ(l)] | R1 = 0,0632, wR2 = 0,1255 | |
| R Werte (alle Reflexe) | R1 = 0,1183, wR2 = 0,1476 | |
| Max. und min. Restelektronendichte | 0,597 und -0.434 e.Å⁻³ | |

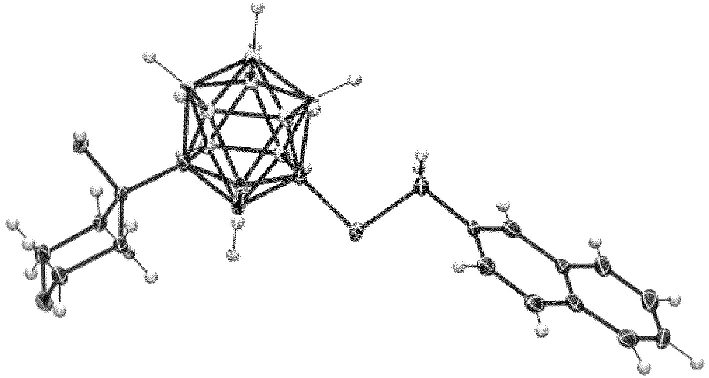

### Vergleichsbeispiel 12a:

### 1-Cyclopentylhydroxymethyl-1,7-dicarba-closo-dodecaboran(12)

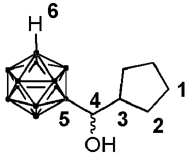

Zu einer Lösung von 1,00 g (6,85 mmol, 1,0 Äq.) 1,7-Dicarba-*closo*-dodecaboran(12) in 50 ml Diethylether werden bei 0 °C langsam 5,0 ml (1,45 M in *n*-Hexan, 7,19 mmol, 1,05 Äq.) einer *n*-Butyllithiumlösung getropft. Nach 30 Minuten wird die Lösung auf Raumtemperatur erwärmt. Nach drei Stunden wird die Lösung erneut auf 0 °C abgekühlt und 0,62 ml (0,67 mg, 6,85 mmol, 1,0 Äq.) Cyclopentylaldehyd zugetropft. Die Lösung wird anschließend auf Raumtemperatur erwärmt. Nach 24 Stunden wird die weiße Suspension mit 20 ml destilliertem Wasser versetzt, mit konzentrierter Salzsäure angesäuert und anschließend dreimal mit je 20 ml Diethylether extrahiert. Die vereinigten organischen Phasen werden mit 20 ml gesättigter Natriumchloridlösung gewaschen, über Magnesiumsulfat getrocknet und das Lösungsmittel anschließend unter vermindertem Druck entfernt. Der Rückstand wird säulenchromatographisch gereinigt (Eluent: Hexan/Ethylacetat 5:1 *v*/*v*). Es wird ein weißer Feststoff erhalten.

| | |
|---|---|
| Ausbeute: | 0,5 g (2,06 mmol, 30,1 %) |
| R_{f}-Wert: | 0,74 (Eluent: Hexan/Ethylacetat 5:1 *v*/*v*) |
| Schmelzpunkt: | 50,2 - 51,3 °C |

¹H-NMR (CDCl₃): δ = 1,25 - 3,10 (br m, 10H, B**H**), 1,36 (m, 2H, 2-C**H₂ₐ**), 1,46 (m, 2H, 1-C**H₂ₐ**), 1,66 (m, 2H, 2-C**H_{2b}**), 1,78 (m, 2H, 1-C**H_{2b}**), 1,94 (brs, 1H, 4-O**H**), 2,08 (dt, 1H, 3-C**H**, ³*J*_{HH} = 8,2 Hz, ³*J*_{HH} =3,6 Hz), 2,92 (brs, 1H, 6-C**H**), 3,81 (d, 1H, 4-C**H**, ³*J*_{HH} = 3,6 Hz) ppm.

¹¹B{¹H}-NMR (CDCl₃): δ = -16,0 (s, 2B, **B**H), -13,6 (s, 2B, **B**H), -11,9 (s, 2B, **B**H), -11,0 (s, 2B, **B**H), -8,5 (s, 1B, **B**H),-4,9 (s, 1B, **B**H) ppm.

¹³C{¹H}-NMR (CDCl₃): δ = 25,5 (s, 1-**C**H₂), 26,0 (s, 2-**C**H₂), 31,8 (s, 2-**C**H₂), 44,6 (s, 3-CH), 54,5 (s, 6-**C**H), 75,5 (s, 4-**C**H), 82,6 (s, 5-**C**_{q}) ppm.

Massenspektrometrie (ESI-neg., CH₂Cl₂/CH₃CN):

| | |
|---|---|
| Berechnet: | *m*/*z* = 242,3 |
| Ermittelt: | *m*/*z* = 241,2 (100%, [M - H]⁻), 278,2 (50%, [M + Cl]⁻) |

IR-Spektroskopie (KBr, ṽ in cm⁻¹): 3591 (m), 3486 (s), 3060 (m), 2955 (s), 2868 (m), 2603 (s), 1450 (w), 1385 (m), 1131 (s), 1009 (m), 817 (w), 732 (m), 707 (w), 669 (w), 608 (w).

Elementaranalyse:

| | | |
|---|---|---|
| Berechnet für C₈H₂₂B₁₀O₁: | C = 39,64 % | H = 9,15 % |
| Gefunden: | C = 39,77 % | H = 9,10 % |

### Vergleichsbeispiel 12b:

### 1-Cyclopentylhydroxymethyl-7-thio-1,7-dicarba-closo-dodecaboran(12)

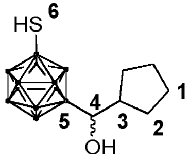

Zu einer Lösung von 425,0 mg (1,76 mmol, 1,0 Äq.) 1-Cyclopentylhydroxymethyl-1,7-dicarba-*closo*-dodecaboran(12) in 50 ml Diethylether werden bei 0 °C langsam 2,54 ml (1,45 M in *n-*Hexan, 3,69 mmol, 2,1 Äq.) einer n-Butyllithiumlösung getropft und nach 30 Minuten wird die Lösung auf Raumtemperatur erwärmt. Nach drei Stunden wird die Lösung erneut auf 0 °C abgekühlt, es werden 56,3 mg (1,76 mmol, 1,0 Äq.) Schwefel zugegeben und anschließend auf Raumtemperatur erwärmt. Nach 24 Stunden wird die weiße Suspension mit 50 ml destilliertem Wasser versetzt, mit konzentrierter Salzsäure angesäuert und dreimal mit je 50 ml Diethylether extrahiert. Die organischen Phasen werden vereinigt und mit gesättigter Natriumchloridlösung gewaschen. Es wird über Magnesiumsulfat getrocknet, das Lösungsmittel unter vermindertem Druck entfernt und der Rückstand säulenchromatographisch gereinigt (Eluent: Hexan/Ethylacetat, 5:1 v/v). Es wird eine leicht gelbliche, hochviskose Flüssigkeit erhalten.

| | |
|---|---|
| Ausbeute: | 6,34 g (25,8 mmol, 94 %) |
| R_{f}-Wert: | 0,64 (Eluent: Hexan/Ethylacetat 5:1 *v*/*v*) |
| Schmelzpunkt: | 57,6 - 58,3 °C |

¹H-NMR (CDCl₃): δ = 1,15 - 3,20 (br m, 10H, B**H**), 1,35 (t, 2H, 2-C**H₂ₐ**, ³*J*_{HH} = 8,7 Hz), 1,46 (m, 2H, 1-C**H₁ₐ**), 1,65 (m, 2H, 1-C**H**_{2b}), 1,78 (m, 2H, 1-C**H_{1b}**), 2,09 (m, 1H, 3-CH),3,81 (br d, 1H, 4-C**H**, ³*J*_{HH} = 3,2 Hz) ppm.

¹¹B{¹H}-NMR (CDCl₃): δ = -14,8 (s, 2B, **B**H), -11,5 (s, 1B, **B**H), -10,6 (s, 2B, **B**H), -9,2 (s, 2B, **B**H),-6,6 (s, 2B, **B**H), -3,8 (s, 1B, **B**H) ppm.

¹³C{¹H}-NMR (CDCl₃): δ = 25,5 (s, 1-**C**H₂), 25,6 (s, 2-**C**H₂), 26,0 (s, 5-**C**_{q}), 31,9 (s, 6-**C_{q}**), 44,5 (s, 3-CH), 75,6 (s, 4-CH) ppm.

Massenspektrometrie (ESI-neg., CH₂Cl₂/CH₃CN):

| | |
|---|---|
| Berechnet: | *m*/*z* = 274,2 |
| Ermittelt: | *m*/*z* = 273,2 (100%, [M - H]⁻) |

IR-Spektroskopie (KBr, ṽ in cm⁻¹): 3448 (m), 2952 (s), 2867 (m), 2598 (s), 1451 (w), 1389 (w), 1462 (m), 1093 (m), 1028 (m), 939 (w), 808 (w), 737 (w).

Elementaranalyse:

| | | |
|---|---|---|
| Berechnet für C₈H₂₂B₁₀O₁S₁: | C = 35,01 % | H = 8,08 % |
| Gefunden: | C = 44,18 % | H = 8,07 % |

### Ausführungsbeispiel 12:

### 1-Cyclopentylhydroxymethyl-7-((chinolin-2-ylmethyl)thio)-1,7-dicarba-closo-dodecaboran(12)

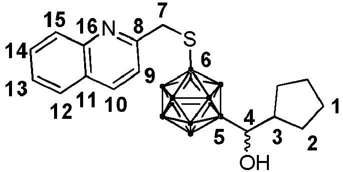

Zu einer Lösung von 1-Cyclopentylhydroxymethyl-7-thio-1,7-dicarba-*closo*-dodecaboran(12) (0,3 g, 1,10 mmol, 1,0 Äq.) und 2-Brommethylchinolin (265,0 mg, 1,20 mmol, 1,2 Äq.) in 20 ml Aceton werden bei Raumtemperatur 456,0 mg (3,30 mmol, 3,0 Äq.) Kaliumcarbonat gegeben und anschließend 24 Stunden unter Rückfluss erhitzt. Nach Abkühlen auf Raumtemperatur werden 20 ml destilliertes Wasser sowie 10 ml gesättigte Natriumchloridlösung zugegeben. Es wird dreimal mit je 20 ml Diethylether extrahiert, die organischen Phasen vereinigt, über Magnesiumsulfat getrocknet und das Lösungsmittel unter vermindertem Druck entfernt. Das Rohprodukt wird säulenchromatographisch gereinigt (Eluent: *n*-Hexan/Ethylacetat, 5:1 *v*/*v*). Das Produkt wird als weißer Feststoff erhalten und lässt sich aus Methanol kristallisieren.

| | |
|---|---|
| Ausbeute: | 260 mg (0,63 mmol, 56,8 %) |
| R_{f}-Wert: | 0,30 (Eluent: Hexan/Ethylacetat 5:1 *v*/*v*) |
| Schmelzpunkt: | 108 - 109 °C |

¹H-NMR (CDCl₃): δ = 1,15 - 2,95 (br m, 10H, B**H**), 1,23 (m, 2H, 2-C**H₂ₐ**), 1,35 (m, 2H, 1-C**H₂ₐ**), 1,51 (m, 2H, 1-C**H**_{2b}), 1,67 (br m, 2H, 2-C**H_{2b}**), 1,96 (m, 1H, 3-C**H**), 3,72 (br m, 1H, 4-C**H**), 4,23 (s, 2H, 7-C**H₂**), 7,41 (d, 1H, 9-C**H**, ³*J*_{HH} = 8,7 Hz), 7,45 (t, 1H, 13-C**H**, ³*J*_{HH} = 8,0 Hz), 7,63 (t, 1H, 14-C**H**, ³*J*_{HH} = 7,9 Hz), 7,71 (d, 1H, 12-C**H**, ³*J*_{HH} = 8,0 Hz), 7,98 (d, 1H, 15-C**H**, ³*J*_{HH} = 7,9 Hz), 8,04 (d, 1H, 10-C**H**, ³*J*_{HH} = 8,7 Hz) ppm.

¹¹B{¹H}-NMR (CDCl₃): δ = -13,4 (s, 3B, **B**H), -11,9 (s, 2B, **B**H), -11,0 (s, 3B, **B**H), -7,3 (s, 1B, **B**H), -5,2 (s, 1B, **B**H) ppm.

¹³C{¹H}-NMR (CDCl₃): δ = 25,5 (s, 1-**C**H₂), 31,8 (s, 2-**C**H₂), 43,1 (s, 7-C**H₂**), 44,5 (s, 3-**C**H), 71,3 (s, 6-**C_{q}**), 75,5 (s, 4-**C**H), 83,0 (s, 5-**C**_{q}), 120,7 (s, 9-**C**H), 126,6 (s, 13-**C**H), 127,0 (s, 11-**C_{q}**), 127,5 (s, 12-**C**H), 129,0 (s, 15-**C**H), 129,8 (s, 14-**C**H), 136,9 (s, 10-**C**H), 147,6 (s, 16-**C**H), 155,5 (s, 8-**C_{q}**) ppm.

Massenspektrometrie (ESI pos., CH₂Cl₂/CH₃OH):

| | |
|---|---|
| Berechnet: | *m*/*z* = 415,3 |
| Ermittelt: | *m*/*z* = 416,3 (40%, [M + H]⁺), 438,3 (100%, [M + Na]⁺) |

IR-Spektroskopie (KBr, ṽ in cm⁻¹): 3181 (m), 2955 (w), 2593 (s), 2582 (s), 2570 (s), 1602 (w), 1507 (m), 821 (m), 761 (m).

Elementaranalyse:

| | | |
|---|---|---|
| Berechnet für C₁₈H₂₉B₁₀N₁O₁S₁: | C = 52,02 % | H = 7,03 % N = 3,37 % |
| Gefunden: | C = 52,38 % | H = 6,86 % N = 3,04 % |

Röntgenkristallstrukturanalyse

| | | |
|---|---|---|
| Summenformel | C₁₈ H₂₉ B₁₀ N₁ O₁ S₁ | |
| Formelgewicht | 415,58 | |
| Temperatur | 130(2) K | |
| Wellenlänge | 71,073 pm | |
| Kristallsystem | Monoklin | |
| Raumgruppe | P 2₁/n | |
| Gitterkonstanten | a = 1310,67(2) pm | α = 90°. |
| | b = 1086,26(2) pm | β = 100,665(2)°. |
| | c = 1569,12(3) pm | γ = 90°. |
| Zellvolumen | 2,19541(7) nm³ | |
| Zahl der Formeleinheiten | 4 | |
| Dichte (berechnet) | 1,257 Mg/m³ | |
| Absorptions koeffizient | 0,159 mm⁻¹ | |
| F(000) | 872 | |
| Größe des Kristalls | 0,4 x 0,35 x 0,3 mm³ | |
| Messbereich von θ | 2,240 bis 32,554°. | |
| Index Bereiche | -19≤h≤18, -16≤k≤15, -23≤l≤23 | |
| Gemessene Reflexe | 28941 | |
| Unabhängige Reflexe | 7380 [R(int) = 0,0440] | |
| Vollständigkeit bis θ = 30.510° | 100,0 % | |
| Absorptionskorrektur | Semi-empirisch von Äquivalenten | |
| Max. und min. Transmission | 1 und 0,97922 | |
| Verfeinerungsmethode | Full-matrix least-squares on F² | |
| Data / restraints / parameters | 7380 / 29 / 381 | |
| Goodness-of-fit on F² | 1,012 | |
| R Werte [l>2σ(l)] | R1 = 0,0446, wR2 = 0,0984 | |
| R Werte (alle Reflexe) | R1 = 0,0681, wR2 = 0,1082 | |
| Max. und min. Restelektronendichte | 0,370 und -0,327 e·Å⁻³ | |

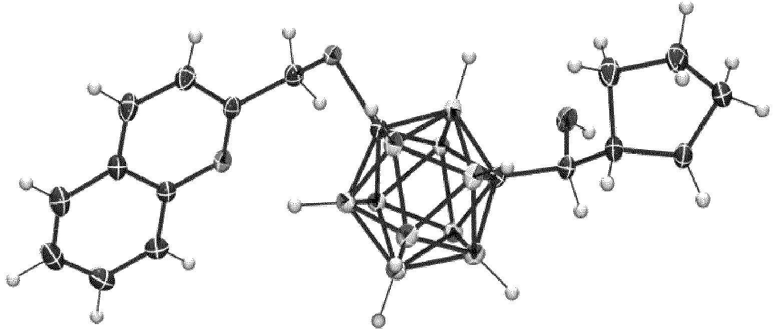

### Vergleichsbeispiel 13:

### 1-Cyclopentylhydroxymethyl-7-hydroxy-1,7-dicarba-closo-dodecaboran(12)

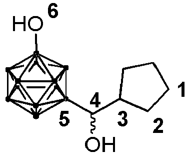

Zu einer Lösung von 350,0 mg (2,16 mmol, 1,0 Äq.) 1-Hydroxy-1,7-dicarba-*closo-*dodecaboran(12) in 100 ml Diethylether werden unter inerten Bedingungen bei 0 °C 3,45 ml (1,45 M in n-Hexan, 4,97 mmol, 2,3 Äq.) einer n-Butyllithiumlösung vorsichtig getropft. Nach 30 Minuten wird die Lösung auf Raumtemperatur erwärmt. Nach drei Stunden wird die Lösung erneut auf 0 °C abgekühlt und 0,2 ml (212,0 mg, 2,16 mmol, 1,0 Äq.) Cyclopentylaldehyd zugetropft. Die Lösung wird anschließend auf Raumtemperatur erwärmt. Nach 24 Stunden wird die weiße Suspension mit 20 ml destilliertem Wasser versetzt, mit konzentrierter Salzsäure angesäuert und anschließend dreimal mit je 20 ml Diethylether extrahiert. Die vereinigten organischen Phasen werden mit 20 ml gesättigter Natriumchloridlösung gewaschen, über Magnesiumsulfat getrocknet und das Lösungsmittel anschließend unter vermindertem Druck entfernt. Der Rückstand wird säulenchromatographisch gereinigt (Eluent: Hexan/Ethylacetat 5:1 *v*/*v*). Es wird eine farblose Flüssigkeit erhalten, die nach längerem Stehen erstarrt.

| | |
|---|---|
| Ausbeute: | 283,0 mg (1,09 mmol, 50,7 %) |
| R_{f}-Wert: | 0,43 (Eluent: Hexan/Ethylacetat 5:1 *v*/*v*) |
| Schmelzpunkt: | 71,9 - 72,8 °C |

¹H-NMR (CDCl₃): δ = 1,05 - 3,39 (br m, 10H, B**H**), 1,35 (m, 2H, 2-C**H₂ₐ**), 1,46 (m, 2H, 1-C**H₂ₐ**), 1,70 (m, 2H, 2-C**H_{2b}**), 1,79 (m, 2H, 1-C**H**_{2b}), 2,09 (m, 1H, 3-CH), 3,80 (d, 1H, 4-C**H**, ³*J*_{HH} = 3,6 Hz) ppm.

¹¹B{¹H}-NMR (CDCl₃): δ = -14,4 (s, 4**B**, **B**H), -13,6 (s, 3**B**, **B**H), -11,5 (s, 2**B**, **B**H), -6,2 (s, 1**B**, **B**H) ppm.

¹³C{¹H}-NMR (CDCl₃): δ = 25,6 (s, 1-**C**H₂), 31,9 (s, 2-**C**H₂), 44,6 (s, 3-CH), 74,1 (s, 5-**C**_{q}), 75,5 (s, 4-CH) ppm.

Massenspektrometrie (ESI neg., CH₂Cl₂/CH₃CN):

| | |
|---|---|
| Berechnet: | *m*/*z* = 258,3 |
| Ermittelt: | *m*/*z* = 257,3 (100%, [M - H]⁻) |

IR-Spektroskopie (KBr, ṽ in cm⁻¹): 3454 (w), 3230 (m), 2956 (w), 2866 (w), 2611 (s), 2600 (s), 2591 (s), 1202 (m), 1020 (m).

Elementaranalyse:

| | |
|---|---|
| Berechnet für C₈H₂₂B₁₀O₂: | C = 37,19 % H = 8,58 % |
| Gefunden: | C = 40,16 % H = 8,52 % |

### Ausführungsbeispiel 14:

### 1-Cyclopentylhydroxymethyl-7-(chinolin-2-ylmethoxy)-1,7-dicarba-closo-dodecaboran(12)

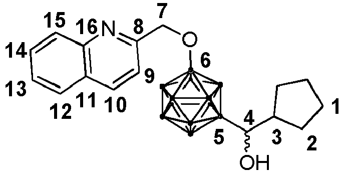

Zu einer Lösung von 1-Cyclopentylhydroxymethyl-7-hydroxy-1,7-dicarba-*closo-*dodecaboran(12) (100,0 mg, 0,38 mmol, 1,0 Äq.) und 2-Brommethylchinolin (85,0 mg, 0,38 mmol, 1,0 Äq.) in 50 ml Aceton werden bei Raumtemperatur 159,2 mg (1,15 mmol, 3,0 Äq.) Kaliumcarbonat gegeben und anschließend 24 Stunden unter Rückfluss erhitzt. Nach Abkühlen auf Raumtemperatur wird die Suspension filtriert und verbliebenes Kaliumcarbonat dreimal mit je 20 ml Diethylether extrahiert. Die organischen Phasen werden vereinigt, über Magnesiumsulfat getrocknet und das Lösungsmittel unter vermindertem Druck entfernt. Das Rohprodukt wird säulenchromatographisch gereinigt (Eluent: *n*-Hexan/Ethylacetat, 5:1 *v*/*v*). Das Produkt wird als weißer Feststoff erhalten und lässt sich aus Ethylacetat kristallisieren.

| | |
|---|---|
| Ausbeute: | 166 mg (0,40 mmol, 51 %) |
| R_{f}-Wert: | 0,25 (Eluent: Hexan/Ethylacetat 10:1 *v*/*v*) |
| Schmelzpunkt: | 108 - 109 °C |

¹H-NMR (CDCl₃): δ = 1,19- 3,63 (br m, 10H, B**H**), 1,39 (m, 2H, 2-C**H₂ₐ**), 1,48 (m, 2H, 1-C**H₂ₐ**), 1,68 (m, 2H, 2-C**H_{2b}**), 1,81 (m, 2H, 1-C**H**_{2b}), 2,11 (dt, 1H, 3-C**H**,³*J*_{HH} = 8,4 Hz, ³*J*_{HH} = 3,5 Hz), 3,83 (br q, 1H, 4-C**H**, ³*J*_{HH} = 3,6 Hz), 4,86 (s, 2H, 7-C**H₂**), 7,47 (d, 1H, 9-C**H**, ³*J*_{HH} = 8,5 Hz), 7,55 (t, 1H, 13-C**H**, ³*J*_{HH} = 8,2 Hz), 7,73 (t, 1H, 14-C**H**, ³*J*_{HH} = 8,5 Hz), 7,82 (d, 1H, 12-C**H**, ³*J*_{HH} = 8,2 Hz), 8,05 (d, 1H, 15-C**H**, ³*J*_{HH} = 8,5 Hz), 8,19 (d, 1H, 10-CH, ³*J*_{HH} = 8,5 Hz) ppm.

¹¹B{¹H}-NMR (CDCl₃): δ = -14,7 (s, 5**B**, **B**H), -13,8 (s, 2**B**, **B**H), -12,8 (s, 2**B**, **B**H), -7,6 (s, 1**B**, **B**H) ppm.

¹³C{¹H}-NMR (CDCl₃): δ = 25,5 (s, 1-**C**H₂), 31,9 (s, 2-**C**H₂), 44,6 (s, 3-**C**H), 75,4 (s, 4-**C**H), 78,2 (s, 5-**C**_{q}), 106,4 (s, 6-**C_{q}**), 119,0 (s, 9-CH), 126,6 (s, 13-**C**H), 127,5 (s, 11-**C_{q}**), 127,6 (s, 12-**C**H), 128,9 (s, 15-**C**H), 129,8 (s, 14-**C**H), 136,9 (s, 10-**C**H), 147,2 (s, 16-**C_{q}**), 156,1 (s, 8-**C_{q}**) ppm.

Massenspektrometrie (ESI pos., CH₂Cl₂/CH₃CN):

| | |
|---|---|
| Berechnet: | *m*/*z* = 399,3 |
| Ermittelt: | *m*/*z* = 400,3 (100%, [M + H]⁺), 422,3 (10%, [M + Na]⁺) |

IR-Spektroskopie (KBr, ṽ in cm⁻¹): 3434 (s), 2963 (m), 2600 (w), 1631 (w), 1262 (s), 1096 (s), 1022 (s), 865 (w), 802 (s), 704 (w).

Elementaranalyse:

| | | |
|---|---|---|
| Berechnet für C₁₈H₂₉B₁₀NOS: | C = 52,02 % | H = 7,03 % N = 3,37 % |
| Gefunden: | C = 51,60 % | H = 7,25 % N = 3,13 % |

### Vergleichsbeispiel 15:

### 1-Cyclopentylhydroxymethyl-12-thio-1,12-dicarba-closo-dodecaboran(12)

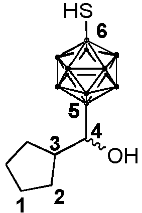

Zu einer Lösung von 176,0 mg (0,99 mmol, 1,0 Äq.) 1-Mercapto-1,12-dicarba-*closo-*dodecaboran(12) in 50 ml Diethylether werden unter inerten Bedingungen bei 0 °C 1,43 ml (1,45 M in n-Hexan, 2,07 mmol, 2,1 Äq.) einer n-Butyllithiumlösung vorsichtig getropft. Nach 30 Minuten wird die Lösung auf Raumtemperatur erwärmt. Nach drei Stunden wird die Lösung erneut auf 0 °C abgekühlt und 84 µl (91,0 mg, 0,93 mmol, 1,0 Äq.) Cyclopentylaldehyd zugetropft. Die Lösung wird anschließend auf Raumtemperatur erwärmt. Nach 24 Stunden wird die weiße Suspension mit 20 ml destilliertem Wasser versetzt, mit konzentrierter Salzsäure angesäuert und anschließend dreimal mit je 20 ml Diethylether extrahiert. Die vereinigten organischen Phasen werden mit 20 ml gesättigter Natriumchloridlösung gewaschen, über Magnesiumsulfat getrocknet und das Lösungsmittel anschließend unter vermindertem Druck entfernt. Der Rückstand wird säulenchromatographisch gereinigt (Eluent: Hexan/Ethylacetat 5:1 *v*/*v*). Es wird ein leicht gelblicher Feststoff erhalten.

| | |
|---|---|
| Ausbeute: | 247,2 mg (0,90 mmol, 91 %) |
| R_{f}-Wert: | 0,64 (Eluent: Hexan/Ethylacetat 5:1 *v*/*v*) |
| Schmelzpunkt: | 57,6 - 58,3 °C |

¹H-NMR (CDCl₃): δ = 1,18 - 3,20 (br m, 10H, B**H**), 1,23 (m, 2H, 2-C**H₂ₐ**), 1,31 (m, 2H, 1-C**H₂ₐ**), 1,55 (m, 2H, 2-C**H_{2b}**), 1,68 (m, 2H, 1-C**H_{2b}**),1,83 (m, 1H, 3-C**H**), 3,44 (d, 1H, 4-C**H**,³*J*_{HH} = 3,4 Hz) ppm.

¹¹B{¹H}-NMR (CDCl₃): δ = -14,7 (s, 2B, **B**H), -13,8 (s, 1B, **B**H), -13,2 (s, 3B, **B**H), -11,1 (s, 4B, **B**H) ppm.

¹³C{¹H}-NMR (CDCl₃): δ = 25,6 (s, 1-**C**H₂), 31,8 (s, 2-**C**H₂), 44,1 (s, 3-**C**H), 75,5 (s, 4-**C**H), 72,6 (s, 6-**C_{q}**), 82,7 (s, 5-**C**_{q}) ppm.

Massenspektrometrie (ESI-neg., CH₂Cl₂/CH₃CN):

| | |
|---|---|
| Berechnet: | *m*/*z* = 274,2 |
| Ermittelt: | *m*/*z* = 273,2 (20%, [M - H]⁻) |

IR-Spektroskopie (KBr, ṽ in cm⁻¹): 3387 (s), 2957 (s), 2928 (s), 2859 (m), 2602 (s), 1461 (w), 1253 (w), 1137 (s), 842 (m), 732 (w).

Elementaranalyse:

| | | |
|---|---|---|
| Berechnet für C₈H₂₂B₁₀OS: | C = 35,01 % | H = 8,08 % |
| Gefunden: | C = 40,10 % | H = 9,74 % |

### Ausführungsbeispiel 16:

### 1-Cyclopentylhydroxymethyl-12-(chinolin-2-ylmethyl)thio-1,12-dicarba-closo-dodecaboran(12)

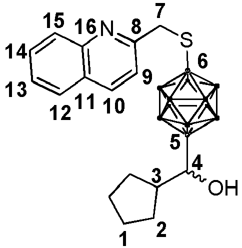

Zu einer Lösung von 1-Cyclopentylhydroxymethyl-12-thio-1,12-dicarba-*closo-*dodecaboran(12) (273,0 mg, 0,99 mmol, 1,0 Äq.) und 2-Brommethylchinolin (242,0 mg, 1,09 mmol, 1,1 Äq.) in 50 ml Aceton werden bei Raumtemperatur 410,0 mg (2,97 mmol, 3,0 Äq.) Kaliumcarbonat gegeben und anschließend 24 Stunden unter Rückfluss erhitzt. Nach Abkühlen auf Raumtemperatur wird die Suspension filtriert und verbliebenes Kaliumcarbonat dreimal mit je 20 ml Diethylether extrahiert. Die organischen Phasen werden vereinigt, über Magnesiumsulfat getrocknet und das Lösungsmittel unter vermindertem Druck entfernt. Das Rohprodukt wird säulenchromatographisch gereinigt (Eluent: *n*-Hexan/Ethylacetat, 5:1 *v*/*v*). Das Produkt wird als weißer Feststoff erhalten und lässt sich aus Ethylacetat kristallisieren.

| | |
|---|---|
| Ausbeute: | 130,0 mg (0,31 mmol, 31,6 %) |
| R_{f}-Wert: | 0,40 (Eluent: Hexan/Ethylacetat 5:1 *v*/*v*) |
| Schmelzpunkt: | 135,0 - 136,0 °C |

¹H-NMR (CDCl₃): δ = 1,12 - 3,30 (br m, 10H, BH), 1,23 (m, 2H, 2-C**H₂ₐ**), 1,35 (m, 2H, 1-C**H₂ₐ**), 1,47 (m, 2H, 2-C**H_{2b}**), 1,54 (m, 2H, 1-C**H**_{2b}), 1,69 (d, 1H, 3-C**H**,³*J*_{HH} = 7,0 Hz), 3,47 (q, 1H, 4-C**H**, ³*J*_{HH} = 3,4 Hz), 4,14 (s, 2H, 7-C**H₂**), 7,39 (d, 1H, 9-C**H**,³*J*_{HH} = 8,4 Hz), 7,52 (t, 1H, 13-C**H**,³*J*_{HH} = 7,4 Hz), 7,69 (t, 1H, 14-C**H**,³*J*_{HH} = 7,4 Hz), 7,76 (d, 1H, 12-C**H**,³*J*_{HH} = 8,1 Hz), 8,01 (d, 1H, 15-C**H**,³*J*_{HH} = 8,3 Hz), 8,08(d, 1H, 10-C**H**,³*J*_{HH} = 8,4 Hz) ppm.

¹¹B{¹H}-NMR (CDCl₃): δ = -13,4 (s, 5B, **B**H), -12,3 (s, 5B, **B**H) ppm.

¹³C{¹H}-NMR (CDCl₃): δ = 26,0 (s, 1-**C**H₂), 31,8 (s, 2-**C**H₂), 42,9 (s, 7-C**H₂**), 44,1 (s, 3-**C**H), 75,6 (s, 4-CH), 78,2 (s, 6-**C_{q}**), 85,5 (s, 5-**C**_{q}), 120,7 (s, 9-CH), 126,5 (s, 13-CH), 127,0 (s, 11-**C_{q}**), 127,4 (s, 12-CH), 129,0 (s, 15-CH), 129,7 (s, 14-CH), 136,8 (s, 10-CH), 147,6 (s, 16-**C_{q}**), 155,6 (s, 8-**C_{q}**) ppm.

Massenspektrometrie (ESI-pos., CH₂Cl₂/CH₃CN):

| | |
|---|---|
| Berechnet: | *m*/*z* = 415,3 |
| Ermittelt: | *m*/*z* = 438,3 (100%, [M + Na]⁺) |

IR-Spektroskopie (KBr, ṽ in cm⁻¹): 3241 (m), 2938 (m), 2856 (w), 2593 (s), 2362 (w), 1597 (w), 1507 (m), 1428 (m), 1133 (m), 1099 (m), 832 (m), 761 (m), 624 (w).

Elementaranalyse:

| | | |
|---|---|---|
| Berechnet für C₁₈H₂₉B₁₀N₁O₁S₁: | C = 52,02 % | H = 7,03 % N = 3,37 % |
| Gefunden: | C = 51,25 % | H = 6,82 % N = 3,04 % |

Röntgenkristallstrukturanalyse:

| | | |
|---|---|---|
| Summenformel | C₁₈ H₂₉ B₁₀ N₁ O₁ S₁ | |
| Formelgewicht | 415,58 | |
| Temperatur | 130(2) K | |
| Wellenlänge | 71,073 pm | |
| Kristallsystem | Monoklin | |
| Raumgruppe | P 21/c | |
| Gitterkonstanten | a = 1015,91(3) pm | α = 90°. |
| | b= 1519,89(4) pm | β = 90,400(2)° |
| | c= 1456,58(4) pm | γ = 90°. |
| Zellvolumen | 2,24901(11) nm³ | |
| Zahl der Formeleinheiten | 4 | |

| | | |
|---|---|---|
| Dichte (berechnet) | 1,227 Mg/m³ | |
| Absorptions koeffizient | 0,156 mm⁻¹ | |
| F(000) | 872 | |
| Größe des Kristalls | 0,3 x 0,2 x 0,1 mm³ | |
| Messbereich von θ | 1,937 bis 30,608°. | |
| Index Bereiche | -13≤h≤13, -21≤k≤21, -12≤l≤20 | |
| Gemessene Reflexe | 20798 | |
| Unabhängige Reflexe | 6289 [R(int) = 0,0515] | |
| Vollständigkeit bis θ = 28.285° | 100,0 % | |
| Absorptionskorrektur | Semi-empirisch von Äquivalenten | |
| Max. und min. Transmission | 1 und 0,99096 | |
| Verfeinerungsmethode | Full-matrix least-squares on F² | |
| Data / restraints / parameters | 6289 / 51 / 388 | |
| Goodness-of-fit on F² | 1,032 | |
| R Werte [l>2σ(l)] | R1 = 0,0545, wR2 = 0,1155 | |
| R Werte (alle Reflexe) | R1 = 0,1041, wR2 = 0,1351 | |
| Max. und min. Restelektronendichte | 0,347 und -0,269 e·Å⁻³ | |

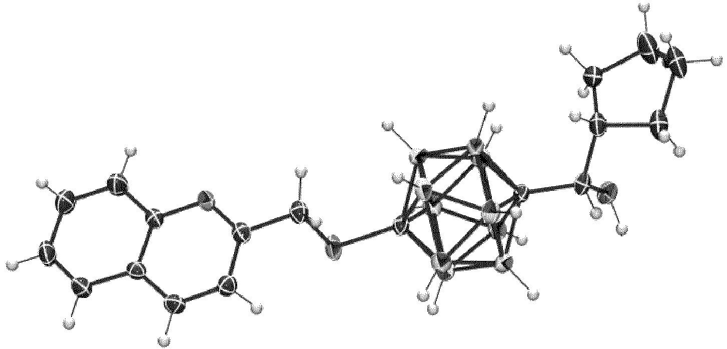

### Vergleichsbeispiel 17:

### 1-Cyclopentylhydroxymethyl-12-hydroxy-1,12-dicarba-closo-dodecaboran(12)

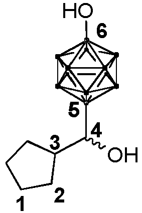

Zu einer Lösung von 150,0 mg (0,93 mmol, 1,0 Äq.) 1-Hydroxy-1,12-dicarba-*closo-*dodecaboran(12) in 50 ml Diethylether werden unter inerten Bedingungen bei 0 °C 1,40 ml (1,45 M in n-Hexan, 2,03 mmol, 2,2 Äq.) einer n-Butyllithiumlösung vorsichtig getropft. Nach 30 Minuten wird die Lösung auf Raumtemperatur erwärmt. Nach drei Stunden wird die Lösung erneut auf 0 °C abgekühlt und 84 µl (91,0 mg, 0,93 mmol, 1,0 Äq.) Cyclopentylaldehyd zugetropft. Die Lösung wird anschließend auf Raumtemperatur erwärmt. Nach 24 Stunden wird die weiße Suspension mit 20 ml destilliertem Wasser versetzt, mit konzentrierter Salzsäure angesäuert und anschließend dreimal mit je 20 ml Diethylether extrahiert. Die vereinigten organischen Phasen werden mit 20 ml gesättigter Natriumchloridlösung gewaschen, über Magnesiumsulfat getrocknet und das Lösungsmittel anschließend unter vermindertem Druck entfernt. Der Rückstand wird säulenchromatographisch gereinigt (Eluent: Hexan/Ethylacetat 5:1 *v*/*v*). Es wird ein weißer Feststoff erhalten.

| | |
|---|---|
| Ausbeute: | 127,0 mg (0,49 mmol, 53 %) |
| R_{f}-Wert: | 0,49 (Eluent: Hexan/Ethylacetat 5:1 *v*/*v*) |
| Schmelzpunkt: | 57,6 - 58,3 °C |

¹H-NMR (CDCl₃): δ = 1,13-3,39 (brm, 10H, B**H**), 1,26 (m, 2H, 2-C**H₂ₐ**), 1,36 (m, 2H, 1-C**H₂ₐ**), 1,56 (m, 2H, 2-C**H_{2b}**), 1,68 (m, 2H, 1-C**H**_{2b}), 1,86 (m, 2H, 3-C**H**), 3,56 (br s, 1H, 4-CH) ppm.

¹¹B{¹H}-NMR (CDCl₃): δ = -15,5 (s, 5B, **B**H), -13,2 (s, 5B, **B**H) ppm.

¹³C{¹H}-NMR (CDCl₃): δ = 25,6 (s, 1-**C**H₂), 31,9 (s, 2-**C**H₂), 44,5 (s, 3-**C**H), 74,9 (s, 4-**C**H), 74,9 (s, 5-**C**_{q}), 74,9 (s, 6-**C**_{q}) ppm.

Massenspektrometrie (ESI-neg., CH₂Cl₂/CH₃OH):

| | |
|---|---|
| Berechnet: | *m*/*z* = 258,3 |
| Ermittelt: | *m*/*z* = 257,3 (100%, [M - H]⁻) |

IR-Spektroskopie (KBr, ṽ in cm⁻¹): 3485 (w), 3255 (m), 2955 (m), 2860 (w), 2607 (s), 1392 (w), 1206 (m), 1145 (w), 1016 (m), 999 (w), 978 (w), 758 (w), 729 (w).

Elementaranalyse:

| | | |
|---|---|---|
| Berechnet für C₈H₂₂B₁₀O₂: | C = 37,19 % | H = 8,58 % |
| Gefunden: | C = 32,89 % | H = 8,19 % |

### Ausführungsbeispiel 18:

### 1-Cyclopentylhydroxymethyl-12-(chinolin-2-ylmethoxy)-1,12-dicarba-closo-dodecaboran(12)

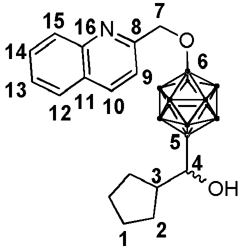

Zu einer Lösung von 1-Cyclopentylhydroxymethyl-12-hydroxy-1,12-dicarba-*closo-*dodecaboran(12) (100,0 mg, 0,38 mmol, 1,0 Äq.) und 2-Brommethylchinolin (85,0 mg, 0,38 mmol, 1,0 Äq.) in 50 ml Aceton werden bei Raumtemperatur 159,2 mg (1,15 mmol, 3,0 Äq.) Kaliumcarbonat gegeben und anschließend 24 Stunden unter Rückfluss erhitzt. Nach Abkühlen auf Raumtemperatur wird die Suspension filtriert und verbliebenes Kaliumcarbonat dreimal mit je 20 ml Diethylether extrahiert. Die organischen Phasen werden vereinigt, über Magnesiumsulfat getrocknet und das Lösungsmittel unter vermindertem Druck entfernt. Das Rohprodukt wird säulenchromatographisch gereinigt (Eluent: *n*-Hexan/Ethylacetat, 5:1 *v*/*v*). Das Produkt wird als weißer Feststoff erhalten und lässt sich aus Ethylacetat kristallisieren.

| | |
|---|---|
| Ausbeute: | 52,0 mg (13,0 mmol, 33,9 %) |
| R_{f}-Wert: | 0,24 (Eluent: Hexan/Ethylacetat 10:1 *v*/*v*) |
| Schmelzpunkt: | 154,0 - 154,9 °C |

¹H-NMR (CDCl₃): δ = 1,16 - 3,40 (br m, 10H, B**H**), 1,27 (m, 2H, 2-C**H₂ₐ**), 1,39 (m, 2H, 1-C**H₂ₐ**), 1,58 (m, 2H, 1-C**H**_{2b}), 1,70 (m, 2H, 2-C**H_{2b}**), 1,89 (m, 1H, 3-C**H**), 3,61 (q, 1H, 4-C**H**, ³*J*_{HH} = 3,4 Hz), 4,66 (s, 2H, 7-C**H₂**), 7,37 (d, 1H, 9-C**H**, ³*J*_{HH} = 8,4 Hz), 7,53 (t, 1H, 13-C**H**, ³*J*_{HH} = 8,0 Hz), 7,71 (t, 1H, 14-C**H**,³*J*_{HH} = 8,5 Hz), 7,79 (d, 1H, 12-C**H**,³*J*_{HH} = 8,0 Hz), 8,00 (d, 1H, 15-C**H**,³*J*_{HH} = 8,5 Hz), 8,13 (d, 1H, 10-C**H**,³*J*_{HH} = 8,4 Hz) ppm.

¹¹B{¹H}-NMR (CDCl₃): δ = -15,7 (s, 5B, **B**H), -14,0 (s, 5B, **B**H) ppm.

¹³C{¹H}-NMR (CDCl₃): δ = 25,6 (s, 1-**C**H₂), 31,9 (s, 2-**C**H₂), 44,5 (s, 3-**C**H₂), 75,1 (s, 4-**C**H), 75,4 (s, 5-**C**_{q}), 76,0 (s, 7-C**H₂**), 112,1 (s, 6-**C_{q}**), 119,0 (s, 9-CH), 126,7 (s, 13-CH), 127,6 (s, 11-**C_{q}**), 127,7 (s, 12-**C**H), 129,0 (s, 15-CH), 129,9 (s, 14-**C**H), 137,0 (s, 10-**C**H), 147,3 (s, 16-**C_{q}**), 155,9 (s, 8-**C_{q}**) ppm.

Massenspektrometrie (ESI-pos., CH₂Cl₂/CH₃OH):

| | |
|---|---|
| Berechnet: | *m*/*z* = 399,3 |
| Ermittelt: | *m*/*z* = 400,3 (100%, [M + H]⁺), 422,3 (40%, [M + Na]⁺) |

IR-Spektroskopie (KBr, ṽ in cm⁻¹): 3215 (m), 2945 (w), 2611 (s), 2361 (w), 1508 (w), 1207 (m), 1138 (w), 1089 (m), 1055 (m), 825 (m), 754 (w).

Elementaranalyse:

| | | |
|---|---|---|
| Berechnet für C₁₈H₂₉B₁₀N₁O₂: | C = 54,11 % | H = 7,32 % N = 3,51 % |
| Gefunden: | C = 54,18 % | H = 7,27 % N = 3,00 % |

Röntgenkristallstrukturanalyse:

| | | |
|---|---|---|
| Summenformel | C₁₈ H₂₉ B₁₀ N₁ O₂ | |
| Formelgewicht | 399,52 | |
| Temperatur | 130(2) K | |
| Wellenlänge | 71,073 pm | |
| Kristallsystem | Monoklin | |
| Raumgruppe | P 21/n | |
| Gitterkonstanten | a = 723,56(2) pm | α = 90°. |
| | b = 1133,24(3) pm | β = 90,135(2)° |
| | c = 2672,53(7) pm | γ = 90°. |
| Zellvolumen | 2,19138(10) nm³ | |
| Zahl der Formeleinheiten | 4 | |
| Dichte (berechnet) | 1,211 Mg/m³ | |
| Absorptions koeffizient | 0,069 mm⁻¹ | |
| F(000) | 840 | |
| Größe des Kristalls | 0,2 x 0,15 x 0,15 mm³ | |
| Messbereich von θ | 1,952 bis 30,587°. | |
| Index Bereiche | -10≤h≤10, -15≤k≤15, -35≤l≤36 | |
| Gemessene Reflexe | 24067 | |
| Unabhängige Reflexe | 6101 [R(int) = 0,0524] | |
| Vollständigkeit bis θ = 28.285° | 100,0 % | |
| Absorptionskorrektur | Semi-empirisch von Äquivalenten | |
| Max. und min. Transmission | 1 und 0,98423 | |
| Verfeinerungsmethode | Full-matrix least-squares on F² | |
| Data / restraints / parameters | 6101 / 16 / 381 | |
| Goodness-of-fit on F² | 1,017 | |
| R Werte [l>2σ(l)] | R1 = 0,0585, wR2 = 0,1260 | |
| R Werte (alle Reflexe) | R1 = 0,0984, wR2 = 0,1434 | |
| Max. und min. Restelektronendichte | 0,312 und -0,322 e·Å⁻³ | |

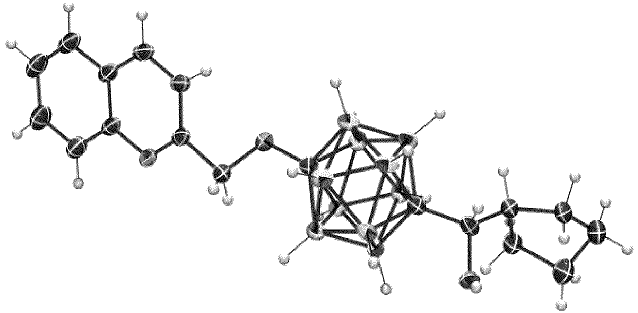

### Ausführungsbeispiel 19:

### 1-(Tetrahydro-2H-pyran-4-olyl)-7-((3-mercaptomethyl-1-(4-methansulfonylphenyl)-5-phenyl)-1H-pyrazolyl)-1,7-dicarba-closo-dodecaboran(12)

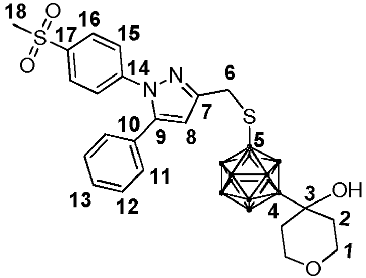

Zu einer Lösung von 1-Mercapto-7-(tetrahydro-2*H*-pyran-4-olyl)-1,7-dicarba-*closo-*dodecaboran(12) (100,0 mg, 0,36 mmol, 1,0 Äq.) und 3-Brommethyl-1-(4-methansulfonylphenyl)-5-phenyl-1*H*-pyrazol (140,7 mg, 0,36 mmol, 1,0 Äq.) in 50 ml Aceton werden bei Raumtemperatur 50,0 mg (0,36 mmol, 1,0 Äq.) Kaliumcarbonat gegeben und anschließend 24 Stunden unter Rückfluss erhitzt. Nach Abkühlen auf Raumtemperatur werden 20 ml destilliertes Wasser sowie 10 ml gesättigte Natriumchloridlösung zugegeben. Es wird dreimal mit je 20 ml Diethylether extrahiert, die organischen Phasen vereinigt, über Magnesiumsulfat getrocknet und das Lösungsmittel unter vermindertem Druck entfernt. Das Rohprodukt wird säulenchromatographisch gereinigt (Eluent: *n*-Hexan/Ethylacetat, 1:1 *v*/*v*). Das Produkt wird als farbloser Feststoff erhalten.

| | |
|---|---|
| Ausbeute: | 84,0 mg (0,14 mmol, 40 %) |
| R_{f}-Wert: | 0,27 (Eluent: *n*-Hexan/Ethylacetat 1:1 *v*/*v*) |
| Schmelzpunkt: | 88,0 - 89,0 °C |

¹H-NMR (CDCl₃): δ = 1,12 - 3,79 (br m, 10H, B**H**), 1,53 (d, 2H, Sessel-2-C**H₂**, ³*J*_{HH} = 13,3 Hz), 1,65 (br s, 1H, 3-O**H**), 1,81 (dt, 2H, Wanne-2-C**H₂**, ³*J*_{HH} = 13,3 Hz), 3,05 (s, 3H, 18-C**H₃**), 3,65 (dt, 2H, Sessel-1-C**H₂**, ³*J*_{HH} = 11,2 Hz), 3,79 (dd, 2H, Wanne-1-C**H₂**, ³*J*_{HH} = 11,2 Hz), 4,08 (s, 2H, 6-C**H₂**), 6,49 (s, 1H, 8-C**H**), 7,21 (d, 2H, 11-C**H**, ³*J*_{HH} = 8,4 Hz), 7,38 (m, 3H, 12,13-C**H**), 7,46 (d, 2H, 15-C**H**, ³*J*_{HH} = 8,9 Hz), 7,88 (d, 2H, 16-C**H**, ³*J*_{HH} = 8,9 Hz) ppm.

¹¹B{¹H}-NMR (CDCl₃): δ =-13,5 (s, 2B, **B**H),-12,5 (s, 2B, **B**H),-10,8 (s, 3B, **B**H), -7,8 (s, 1B, **B**H),-4,4 (s, 2B, **B**H) ppm.

¹³C{¹H}-NMR (CDCl₃): δ = 33,4 (s, 6-C**H₂**), 39,4 (s, 2-**C**H₂), 44,5 (s, 18-**C**H₃), 63,8 (s, 1-**C**H₂), 69,5 (s, 4-**C**_{q}), 70,8 (s, 5-**C**_{q}), 88,2 (s, 3-**C**_{q}), 109,0 (s, 8-CH), 125,0 (s, 15-CH), 128,4 (s, 16-CH), 128,7 (s, 11-CH), 128,9 (s, 12-CH), 129,2 (s, 13-CH), 129,6 (s, 10-**C**_{q}), 138,8 (s, 17-**C**_{q}), 143,9 (s, 14-**C**_{q}), 144,8 (s, 9-**C**_{q}), 148,6 (s, 7-**C**_{q}) ppm.

Massenspektrometrie (ESI pos., CH₂Cl₂/CH₃CN):

| | |
|---|---|
| Berechnet: | *m*/*z* = 586,3 |
| Ermittelt: | *m*/*z* = 588,3 (100%, [M + H]⁺), 587,3 (95%, [M + H]+) |

IR-Spektroskopie (KBr, ṽ in cm⁻¹): 3473 (s), 2960 (s), 2928 (s), 2869 (s), 2602 (s), 1595 (s), 1548 (w), 1504 (s), 1454 (w), 1436 (w), 1406 (m), 1372 (s), 1317 (s), 1300 (s), 1242 (m), 1156 (w), 1152 (s), 1091 (s) 1091 (m), 1016 (m), 956 (m), 876 (w), 844 (m), 807 (w), 782 (s), 762 (m), 697 (m), 587 (m), 556 (m), 547 (m), 533 (m), 490 (w), 446 (w).

Elementaranalyse:

| | |
|---|---|
| Berechnet für C₂₄H₃₄B₁₀N₂O₄S₂: | C = 49,13 % H = 5,84 % N = 4,77 % |
| Gefunden: | C = 49,56 % H = 5,93 % N = 4,42 % |

### Vergleichsbeispiel 20

### 9-Iodo-1-mercapto-7-(tetrahydro-2H-pyran-4-olyl)-1,7-dicarba-closo-dodecaboran(12)

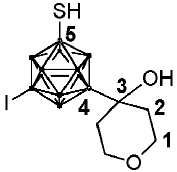

Es werden 1,5 g (5,50 mmol, 1,0 Äq.) 9-Iodo-1,7-dicarba-*closo*-dodecaboran(12) in 300 ml Diethylether gelöst und bei 0 °C 4,17 ml (1,45 M in n-Hexan, 6,04 mmol, 1,1 Äq.) einer n-Butyllithiumlösung vorsichtig zugetropft. Die klare gelbliche Lösung wird anschließend auf Raumtemperatur erwärmt. Nach drei Stunden wird erneut auf 0 °C abgekühlt und 176,0 mg (5,50 mmol, 1,0 Äq.) Schwefel zugegeben. Die Lösung wird auf Raumtemperatur erwärmt, nach 24 Stunden bei 0 °C mit 4,17 ml (1,45 M in *n*-Hexan, 6,04 mmol, 1,1 Äq.) *n*-Butyllithium versetzt und anschließend auf Raumtemperatur erwärmt. Nach drei Stunden wird die Lösung auf 0 °C abgekühlt und mit 0,51 ml (550,7 mg, 5,50 mmol, 1,0 Äq.) Tetrahydro-2*H*-pyranon versetzt. Anschließend wird auf Raumtemperatur erwärmt. Nach 24 Stunden wird die erhaltene weiße Suspension mit 30 ml destilliertem Wasser versetzt, mit konzentrierter Salzsäure angesäuert und dreimal mit je 20 ml Diethylether extrahiert. Die vereinigten organischen Phasen werden mit destilliertem Wasser neutralisiert, mit gesättigter Natriumchloridlösung gewaschen, über Magnesiumsulfat getrocknet und das Lösungsmittel unter vermindertem Druck entfernt. Der Rückstand wird aus n-Hexan umkristallisiert und das Produkt als hellgelber Feststoff erhalten.

| | |
|---|---|
| Ausbeute: | 2,12 g (5,3 mmol, 96 %) |
| R_{f}-Wert: | 0,08 (Eluent: *n*-Hexan/Ethylacetat 5:2 *v*/*v*) |

¹H-NMR (Aceton-d₆): δ = 1,10 - 4,02 (br m, 9H, B**H**), 1,52 (d, 2H, Sessel-2-C**H₂**, ³*J*_{HH} = 12,6 Hz), 1,58 (dt, 2H, Wanne-2-C**H₂**, ³*J*_{HH} = 12,6 Hz), 3,48 (dt, 2H, Sessel-1-C**H₂**, ³*J*_{HH} = 11,4 Hz), 3,58 (dd, 2H, Wanne-1-C**H₂**, ³*J*_{HH} = 11,4 Hz) ppm.

¹¹B-NMR (Aceton-d₆): δ = -24,0 (s, 1B, **BI**), -14,3 (d, 2B, **B**H), -12,3 (d, 2B, **B**H), -9,1 (s, 3B, **B**H), -3,0 (d, 2B, **B**H) ppm.

¹³C{¹H}-NMR (Aceton-d₆): δ = 35,7 (s, 5-**C**_{q}), 38,9 (s, 2-CH), 63,2 (s, 1-**C**H₂), 68,1 (s, 3-**C**_{q}), 69,4 (s, 4-**C**_{q}) ppm.

Massenspektrometrie (ESI neg., CH₃OH):

| | |
|---|---|
| Berechnet: | *m*/*z* = 402,1 |
| Ermittelt: | *m*/*z* = 401,1 (100%, [M - H]⁻), 301,1 (15%, [M - C₅H₉O₂]⁻) |

IR-Spektroskopie (KBr, ṽ in cm⁻¹): 3407 (s), 3214 (s), 2963 (s), 2871 (m), 2606 (s), 2260 (w), 1969 (w), 1630 (w), 1419 (m), 1385 (m), 1302 (w), 1262 (m) 1199 (m), 1159 (m), 1095 (s), 1020 (s), 973 (w) 885 (w), 802 (s), 684 (w), 641 (w), 546 (w)

### Ausführungsbeispiel 21:

### 9-Iodo-1-(2-mercaptomethyl)naphthyl-7-(tetrahydro-2H-pyran-4-olyl)-1,7-dicarba-closo-dodecaboran(12)

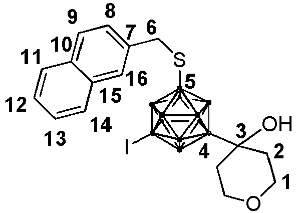

Zu einer Lösung von 9-Iodo-1-mercapto-7-(tetrahydro-2*H*-pyran-4-olyl)-1,7-dicarba-*closo-*dodecaboran(12) (0,8 g, 1,99 mmol, 1,0 Äq.) und 2-Brommethylnaphthalin (439,5 mg, 1,99 mmol, 1,0 Äq.) in 80 ml Aceton werden bei Raumtemperatur 1,10 g (7,95 mmol, 4,0 Äq.) Kaliumcarbonat gegeben und anschließend 24 Stunden unter Rückfluss erhitzt. Nach Abkühlen auf Raumtemperatur wird die Suspension filtriert und das Lösungsmittel unter vermindertem Druck entfernt. Das Rohprodukt wird säulenchromatographisch gereinigt (Eluent: *n*-Hexan/Ethylacetat, 5:2 *v*/*v*). Das Produkt wird als bräunliches Öl erhalten.

| | |
|---|---|
| Ausbeute: | 400,0 mg (0,74 mmol, 37 %) |
| R_{f}-Wert: | 0,38 (Eluent: n-Hexan/Ethylacetat 5:2 *v*/*v*) |

¹H-NMR (CDCl₃): δ = 1,11 -4,01 (br m, 9H, B**H**), 1,57 (d, 2H, Sessel-2-C**H₂**, ³*J*_{HH} = 13,4 Hz), 1,80 (dt, 2H, Wanne-2-C**H₂**, ³*J*_{HH} = 13,4 Hz), 3,64 (dt, 2H, Sessel-1-C**H₂**, ³*J*_{HH} = 11,7 Hz), 3,78 (dd, 2H, Wanne-1-C**H₂**, ³*J*_{HH} = 11,7 Hz), 4,14 (s, 2H, 6-C**H₂**), 7,38 (dd, 1H, 8-C**H**, ³*J*_{HH} = 8,5 Hz), 7,46 - 7,51 (m, 2H, 12,13-C**H**), 7,74 (br s, 1H, 16-C**H**), 7,78 - 7,84 (m, 2H, 11, 14-CH) ppm.

¹¹B-NMR (CDCl₃): δ = -24,1 (s, 1B, **BI**), -12,8 (d, 2B, **B**H), -9,6 (d, 2B, **B**H), -8,4 (d, 3B, **B**H), -3,9 (d, 2B, **B**H) ppm.

¹³C{¹H}-NMR (CDCl₃): δ = 39,3 (s, 2-**C**H₂), 41,4 (s, 6-**C**H), 63,6 (s, 1-**C**H₂), 69,8 (s, 3-**C**_{q}), 72,4 (s, 5-**C**_{q}), 89,4 (s, 4-**C**_{q}), 126,4 (s, 12-**C**H), 126,5 (s, 13-**C**H), 126,8 (s, 8-**C**H), 127,7 (s, 11,14-**C**H), 128,4 (s, 9-**C**H), 128,7 (s, 16-**C**H), 131,1 (s, 7-**C**_{q}), 132,8 (s, 10-**C**_{q}), 133,2 (s, 15-**C_{q}**) ppm.

Massenspektrometrie (ESI neg., CH₃OH):

| | |
|---|---|
| Berechnet: | *m*/*z* = 542,2 |
| Ermittelt: | *m*/*z* = 541,2 (100%, [M - H]⁻) |

IR-Spektroskopie (KBr, ṽ in cm⁻¹): 3387 (s), 3053 (m), 2960 (s), 2869 (s), 2607 (s), 1708 (m), 1600 (w), 1509 (m), 1467 (w), 1358 (m), 1301 (m), 1272 (m) 1243 (s), 1204 (w), 1159 (s), 1126 (s), 1020 (m), 973(w), 944(w), 894 (w), 853 (m), 816 (s), 752 (s), 633 (w), 620 (w), 547 (m), 472 (m).

### Ausführungsbeispiel 22:

### 9-Iodo-1-(2-mercaptomethyl)chinolinyl-7-(tetrahydro-2H-pyran-4-olyl)-1,7-dicarba-closo-dodecaboran(12)

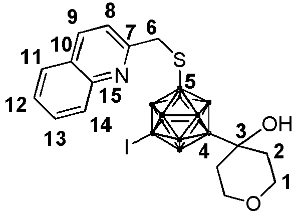

Zu einer Lösung von 9-Iodo-1-mercapto-7-(tetrahydro-2*H*-pyran-4-olyl)-1,7-dicarba-*closo-*dodecaboran(12) (0,3 g, 0,75 mmol, 1,0 Äq.) und 2-Chlormethylchinolinhydrochlorid (159,7 mg, 0,75 mmol, 1,0 Äq.) in 50 ml Aceton werden bei Raumtemperatur 412 mg (2,99 mmol, 4,0 Äq.) Kaliumcarbonat gegeben und anschließend 24 Stunden unter Rückfluss erhitzt. Nach Abkühlen auf Raumtemperatur wird die Suspension filtriert und das Lösungsmittel unter vermindertem Druck entfernt. Das Rohprodukt wird äulenchromatographisch gereinigt (Eluent: *n*-Hexan/Ethylacetat, 5:2 *v*/*v*). Das Produkt wird als weißer Feststoff erhalten.

| | |
|---|---|
| Ausbeute: | 250 mg (0,46 mmol, 62 %) |
| R_{f}-Wert: | 0,10 (Eluent: *n*-Hexan/Ethylacetat 5:2 *v*/*v*) |

¹H-NMR (CDCl₃): δ = 1,29 - 3,87 (br m, 9H, B**H**), 1,59 (d, 2H, Sessel-2-C**H₂**, ³*J*_{HH} = 13,2 Hz), 1,75 (dt, 2H, Wanne-2-C**H₂**, ³*J*_{HH} = 13,2 Hz), 3,61 (t, 2H, Sessel-1-C**H₂**, ³*J*_{HH} = 12,1 Hz), 3,76 (dd, 2H, Wanne-1-C**H₂**, ³*J*_{HH} = 12,1 Hz), 4,31 (s, 2H, 6-C**H₂**), 7,45 (d, 1H, 8-C**H**, ³*J*_{HH} = 8,4 Hz), 7,54 (t, 1H, 12-C**H**, ³*J*_{HH} = 7,6 Hz), 7,71 (t, 1H, 13-C**H**, ³*J*_{HH} = 7,6 Hz), 7,81 (d, 1H, 11-C**H**, ³*J*_{HH} = 8,4 Hz), 8,02 (d, 1H, 14-C**H**, ³*J*_{HH} = 8,4 Hz), 8,15 (d, 1H, 9-C**H**, ³*J*_{HH} = 8,4 Hz) ppm.

¹¹B-NMR (CDCl₃): δ = -24,1 (s, 1B, **BI**), -14,5 (d, 1B, **B**H), -12,8 (d, 2B, **B**H), -9,6 (d, 2B, **B**H), -6,1 (d, 2B, **B**H), -4,7 (d, 2B, **B**H) ppm.

¹³C{¹H}-NMR (CDCl₃): δ = 39,1 (s, 2-**C**H₂), 43,3 (s, 6-**C**H), 63,6 (s, 1-**C**H₂), 69,7 (s, 3-**C**_{q}), 72,0 (s, 5-**C**_{q}), 89,5 (s, 4-**C**_{q}), 120,6 (s, 8-CH), 126,8 (s, 12-**C**H), 127,1 (s, 10-**C**_{q}), 127,5 (s, 11-**C**H), 128,9 (s, 14-**C**H), 129,9 (s, 13-**C**H), 137,1 (s, 9-**C**H), 147,5 (s, 15-**C_{q}**), 155,0 (s, 7-**C_{q}**) ppm.

Massenspektrometrie (ESI pos., CH₃OH):

| | |
|---|---|
| Berechnet: | *m*/*z* = 543,2 |
| Ermittelt: | *m*/*z* = 566,2 (100%, [M + Na]⁺), 544,2 (30%, [M + H]⁺) |

IR-Spektroskopie (KBr, ṽ in cm⁻¹): 3418 (s), 2960 (m), 2930 (m), 2869 (m), 2610 (s), 1703 (w), 1619 (w), 1598 (m), 1562 (w), 1505 (m), 1427 (m), 1385 (m) 1355 (m), 1302 (w), 1244 (m), 1160 (s), 1137 (s), 1099 (m), 1020 (w), 975 (w), 941 (w), 850 (w), 803 (m), 765 (m), 668 (w), 618 (w), 546 (w), 476 (w).

### Ausführungsbeispiel 23:

### 9-Iodo-1-mercaptocarbonylphenyl-7-(tetrahydro-2H-pyran-4-olyl)-1,7-dicarba-closo-dodecaboran(12)

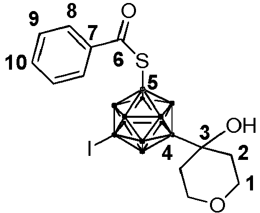

Es werden 250,0 mg (0,62 mmol, 1,0 Äq.) 9-Iodo-1-mercapto-7-(tetrahydro-2*H*-pyran-4-olyl)-1,7-dicarba-*closo*-dodecaboran(12) in 30 ml Dichlormethan gelöst und bei Raumtemperatur mit 0,33 ml (1,86 mmol, 3,0 Äq.) Diisopropylamin und anschließend mit 72 µl (87 mg, 0,62 mmol, 1,0 Äq.) Benzoylchlorid versetzt. Nach 24 Stunden wird die erhaltene weiße Suspension mit 30 ml destilliertem Wasser versetzt, mit verdünnter Salzsäure angesäuert und die entstehenden Phasen getrennt. Die organische Phase wird zweimal mit je 20 ml verdünnter Salzsäure gewaschen, mit destilliertem Wasser neutralisiert und mit gesättigter Natriumchloridlösung gewaschen. Die organische Phase wird über Magnesiumsulfat getrocknet und das Lösungsmittel unter vermindertem Druck entfernt. Das Rohprodukt wird säulenchromatographisch gereinigt (Eluent: *n*-Hexan/Ethylacetat, 5:2 *v*/*v*). Das Produkt wird als farbloses Öl erhalten.

| | |
|---|---|
| Ausbeute: | 80,0 mg (0,26 mmol, 25 %) |
| R_{f}-Wert: | 0,35 (Eluent: *n*-Hexan/Ethylacetat 5:2 *v*/*v*) |

¹H-NMR (CDCl₃): δ = 1,04-4,07 (br m, 9H, B**H**), 1,61 (d, 2H, Sessel-2-C**H₂**, ³*J*_{HH} = 13,2 Hz), 1,87 (dt, 2H, Wanne-2-C**H₂**, ³*J*_{HH} = 13,2 Hz), 3,67 (t, 2H, Sessel-1-C**H₂**, ³*J*_{HH} = 11,5 Hz), 3,82 (dd, 2H, Wanne-1-C**H₂**, ³*J*_{HH} = 11,5 Hz), 7,46 (t, 2H, 8-C**H**, ³*J*_{HH} = 7,6 Hz), 7,62 (t, 1H, 12-C**H**, ³*J*_{HH} = 7,3 Hz), 7,81 (d, 2H, 13-C**H**, ³*J*_{HH} = 7,6 Hz) ppm.

¹¹B-NMR (CDCl₃): δ = -24,3 (s, 1B, **BI**), -14,6 (d, 2B, **B**H), -13,0 (d, 2B, **B**H), -9,9 (d, 3B, **B**H), -3,9 (d, 2B, **B**H) ppm.

¹³C{¹H}-NMR (CDCl₃): δ = 39,2 (s, 2-**C**H₂), 63,6 (s, 1-**C**H₂), 66,3 (s, 5-**C**_{q}), 66,9 (s, 3-**C**_{q}), 89,3 (s, 4-**C**_{q}), 127,1 (s, 8-**C**H), 129,0 (s, 9-**C**H), 134,5 (s, 10-**CH**), 135,0 (s, 7-**C**_{q}), 184,9 (s, 6-**C_{q}**) ppm.

Massenspektrometrie (ESI pos., CH₃OH):

| | |
|---|---|
| Berechnet: | *m*/*z* = 506,1 |
| Ermittelt: | *m*/*z* = 529,1 (100%, [M + Na]⁺) |

IR-Spektroskopie (KBr, ṽ in cm⁻¹): 3427 (s), 2967 (w), 2870 (w), 2610 (m), 1696 (m), 1580 (w), 1467 (w), 1448 (w), 1385 (m), 1302 (w), 1244 (w), 1201 (m), 1177 (w), 1159 (m), 1130 (m), 1098 (w), 1021 (w), 974 (w), 940 (w), 888 (m), 850 (w), 804 (w), 769 (w), 683 (m), 668 (m), 641 (w), 546 (w).

### Vergleichsbeispiel 24:

### 9-Iodo-1-mercaptocarbonylphenyl-1,7-dicarba-closo-dodecaboran(12)

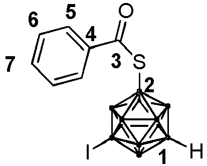

Es werden 250,0 mg (0,83 mmol, 1,0 Äq.) 9-Iodo-1-mercapto-1,7-dicarba-*closo-*dodecaboran(12) in 30 ml Dichlormethan gelöst und bei Raumtemperatur mit 0,33 ml (1,86 mmol, 3,0 Äq.) Diisopropylamin und anschließend mit 72 µl (87 mg, 0,62 mmol, 1,0 Äq.) Benzoylchlorid versetzt. Nach 24 Stunden wird die erhaltene weiße Suspension mit 30 ml destilliertem Wasser versetzt, mit verdünnter Salzsäure angesäuert und die entstehenden Phasen getrennt. Die organische Phase wird zweimal mit je 20 ml verdünnter Salzsäure gewaschen, mit destilliertem Wasser neutralisiert und mit gesättigter Natriumchloridlösung gewaschen.. Die organische Phase wird über Magnesiumsulfat getrocknet und das Lösungsmittel unter vermindertem Druck entfernt. Das Rohprodukt wird säulenchromatographisch gereinigt (Eluent: *n*-Hexan/Ethylacetat, 5:2 *v*/*v*). Das Produkt wird als farbloses Öl erhalten.

| | |
|---|---|
| Ausbeute: | 40 mg (0,1 mmol, 11 %) |
| R_{f}-Wert: | 0,83 (Eluent: *n*-Hexan/Ethylacetat 5:2 *v*/*v*) |

¹H-NMR (CDCl₃): δ = 1,17 - 4,24 (br m, 9H, B**H**), 3,16 (s, 1H, 1-C**H**), 7,47 (t, 2H, 6-C**H**, ³*J*_{HH} = 8,1 Hz), 7,62 (t, 1H, 7-C**H**, ³*J*_{HH} = 7,7 Hz), 7,82 (d, 2H, 5-C**H**, ³*J*_{HH} = 8,1 Hz) ppm.

¹¹B-NMR (CDCl₃): δ = -24,0 (s, 1B, **BI**), -15,6 (d, 2B, **B**H), -12,6 (d, 2B, **B**H), -8,1 (d, 3B, **B**H), -2,1 (d, 2B, **B**H) ppm.

¹³C{¹H}-NMR (CDCl₃): δ = 56,5 (s, 1-CH), 77,2 (s, 2-**C_{q}**), 127,2 (s, 5-**C**H), 129,1 (s, 6-**C**H), 134,5 (s, 7-**C**H), 135,1 (s, 4-**C**_{q}), 184,9 (s, 3-**C**H) ppm.

Massenspektrometrie (ESI pos., CH₃OH):

| | |
|---|---|
| Berechnet: | *m*/*z* = 406,1 |
| Ermittelt: | *m*/*z* = 429,1 (100%, [M + Na]⁺) |

IR-Spektroskopie (KBr, ṽ in cm⁻¹): 3053 (m), 2942 (w), 2608 (s), 1697 (s), 1596 (w), 1581 (w), 1448 (m), 1385 (w), 1202 (s), 1178 (m), 1130 (w), 1075 (w), 1000 (w), 931 (w), 888 (s), 806 (m), 769 (m), 786 (w), 684 (s), 641 (m), 615 (w).

### Vergleichsbeispiel 10:

### 1-(Chinolin-2-ylmethoxy)-1,7-dicarba-closo-dodecaboran(12)

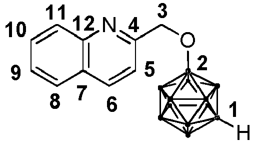

Es werden 300 mg (1,87 mmol, 1,0 Äq.) 1-Hydroxy-1,7-dicarba-*closo*-dodecaboran(12) sowie 415 mg (1,87 mmol, 1,0 Äq.) 2-(Brommethyl)chinolin in 30 ml Aceton gelöst und bei Raumtemperatur mit 0,78 g (5,61 mmol, 3,0 Äq.) Kaliumcarbonat versetzt und anschließend 24 Stunden unter Rückfluss erhitzt. Die erhaltene Suspension wird auf Raumtemperatur abgekühlt und mit 20 ml destilliertem Wasser sowie 10 ml gesättigter Natriumchloridlösung versetzt. Es wird dreimal mit je 20 ml Diethylether extrahiert, die vereinigten organischen Phasen über Magnesiumsulfat getrocknet und das Lösungsmittel unter vermindertem Druck entfernt. Der Rückstand wird auf Kieselgel aufgetragen und säulenchromatographisch gereinigt (Eluent: *n*-Hexan/Ethylacetat 5:1 *v*/*v*). Es wird ein farbloser Feststoff erhalten.

| | |
|---|---|
| Ausbeute: | 0,43 g (1,42 mmol, 76 %) |
| R_{f}-Wert: | 0,47 (Eluent: *n*-Hexan/Ethylacetat 5:1 *v*/*v*) |
| Schmelzpunkt: | 121,0 - 122,0 °C |

¹H-NMR (CDCl₃): δ = 1,22 - 3,74 (br m, 10H, B**H**), 2,84 (br s, 1H, 1-C**H**), 4,86 (s, 2H, 3-**C**H₂), 7,47 (d, 1H, 5-C**H**, ³*J*_{HH} = 8,4 Hz), 7,55 (t, 1H, 9-C**H**, ³*J*_{HH} = 8,2 Hz), 7,73 (t, 1H, 10-C**H**, ³*J*_{HH} = 8,2 Hz), 7,81 (d, 1H, 8-C**H**, ³*J*_{HH} = 8,2 Hz), 8,02 (d, 1H, 11-C**H**, ³*J*_{HH} = 8,7 Hz), 8,17 (d, 1H, 6-C**H**, ³*J*_{HH} = 8,4 Hz) ppm.

¹¹B{¹H}-NMR (CDCl₃): δ = -16,1 (s, 4B), -15,0 (s, 1B), -13,3 (s, 2B), -12,3 (s, 2B), -5,9 (s, 1B) ppm.

¹³C{¹H}-NMR (CDCl₃): δ = 50,7 (s, 1-**C**H), 76,6 (s, 3-**C**H₂), 107,0 (s, 2-**C_{q}**), 119,0 (s, 5-**C**H), 126,7 (s, 9-**C**H), 127,6 (s, 8-**C**H), 128,1 (s, 7-**C**_{q}), 128,9 (s, 11-**C**H), 129,9 (s, 10-**C**H), 137,0 (s, 6-**C**H), 147,3 (s, 12-**C**_{q}), 156,1 (s, 4-**C**_{q}) ppm.

Massenspektrometrie (ESI pos., Aceton):

| | |
|---|---|
| Berechnet: | *m*/*z* = 301,2 |
| Ermittelt: | *m*/*z* = 302,3 (100%, [M + H]⁺) |

IR-Spektroskopie (KBr, ṽ in cm⁻¹): 3445 (m), 2977 (s), 2624 (s), 2603 (s), 2570 (s), 1601 (m), 1507 (m), 1448 (m), 1428 (m), 1216 (s), 1051 (s), 1009 (m), 821 (s), 780 (m), 745 (m), 727 (m), 621 (w), 473 (w), 437 (w).

Elementaranalyse:

| | | |
|---|---|---|
| Berechnet für C₁₂H₁₉B₁₀N₁O₁: | C = 47,82 % | H = 6,35 % N = 4,65 % |
| Gefunden: | C = 47,23 % | H = 6,42 % N = 4,31 % |

### Vergleichsbeispiel 11:

### 1-(Chinolin-2-ylmethyl)thio-1,7-dicarba-closo-dodecaboran(12)

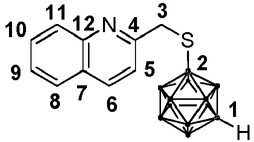

Es werden 300 mg (1,68 mmol, 1,0 Äq.) 1-Mercapto-1,7-dicarba-*closo*-dodecaboran(12) sowie 373 mg (1,68 mmol, 1,0 Äq.) 2-(Brommethyl)chinolin in 30 ml Aceton gelöst und bei Raumtemperatur mit 0,70 g (5,04 mmol, 3,0 Äq.) Kaliumcarbonat versetzt und anschließend 24 Stunden unter Rückfluss erhitzt. Die erhaltene Suspension wird auf Raumtemperatur abgekühlt und mit 20 ml destilliertem Wasser sowie 10 ml gesättigter Natriumchloridlösung versetzt. Es wird dreimal mit je 20 ml Diethylether extrahiert, die vereinigten organischen Phasen über Magnesiumsulfat getrocknet und das Lösungsmittel unter vermindertem Druck entfernt. Der Rückstand wird auf Kieselgel aufgetragen und säulenchromatographisch gereinigt (Eluent: *n*-Hexan/Ethylacetat 5:1 *v*/*v*). Es wird eine gelbliche Flüssigkeit erhalten.

| | |
|---|---|
| Ausbeute: | 0,42 g (1,32 mmol, 79 %) |
| R_{f}-Wert: | 0,63 (Eluent: Hexan/Ethylacetat 5:1 *v*/*v*) |
| Schmelzpunkt: | 127,0 - 128,0 °C |

¹H-NMR (Aceton-d₆): δ = 1,50 - 3,55 (br m, 10H, B**H**), 3,79 (br s, 1H, 1-C**H**), 4,43 (s, 2H, 3-C**H₂**), 7,60 (t, 1H, 9-C**H**, ³*J*_{HH} = 7,2 Hz), 7,61 (d, 1H, 5-C**H**, ³*J*_{HH} = 8,4 Hz), 7,77 (t, 1H, 10-C**H**, ³*J*_{HH} = 8,6 Hz), 7,95 (d, 1H, 8-C**H**, ³*J*_{HH} = 8,1 Hz), 8,00 (d, 1H, 11-C**H**, ³*J*_{HH} = 8,6 Hz), 8,32 (d, 1H, 6-C**H**, ³*J*_{HH} = 8,4 Hz) ppm.

¹¹B{¹H}-NMR (Aceton-d₆): δ = -14,3 (s, 2B), -13,3 (s, 2B), -10,7 (s, 3B), -10,1 (s, 2B), -3,5 (s, 1B) ppm.

¹³C{¹H}-NMR (Aceton-d₆): δ = 42,8 (s, 3-**C**H₂), 56,5 (s, 1-**C**H), 72,6 (s, 2-**C_{q}**), 120,9 (s, 5-**C**H), 126,6 (s, 9-**C**H), 127,1 (s, 7-**C**_{q}), 127,7 (s 8-**C**H), 128,7 (s, 11-CH), 129,7 (s, 10-**C**H), 136,8 (s, 6-**C**H), 147,5 (s, 12-**C**_{q}), 155,9 (s, 4-**C**_{q}) ppm.

Massenspektrometrie (ESI pos., CHCl₈/CH₈OH):

| | |
|---|---|
| Berechnet: | *m*/*z* = 317,2 |
| Ermittelt: | *m*/*z* = 340,2 (100%, [M + Na]⁺), 318,2 (20%, [M + H]⁺) |

IR-Spektroskopie (KBr, ṽ in cm⁻¹): 3428 (s), 2625 (s), 2602 (s), 2590 (s), 1598 (m), 1505 (m), 1427 (m), 1262 (w), 1122 (w), 1084 (w), 877 (w), 840 (w), 802 (w), 770 (m), 729 (w), 618 (w), 498 (w), 472 (w).

Elementaranalyse:

| | | |
|---|---|---|
| Berechnet für C₁₂H₁₉B₁₀N₁S₁: | C = 45,40 % | H = 6,03 % N = 4,41 % |
| Gefunden: | C = 45,69 % | H = 6,03 % N = 4,29 % |

### Vergleichsbeispiel 30:

### 1-Chinolin-2-ylmethoxy-1,12-dicarba-closo-dodecaboran(12)

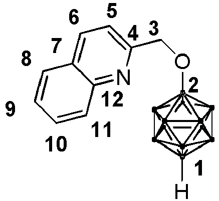

Es werden 300 mg (1,87 mmol, 1,0 Äq.) 1-Hydroxy-1,12-dicarba-*closo*-dodecaboran(12) sowie 415 mg (1,87 mmol, 1,0 Äq.) 2-(Brommethyl)chinolin in 30 ml Aceton gelöst und bei Raumtemperatur mit 0,78 g (5,61 mmol, 3,0 Äq.) Kaliumcarbonat versetzt und anschließend 24 Stunden unter Rückfluss erhitzt. Die erhaltene Suspension wird auf Raumtemperatur abgekühlt und filtriert. Die vereinigten organischen Phasen werden über Magnesiumsulfat getrocknet und das Lösungsmittel unter vermindertem Druck entfernt. Der Rückstand wird auf Kieselgel aufgetragen und säulenchromatographisch gereinigt (Eluent: Hexan/Ethylacetat 5:1 *v*/*v*). Es wird ein farbloser Feststoff erhalten.

| | |
|---|---|
| Ausbeute: | 0,40 g (1,38 mmol, 74 %) |
| R_{f}-Wert: | 0,43 (Eluent: Hexan/Ethylacetat 10:1 *v*/*v*) |
| Schmelzpunkt: | 110,3 - 111,2 °C |

¹H-NMR (CDCl₃): δ = 1,20 - 3,30 (br m, 10H, B**H**), 2,56 (br s, 1H, 1-C**H**), 4,67 (s, 2H, 3-**C**H₂), 7,37 (d, 1H, 5-C**H**, ³*J*_{HH} = 8,5 Hz), 7,53 (t, 1H, 9-C**H**, ³*J*_{HH} = 8,0 Hz), 7,70 (t, 1H, 10-C**H**, ³*J*_{HH} = 8,5 Hz), 7,79 (d, 1H, 8-C**H**,³*J*_{HH} = 8,0 Hz), 7,99 (d, 1H, 11-C**H**, ³*J*_{HH} = 8,5 Hz), 8,15 (d, 1H, 6-C**H**, ³*J*_{HH} = 8,5 Hz) ppm.

¹¹B{¹H}-NMR (CDCl₃): δ = -13,6 (s, 2B),-17,4 (s, 5B), -13,7 (s, 5B) ppm.

¹³C{¹H}-NMR (CDCl₃): δ = 50,2 (s, 1-**C**H), 75,6 (s, 3-**C**H₂), 113,9 (s, 2-**C_{q}**), 118,9 (s, 5-**C**H), 126,5 (s, 9-**C**H), 127,5 (s, 7-**C**_{q}), 127,6 (s, 8-**C**H), 128,7 (s-11-**C**H), 129,7 (s, 10-**C**H), 137,1 (s, 6-CH), 147,1 (s, 12-**C**_{q}), 156,3 (s, 4-**C**_{q}) ppm.

Massenspektrometrie (ESI pos., CH₂Cl₂/CH₃OH):

| | |
|---|---|
| Berechnet: | *m*/*z* = 301,2 |
| Ermittelt: | *m*/*z* = 302,3 (100%, [M + H]⁺), 324,2 (10%, [M + Na]⁺) |

IR-Spektroskopie (KBr, ṽ in cm⁻¹): 3387 (s), 2957 (s), 2928 (s), 2859 (m), 2602 (s), 1461 (w), 1253 (w), 1137 (s), 842 (m), 732 (w).

Elementaranalyse:

| | | |
|---|---|---|
| Berechnet für C₁₈H₂₈B₁₀O₂S₁: | C = 51,90 % | H = 6,77 % |
| Gefunden: | C = 53,23 % | H = 6,92 % |

### Löslichkeitstests

Zur Bestimmung der Löslichkeit wurden Löslichkeitstests in Ethanol und DMSO durchgeführt. Dabei wurden 4 mg der Verbindungen in einem Eppendorfgefäß mit der Menge an Lösungsmittel versetzt, die nötig war, um eine klare Lösung zu erhalten. Anschließend wurde anhand der Masse der Verbindungen und der Lösungsmittelmenge die Konzentration der Lösung bestimmt. Die Ergebnisse der Tests sind in **Tabelle 1** dargestellt.

**Tabelle 1**

| Löslichkeitsverhalten | | | |
|---|---|---|---|
| Molekül | Ausführungsbeispiel | Löslichkeit EtOH | Löslichkeit DMSO |
| 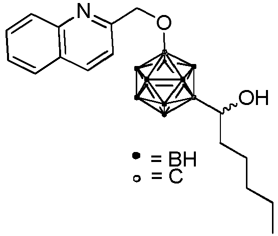 | 3 | 86,8 mmol/L | 208,3 mmol/L |
| 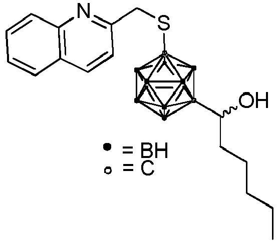 | 5 | 238,5 mmol/L | 953,9 mmol/L |
| 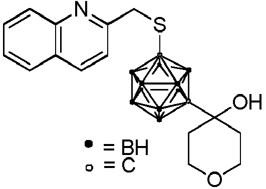 | 8 | 155 mmol/L | 619 mmol/L |
| 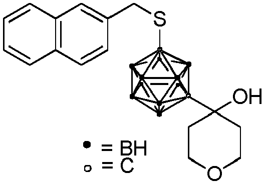 | 9 | 76 mmol/L | 228 mmol/L |
| 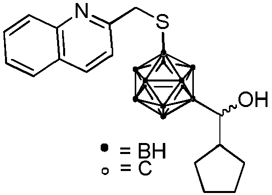 | 12 | 57,7 mmol/L | 144,4 mmol/L |
| 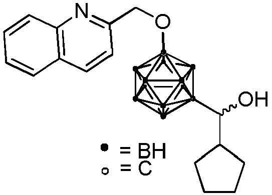 | 14 | 38,9 mmol/L | 87,6 mmol/L |
| 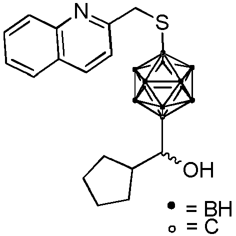 | 16 | 24,06 mmol/L | 132,3 mmol/L |
| 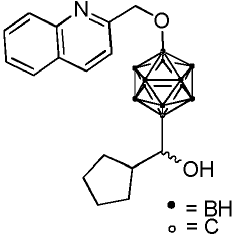 | 18 | 21,2 mmol/L | 91,8 mmol/L |
| 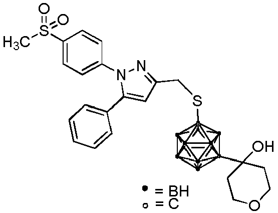 | 19 | 204.50 mmol/L | 409.00 mmol/L |

| | | | |
|---|---|---|---|
| 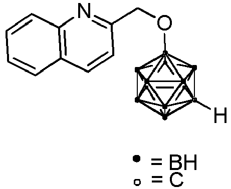 | 10 | 72 mmol/L | 432 mmol/L |
| 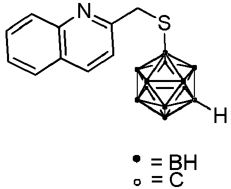 | 11 | 67 mmol/L | 600 mmol/L |

### Inhibitionstests

Zur Bestimmung der Inhibitionswirkung wurden IC₅₀-Tests mit den Verbindungen aus Ausführungsbeispiel 3, 5, 8 und 9 sowie der Vergleichsbeispiele 10 und 11 im Vergleich zur Referenz Rev-5901 durchgeführt.
Es wurde die 5-LOX-Aktivität der Zellen in Abhängigkeit von der Konzentration an Inhibitor ermittelt.
Dabei wurden gemäß einer literaturbekannten Vorschrift (C. Greiner et al., British Journal of Pharmacology, 2011, 164, 781-793) intakte polymorphonukleare Leukocyten aus Blut isoliert. Die Zellen wurden auf Zellplatten gezüchtet, bis eine Konfluenz von 95% erreicht wurde, also 95% der Zellplatte eine Bedeckung mit Zellen aufwies. Jede Zellplatte wurde anschließend mit je 10 µl einer Lösung des Inhibitors mit einer Konzentration von 0,05 µM, 0,1 µM, 0,5 µM, 1µM, 5 µM, 10 µM, 50 µM bzw. 100 µM bei 37 °C für 15 min behandelt. Anschließend wurde sie jeweils für 10 min mit einer Lösung aus Arachidonsäure behandelt. Dabei wurden 10 µl einer 1:1-Mischung 2 mM Arachidonsäure und 250 µMA23187 Ca²⁺ ionophore Lösung in Ethanol verwendet. Anschließend wurde jeweils mittels HPLC geprüft, wieviel Arachidonsäure durch das Enzym im Vergleich zu einer Probe ohne Inhibitor umgewandelt wurde.
Eine geringe Umsetzung von Arachidonsäure belegt eine starke Inhibitionswirkung.
Die Konzentration an Inhibitor, bei der nur noch 50% der Arachidonsäure umgesetzt werden, bezeichnet man als IC₅₀-Wert (Enzym ist also zu 50% gehemmt). Die IC₅₀-Werte für die jeweilige Verbindung sind in **Tabelle 2** dargelegt.

**Tabelle 2**

| Ergebnisse der Inhibitionstests | |
|---|---|
| **Verbindung/** | **IC₅₀-Wert** |
| **Ausführungsbeispiel** | (bei Konzentration) |
| Vergleichsbeispiel: | 0,58 µM |
| **Rev-5901** | |
| Ausführungsbeispiele: | |
| **3** | 1,4 µM |
| **5** | 2,5 µM |
| **8** | 3,8 µM |
| **9** | 5,1 µM |

| **Verbindung/** | **IC₅₀-Wert** |
|---|---|
| **Vergleichsbeispiel** | (bei Konzentration) |
| **Rev-5901** | 0,58 µM |
| **10** | 2,3 µM |
| **11** | 5,8 µM |

Es ist eindeutig zu erkennen, dass sämtliche dargestellte Verbindungen in der Lage sind, das Enzymsystem 5-LOX-FLAP effizient zu hemmen. Die IC₅₀-Werte aller Clusteranaloga liegen im Bereich der Referenz.

Die Ergebnisse der Inhibitionstests sind in **Abb. 1 bis Abb. 3** graphisch dargelegt. Dabei wurde die Enzymaktivität gegen die Konzentration des entsprechenden Inhibitors aufgetragen.
In **Abb. 1** sind die Enzymaktivitätswerte gegen die Konzentration an den Inhibitoren aus Ausführungsbeispiel 3 (Symbol: •) und 5 (Symbol: ▪) und dem Inhibitor Rev-5901 (Symbol: ▲ ) aufgetragen.
In **Abb. 2** sind die Enzymaktivitätswerte gegen die Konzentration an den Inhibitoren aus Ausführungsbeispiel 8 (Symbol: ▲) und 9 (Symbol: ▼) aufgetragen.
In **Abb. 3** sind die Enzymaktivitätswerte gegen die Konzentration an den Inhibitoren aus Vergleichsbeispiel 10 (Symbol: ▪) und 11 (Symbol: •) aufgetragen.

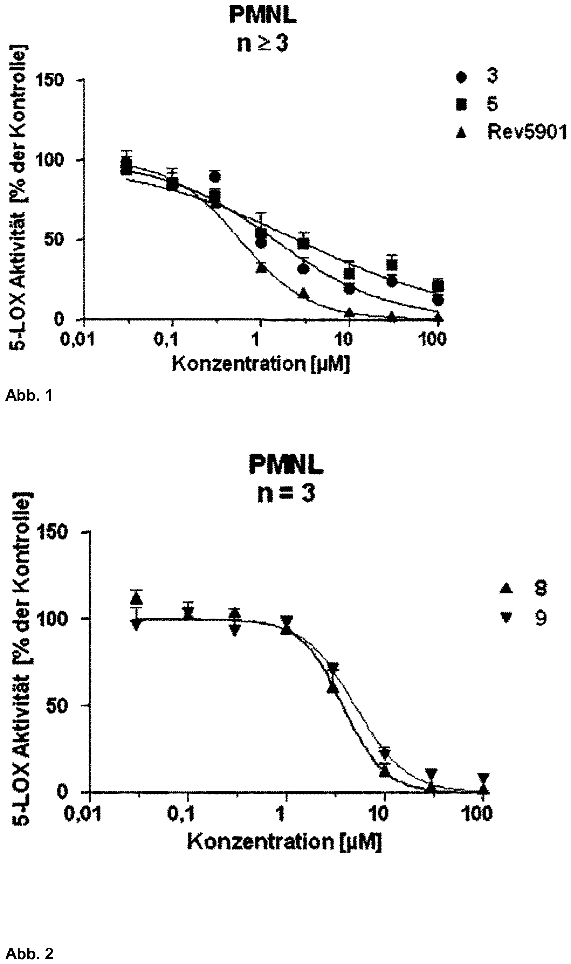

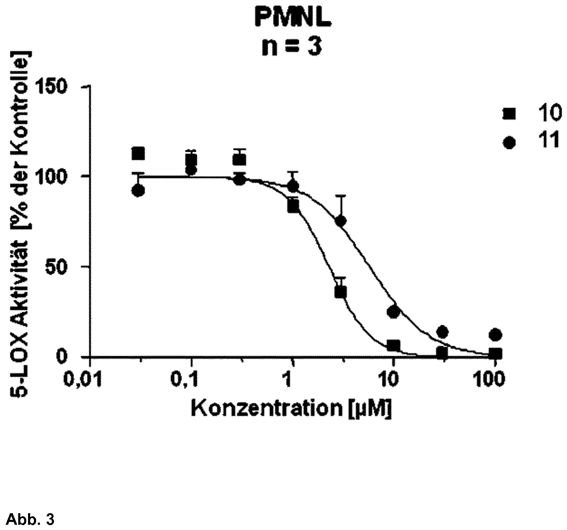

Man sieht an den Kurven, dass die Inhibitoren bei einer Konzentration von 1-6 µM die Aktivität des Enzymsystems halbieren und bei höherer Konzentration sogar eine vollständige Inhibition erreichen. Besonders ragen die Ausführungsbeispiele **3** (Abb. 1) und Vergleichsbeispiel 10 (Abb. 3) heraus, die bereits bei 1,4 bzw. 2,3 µM das System zu 50% hemmen. An Vergleichsbeispiel **10** ist besonders, dass es eine sehr kleine, nur monofunktionalisierte Verbindung ist, die trotzdem eine effiziente Enzyminhibition bewirkt. Die Vergleichsbeispiele **10** und **11** zeigen sogar eine verbesserte Inhibitionswirkung gegenüber den aus der Literatur bekannten phenylsubstituierten LOX-Inhibitoren. Trotzdem zeigt sich im Cytotoxizitätstest, dass dies nicht ausreicht, um einen cytotoxischen Effekt auf diverse Krebszelllinien zu bewirken.

### Cytotoxizitätstests

Zur Bestimmung der Cytotoxizität gegenüber diversen Krebszelllinien wurden MTT- sowie Kristallviolett(CV)-Assays durchgeführt. Der MTT-Assay erfolgt nach einer Vorschrift von T. Mosmann (T. Mosmann, J. Immunol. Methods, 1983, 65, 55), der CV-Test nach einer Vorschrift von D. A. Flick *et al.* (D. A. Flick, G. E. Gifford, J. Immunol. Methods, 1984, 68, 167).

Zur Bestimmung der selektiven Cytotoxizität der erhaltenen Verbindungen aus den Ausführungsbeispielen 3, 5, 8 und 9 sowie der Vergleichsbeispiele 10 und 11 wurden Untersuchungen an Krebszelllinien und an primären Fibroblasten (MRC-5), welche aus humanem Lungengewebe isoliert wurden, im Vergleich zur Referenzverbindung Rev-5901 durchgeführt. Dabei wurde der prozentuale Anteil toter Zellen in Abhängigkeit der Konzentration der Inhibitoren untersucht. Der erhaltene IC₅₀-Wert kennzeichnet dabei die Konzentration eines bestimmten Inhibitors, bei dem 50% der untersuchten Zellen absterben.

Der Anteil lebender Zellen wurde durch den MTT (3-(4, 5-Dimethylthiazolyl-2)-2,5-diphenyltetrazoliumbromid) und den Kristallviolett (CV) Assay bestimmt. Im MTT-Assay wird das Vorhandensein von NADH und NADPH nachgewiesen. Diese beiden Verbindungen sind charakteristische Stoffwechselprodukte bei funktionierender Atmungskette. NADH und NADPH reduzieren dabei den Farbstoff MTT, was zu einer Farbänderung führt, welche gemessen werden kann und damit direkt proportional zur Anzahl lebender Zellen ist.

Im Kristallviolett Assay hingegen kann der Farbstoff Kristallviolett nur durch die perforierten Zellmembranen toter Zellen diffundieren. In diesem Falle steht die Anzahl angefärbter Zellen für die Anzahl toter Zellen.

Es wurden drei Hautkrebszelllinien (A375, B16, B16F10) sowie drei Darmkrebszelllinien (CT26CL25, HCT116, SW480) untersucht. Als Beispiel für gesunde Zellen und somit Negativkontrolle dienten primäre Fibroblasten aus humanem Lungengewebe (MRC-5). Die Ergebnisse sind in den Tabellen 3 a bis c dargestellt.

### Ergebnisse der Cytotoxizitätstests

**Tabelle 3a**

| Molekül | Ausführungsbeispiel | IC₅₀ MRC-5 | IC₅₀ A375 | IC₅₀ B16 | IC₅₀ HCT116 | IC₅₀ CT26CL2 5 | IC₅₀ SW48 0 |
|---|---|---|---|---|---|---|---|
| Rev-5901 | - Referenzsubstanz | 70.10 µM | 25 µM | 27.57 µM | 50 µM | 28.05 µM | 50 µM |
| | | - | 46.45 µM | 44.95 µM | 50 µM | >50 µM | >50 µM |
| 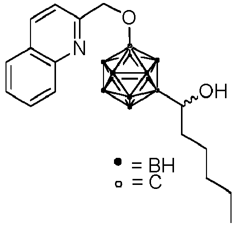 | 3 | >100 | 8,8 µM | 45,7 µM | 50 µM | 35 µM | 29 µM |
| | | | 26,9 µM | 46 µM | >50 µM | 50 µM | 50 µM |
| 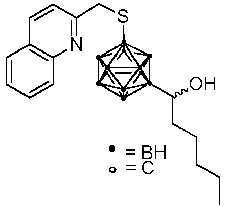 | 5 | 82,0 | 8 µM | 50 µM | 45 µM | 46 µM | 33,1 µM |
| | | | 30,5 µM | >50 µM | >50 µM | 50 µM | >50 µM |
| 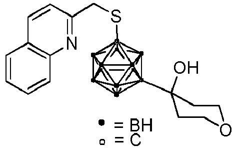 | 8 | | 4,7 µM | 30,7 µM | 28 µM | 10,8 µM | 8,5 µM |
| | | | 8,7 µM | 44,3 µM | 39,8 µM | 18,6 µM | 9,9 µM |

Der obere Wert jeder Zelle steht jeweils für den IC₅₀-Wert im MTT-Assay, der untere Wert für den IC₅₀-Wert im CV-Assay (Kristallviolett)

**Tabelle 3b**

| Molekül | Ausführungsbeispiel | IC₅₀ MRC-5 | IC₅₀ A375 | IC₅₀ B16 | IC₅₀ HCT116 | IC₅₀ CT26 CL25 | IC₅₀ SW48 0 |
|---|---|---|---|---|---|---|---|
| 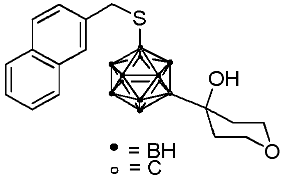 | 9 | | 42,4 µM | 50 µM | 50 µM | 21,4 µM | 28 µM |
| | | | >50 µM | >50 µM | 50 µM | 22,4 µM | 40 µM |
| 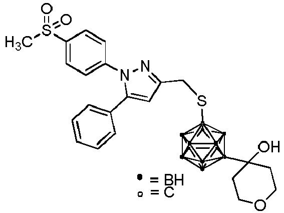 | 19 | | 10.4-11.8 µM | | | | 10.8-12.5 µM |
| | | | 15.1-17.8 µM | | | | 25.8-28.6 µM |

Der obere Wert jeder Zelle steht jeweils für den IC₅₀-Wert im MTT-Assay, der untere Wert für den IC₅₀-Wert im CV-Assay (Kristallviolett)

**Tabelle 3c**

| Molekül | Vergleichsbeispiel | IC₅₀ MRC-5 | IC₅₀ A375 | IC₅₀ B16 | IC₅₀ HCT116 | IC₅₀ CT26-CL25 | IC₅₀ SW480 |
|---|---|---|---|---|---|---|---|
| Rev-5901 | - Referenzsubstanz | 70.10 µM | 25 µM | 27.57 µM | 50 µM | 28.05 µM | 50 µM |
| | | - | 46.45 µM | 44.95 µM | 50 µM | >50 µM | >50 µM |
| 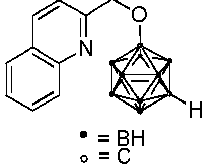 | 10 | | 50 µM | 47,4 µM | >50 µM | >50 µM | >50 µM |
| | | | >50 µM | 47,6 µM | 50 µM | >50 µM | >50 µM |
| 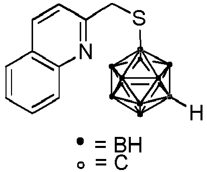 | 11 | | 30 µM | >50 µM | >50 µM | >50 µM | 50 µM |
| | | | 34,8 µM | >50 µM | >50 µM | >50 µM | >50 µM |

Der obere Wert jeder Zelle steht jeweils für den IC₅₀-Wert im MTT-Assay, der untere Wert für den IC₅₀-Wert im CV-Assay (Kristallviolett)

## Patentansprüche

1. Chemische Verbindung der allgemeinen Struktur
[A - R₃ - X - R₄]
wobei
A = [R₁ - R₂] oder [R₁]
R₁ = Aryl mit 6 - 20 C-Atomen oder Heteroaryl mit 2-20 C-Atomen
R₂ = C1-C5-Alkyl, Carbonyl,
R₃ = O, S,
X = *closo*- oder *nido*-Borcluster 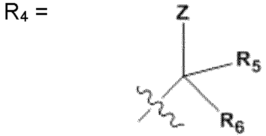
wobei Z = OH"
wobei R₅ ausgewählt ist aus H, Alkyl, Aryl, Heteroaryl, Alkylether, Alkylthioether, Alkylamin und
R₆ ausgewählt ist aus Alkyl, Aryl, Heteroaryl, Alkylether, Alkylthioether, Alkylamin
oder R₅ und R₆ eine Tetrahydropyranyl-Einheit bilden,
und wobei R₃ und R₄ in *meta-* oder *para-*Position zueinander stehen.

2. Chemische Verbindung nach Anspruch 1, **gekennzeichnet dadurch, dass** R₁ ausgewählt ist aus Chinolin- oder Naphthylsubstituenten.

3. Chemische Verbindung nach einem der Ansprüche 1 bis 2, **gekennzeichnet dadurch, dass** X ausgewählt ist aus C₂B₈H₁₀, C₂B₁₀H₁₂, Si₁₂B₁₀H₁₂, P₂B₁₀H₁₀, SB₁₁H₁₁, NB₁₁H₁₁⁽⁻⁾, PB₁₁H₁₁⁽⁻⁾, CB₆H₇⁽⁻⁾, CB₇H₈⁽⁻⁾, CB₉H₁₀⁽⁻⁾, CB₉H₁₂⁽⁻⁾, CB₁₀H₁₁⁽⁻⁾, CB₁₁H₁₂⁽⁻⁾, SiB₁₁H₁₂⁽⁻⁾, CB₁₁H₁₁⁽²⁻⁾, SiB₁₁H₁₁⁽²⁻⁾, SnB₁₁H₁₁⁽²⁻⁾, GeB₁₁H₁₁⁽²⁻⁾, C₂B₉H₁₂, C₂B₉H₁₂⁽⁻⁾, C₂B₉H₁₁⁽²⁻⁾ RₐC₃BₙHₙ₊₃₋ₐ⁽⁻⁾, RC₂BₙHₙ₊₂₋ₐ⁽⁻⁾, C₃B₈H₁₁⁽⁻⁾, R₂C₃B₈H₉⁽⁻⁾, C₂B₉H₁₁⁽⁻⁾, R₂C₂B₉H₉⁽⁻⁾ (mit R = H, Alkyl, Aryl, Silyl).

4. Chemische Verbindung nach einem der Ansprüche 1 bis3, wobei mindestens eine BH-Einheit durch eine radiomarkierte B-Hal-Einheit substituiert ist.

5. Verfahren zur Herstellung einer chemischen Verbindung der allgemeinen Formel
[A - R₃ - X - R₄]
wobei
A = [R₁ - R₂] oder [R₁]
R₁ = Aryl mit 6-20 C-Atomen oder Heteroaryl mit 2-20 C-Atomen
R₂ = C1-C5-Alkyl, Carbonyl,
R₃ = O, S,
X = *closo*- oder *nido*-Borcluster 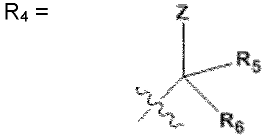
wobei Z = OH"
wobei R₅ ausgewählt ist aus H, Alkyl, Aryl, Heteroaryl, Alkylether, Alkylthioether, Alkylamin und
R₆ ausgewählt ist aus Alkyl, Aryl, Heteroaryl, Alkylether, Alkylthioether, Alkylamin
oder R₅ und R₆ eine Tetrahydropyranyl-Einheit bilden,
und wobei R₃ und R₄ in *meta-* oder para-Position zueinander stehen,
mit den Schritten:
a) Hydroxyalkylierung des Clusters X
b) Hydroxylierung oder Thiolierung des Clusters X,
zu einer Zwischenverbindung der allgemeinen Formel [H-R₃ - X - R₄]
wobei
R₃ = O, S
X = *closo*- oder *nido-* Borcluster 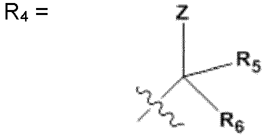
wobei Z = OH
wobei R₅ ausgewählt ist aus H, Alkyl, Aryl, Heteroaryl, Alkylether, Alkylthioether, Alkylamin und
R₆ ausgewählt ist aus Alkyl, Aryl, Heteroaryl, Alkylether, Alkylthioether, Alkylamin
oder R₅ und R₆ eine Tetrahydropyranyl-Einheit bilden,
und wobei R₃ und R₄ in *meta-* oder *para-* Position zueinander stehen,
und
c) Selektive Veretherung oder Veresterung von H-R₃ zur Einführung von A,
wobei die Schritte a) und b) beliebig vertauscht werden können.

6. Verwendung einer chemischen Verbindung nach einem der Ansprüche 1 bis 4 oder deren Salz, zur Inhibition oder Modulation von Lipoxygenasen außerhalb des menschlichen oder tierischen Körpers.

7. Verwendung einer chemischen Verbindung nach einem der Ansprüche 1 bis 4 oder deren Salz für die Imitation von Arachidonsäure und deren Abkömmlinge in biologischen Systemen außerhalb des menschlichen oder tierischen Körpers.

8. Verwendung einer chemischen Verbindung nach einem der Ansprüche 1 bis 4 oder deren Salz in Imaging-Verfahren.

9. Chemische Verbindung nach einem der Ansprüche 1 bis 4 oder deren Salz zur Verwendung als Arzneimittel.

10. Chemische Verbindung nach einem der Ansprüche 1 bis 4 oder deren Salz zur Verwendung als Arzneimittel zur Behandlung von Asthma, von Krankheiten des ZNS (Zentrales Nervensystem), Allergien, Rhinitis, Herz-Kreislauf-Erkrankungen, bei der Schmerzvermittlung, der Alzheimer-Krankheit, des Sehprozesses, bei Krebsleiden, und Beschwerden in Magen-Darm-, Nieren-, Gefäßbereich und Schwangerschaftsstörungen.

11. Chemische Verbindung nach einem der Ansprüche 1 bis 4 oder deren Salz zur Verwendung für diagnostische oder therapeutische Verfahren.

12. Chemische Verbindung nach Anspruch 4 oder deren Salz zur Verwendung für BNCT (boron neutron capture therapy), BNCS (boron neutron capture synovectomy), MRI (magnetic resonance imaging), PET (positron emission tomography), SPECT (single-photon emission computed tomography), PIGE (Particle induced γ-ray emission) und AFM-NIAR (atomic force microscopy with neutron-induced alpha-autoradiography).

13. Pharmazeutische Zusammensetzung enthaltend eine oder mehrere Verbindungen nach den Ansprüchen 1 bis 4 oder deren Salze, in Verbindung mit einem pharmazeutisch verträglichen Träger.

## Claims

1. Chemical compound with general structure
[A - R₃ - X - R₄]
wherein
A = [R₁ - R₂] or [R₁]
R₁ = aryl with 6 to 20 C-atoms or heteroaryl with 2 to 20 C-atoms
R₂ = C1-C5-alkyl, carbonyl,
R₃ = O, S,
X = *closo*- or nido-boron cluster 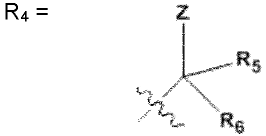
wherein Z = OH,
wherein R₅ is selected from H, akyl, aryl, heteroaryl, alkyl ether, alkyl thioether, and alkyl amin, and
R₆ is selected from alkyl, aryl, heteroaryl, alkyl ether, alkyl thioether, and alkyl amin,
or R₅ and R₆ form a tetrahydropyranyl unit, and
wherein R₃ and R₄ are in *meta* or *para* positions in relation to one another.

2. Chemical compound according to claim 1, **characterized in that** R₁ is selected from quinolin or naphthyl substituents.

3. Chemical compound according to one of claims 1 to 2, **characterized in that** X is selected from C₂B₈H₁₀, C₂B₁₀H₁₂, Si₂B₁₀H₁₂, P₂B₁₀H₁₀, SB₁₁H₁₁, NB₁₁H₁₁⁽⁻⁾, PB₁₁H₁₁⁽⁻⁾, CB₆H₇⁽⁻⁾, CB₇H₈⁽⁻⁾, CB₉H₁₀⁽⁻⁾, CB₉H₁₂⁽⁻⁾, CB₁₀H₁₁⁽⁻⁾, CB₁₁H₁₂⁽⁻⁾, SiB₁₁H₁₂⁽⁻⁾, CB₁₁H₁₁⁽²⁻⁾, SiB₁₁H₁₁⁽²⁻⁾, SnB₁₁H₁₁⁽²⁻⁾, GeB₁₁H₁₁⁽²⁻⁾, C₂B₉H₁₂, C₂B₉H₁₂⁽⁻⁾, C₂BgH₁₁⁽²⁻⁾ RₐC₃BₙHₙ₊₃₋ₐ⁽⁻⁾, RC₂BₙHₙ₊₂₋ₐ⁽⁻⁾, C₃B₈H₁₁⁽⁻⁾, R₂C₃B₈H₉⁽⁻⁾, C₂B₉H₁₁⁽⁻⁾, R₂C₂B₉H₉⁽⁻⁾ (with R = H, alkyl, aryl, silyl).

4. Chemical compound according to one of claims 1 to 3, wherein at least one BH unit is substituted by a radiolabelled B-halogen unit.

5. Process for preparing a compound with general structure
[A - R₃ - X - R₄]
wherein
A = [R₁ - R₂] or [R₁]
R₁ = aryl with 6 to 20 C-atoms or heteroaryl with 2 to 20 C-atoms
R₂ = C1-C5-alkyl, carbonyl,
R₃ = O, S,
X = *closo*- or nido-boron cluster 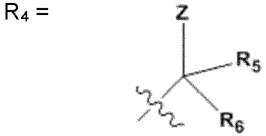
wherein Z = OH,
wherein R₅ is selected from H, akyl, aryl, heteroaryl, alkyl ether, alkyl thioether, and alkyl amin, and
R₆ is selected from alkyl, aryl, heteroaryl, alkyl ether, alkyl thioether, and alkyl amin,
or R₅ and R₆ form a tetrahydropyranyl unit, and
wherein R₃ and R₄ are in *meta* or *para* positions in relation to one another,
with the steps:
a) hydroxyalkylating the cluster X
b) hydroxylating or thiolating the cluster X,
so as to form an intermediate compound of general formula [H-R₃ - X - R₄]
wherein
R₃ = O, S
X = *closo*- or *nido-*boron cluster 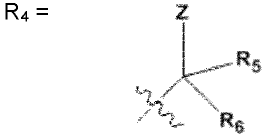
wherein Z = OH,
wherein R₅ is selected from H, akyl, aryl, heteroaryl, alkyl ether, alkyl thioether, and alkyl amin, and
R₆ is selected from alkyl, aryl, heteroaryl, alkyl ether, alkyl thioether, and alkyl amin,
or R₅ and R₆ form a tetrahydropyranyl unit, and
wherein R₃ and R₄ are in *meta* or *para* positions in relation to one another,
and
c) selectively etherifying or esterifying H-R₃ in order to introduce A,
wherein steps a) and b) can be interchanged as desired.

6. Use of the chemical compound according to one of claims 1 to 4 or its salt, for inhibiting or modulating lipoxygenases outside the human or animal body.

7. Use of the chemical compound according to one of claims 1 to 4 or its salt, for imitating arachidonic acid and derivatives thereof in biological systems outside the human or animal body.

8. Use of the chemical compound according to one of claims 1 to 4 or its salt, in imaging methods.

9. Chemical compound according to one of claims 1 to 4 or its salt, for use as pharmaceutical product.

10. Chemical compound according to one of claims 1 to 4 or its salt, for use as pharmaceutical product for the treatment of asthma, disorders of the CNS (central nervous system), allergies, rhinitis, cardiovascular diseases, in pain mediation , Alzheimer's disease, the visual process, cancer, and in stomach/intestinal, renal, vascular complaints and complications of pregnancy.

11. Chemical compound according to one of claims 1 to 4 or its salt, for use in diagnostic or therapeutic methods.

12. Chemical compound according to claim 4 or its salt, for use in BNCT (boron neutron capture therapy), BNCS (boron neutron capture synovectomy), MRI (magnetic resonance imaging), PET (positron emission tomography), SPECT (single-photon emission computed tomography), PIGE (Particle induced γ-ray emission) and AFM-NIAR (atomic force microscopy with neutron-induced alpha-autoradiography).

13. Pharmaceutical composition containing one or more compounds according to claims 1 to 4 or their salts in conjunction with a pharmaceutically acceptable carrier.

## Revendications

1. Composé chimique de structure générale
[A - R₃- X - R₄]
dans laquelle
A = [R₁ - R₂] ou [R₁]
R₁ = un aryle comportant 6 à 20 atomes de C ou un hétéroaryle comportant 2 à 20 atomes de C
R₂ = un alkyle en C1-C5, un carbonyle,
R₃ = O, S,
X = un agrégat *closo* ou *nido* de bore 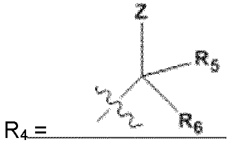
dans laquelle Z = OH,
dans laquelle R₅ est choisi parmi H, un alkyle, un aryle, un hétéroaryle, un alkyléther, un alkylthioéther, une alkylamine et
R₆ est choisi parmi un alkyle, un aryle, un hétéroaryle, un alkyléther, un alkylthioéther, une alkylamine
ou R₅ et R₆ forment un motif tétrahydropyranyle,
et dans laquelle R₃ et R₄ sont en position *méta* ou *para* l'un par rapport à l'autre.

2. Composé chimique selon la revendication 1, **caractérisé en ce que** R₁ est choisi parmi les substituants quinoléine ou naphtyle.

3. Composé chimique selon l'une des revendications 1 à 2, **caractérisé en ce que** X est choisi parmi C₂B₈H₁₀**,** C₂B₁₀H₁₂, Si₁₂B₁₀H₁₂, P₂B₁₀H₁₀, SB₁₁H₁₁, NB₁₁H₁₁⁽⁻⁾, PB₁₁H₁₁⁽⁻⁾, CB₆H₇⁽⁻⁾, CB₇H₈⁽⁻⁾, CB₉H₁₀⁽⁻⁾, CB₉H₁₂⁽⁻⁾, CB₁₀H₁₁⁽⁻⁾, CB₁₁H₁₂⁽⁻⁾, SiB₁₁H₁₂⁽⁻⁾ CB₁₁H₁₁⁽²⁻⁾, SiB₁₁H₁₁⁽²⁻⁾, SnB₁₁H₁₁⁽²⁻⁾, GeB₁₁H₁₁⁽²⁻⁾, C₂B₉H₁₂, C₂B₉H₁₂⁽⁻⁾, C₂B₉H₁₁⁽²⁻⁾ RₐC₃BₙHₙ₊₃₋ₐ⁽⁻⁾, RC₂BₙHₙ₊₂₋ₐ⁽⁻⁾, C₃B₈H₁₁⁽⁻⁾, R₂C₃B₈H₉⁽⁻⁾, C₂B₉H₁₁⁽⁻⁾, R₂C₂B₉H₉⁽⁻⁾ (avec R = H, alkyle, aryle, silyle).

4. Composé chimique selon l'une des revendications 1 à 3, dans lequel au moins un motif BH est remplacé par un motif B-Hal radiomarqué.

5. Procédé de préparation d'un composé chimique de formule générale
[A - R₃ - X - R₄]
dans laquelle
A = [R₁ - R₂] ou [R₁]
R₁ = un aryle comportant 6 à 20 atomes de C ou un hétéroaryle comportant 2 à 20 atomes de C
R₂ = un alkyle en C1-C5, un carbonyle,
R₃ = O, S,
X = un agrégat *closo* ou *nido* de bore 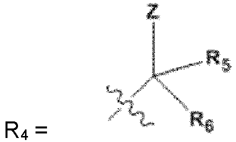
dans laquelle Z = OH,
dans laquelle R₅ est choisi parmi H, un alkyle, un aryle, un hétéroaryle, un alkyléther, un alkylthioéther, une alkylamine et
R₆ est choisi parmi un alkyle, un aryle, un hétéroaryle, un alkyléther, un alkylthioéther, une alkylamine
ou R₅ et R₆ forment un motif tétrahydropyranyle,
et dans laquelle R₃ et R₄ sont en position *méta* ou *para* l'un par rapport à l'autre, comportant les étapes :
a) d'hydroxyalkylation de l'agrégat X
b) d'hydroxylation ou de thiolation de l'agrégat X,
pour obtenir un composé intermédiaire de formule générale [H-R₃ - X - R₄] dans laquelle
R₃ = O, S
X = un agrégat *closo* ou *nido* de bore 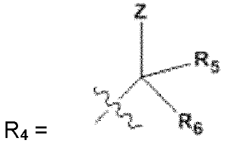
dans laquelle Z = OH
dans laquelle R₅ est choisi parmi H, un alkyle, un aryle, un hétéroaryle, un alkyléther, un alkylthioéther, une alkylamine et
R₆ est choisi parmi un alkyle, un aryle, un hétéroaryle, un alkyléther, un alkylthioéther, une alkylamine
ou R₅ et R₆ forment un motif tétrahydropyranyle,
et dans laquelle R₃ et R₄ sont en position *méta* ou *para* l'un par rapport à l'autre, et
c) d'éthérification ou d'estérification sélective de H-R₃ pour introduire A,
les étapes a) et b) pouvant être interchangées à volonté.

6. Utilisation d'un composé chimique selon l'une des revendications 1 à 4 ou de son sel pour l'inhibition ou la modulation de lipoxygénases extérieurs au corps humain ou animal.

7. Utilisation d'un composé chimique selon l'une des revendications 1 à 4 ou de son sel pour l'imitation de l'acide arachidonique et de ses dérivés dans des systèmes biologiques extérieurs au corps humain ou animal.

8. Utilisation d'un composé chimique selon l'une des revendications 1 à 4 ou de son sel dans des procédés d'imagerie.

9. Composé chimique selon l'une des revendications 1 à 4 ou son sel, destiné à être utilisé comme médicament.

10. Composé chimique selon l'une des revendications 1 à 4 ou son sel, destiné à être utilisé comme médicament pour le traitement de l'asthme, des maladies du SNC (système nerveux central), des allergies, de la rhinite, des maladies cardiovasculaires, dans la médiation de la douleur, dans la maladie d'Alzheimer, dans le processus visuel, dans les cancers, et dans les troubles gastro-intestinaux, rénaux, vasculaires et les problèmes de grossesse.

11. Composé chimique selon l'une des revendications 1 à 4 ou son sel, destiné à être utilisé dans des procédés diagnostiques ou thérapeutiques.

12. Composé chimique selon la revendication 4 ou son sel, destiné à être utilisé pour la BNCT (thérapie par capture de neutrons par le bore), la BNCS (synovectomie par capture de neutrons par le bore), l'IRM (imagerie par résonance magnétique), la TEP (tomographie par émission de positons), le TEMP (tomographie d'émission monophotonique), le PIGE (émission de rayons γ induite par particules) et l'AFM-NIAR (microscopie à force atomique avec alpha-autoradiographie induite par les neutrons).

13. Composition pharmaceutique contenant un ou plusieurs composés selon les revendications 1 à 4 ou leurs sels, en association avec un véhicule pharmaceutiquement acceptable.
